# EUROPEAN PATENT SPECIFICATION

(11) **EP 0 922 957 B1**
(45) Date of publication and mention of the grant of the patent: **29.03.2000**
(21) Application number: 97121451.5
(22) Date of filing: 05.12.1997
(51) Int. Cl.: G01N 33/48, G01N 33/50, G01N 33/569, G01N 33/68

(54) **Method to determine biomolecular interaction**
Verfahren zum Biomolekularinteraktionsnachweis
Méthode pour déterminer d'action réciproque biomoléculaire

(43) Date of publication of application: 16.06.1999
(73) Proprietor: von Gabain, Alexander, 1180 Wien (AT); Hirsh, Aaron, Boulder, CO 80302 (US)
(72) Inventor: von Gabain, Alexander, 1180 Wien (AT); Hirsh, Aaron, Boulder, CO 80302 (US)
(74) Representative: Alge, Daniel, Mag. Dr. rer.nat.

(56) References cited:
- E.T. BODER ET AL.: "Yeast surface display for screening combinatorial polypeptide libraries" NATURE BIOTECHNOLOGY, vol. 15, no. 6, 1 June 1997, WASHINGTON DC USA, pages 553-557, XP002064402
- M.C. KIEKE ET AL.: "Isolation of anti-T cell receptor scFv mutants by yeast surface display." PROTEIN ENINEERING, vol. 10, no. 11, 1 November 1997, OXFORD UK, pages 1303-1310, XP002064403

## Description

### FIELD OF THE INVENTION

Combinatorial chemistry (reviewed in 99), in which a diverse library of laboratory-constructed molecules is screened for structures that exhibit properties of interest, is a relatively novel and potentially powerful technology, promising useful application in three general areas. First, combinatorial chemistry may be used to identify molecules with highly specific adhesive or enzymatic activities useful in biomolecular research, clinical therapy, or diagnostics (e.g. 7, 12, and 51). Second, by screening a library for molecules that interact specifically with a known compound of significant interest, combinatorial chemistry may assist in identification of the known compound's native interactants (e.g. 138 and 179). Third, combinatorial chemistry may be used for epitope mapping, serving to elucidate the surface of structural interaction between two molecules of interest (e.g. 32, 51, and 138).

The current combinatorial chemistry of proteins, powerful though it may be, has shown two distinct limitations. In many libraries, the diverse proteins are presented on a platform that fails to confer much structural integrity; as a result, the proteins can be too plastic to exhibit a stable shape and consistent properties. (This drawback of many current libraries of proteins is demonstrated and discussed in the literature (8, 83).) Equally as important, the screening procedure can be labor-intensive, and factors outside of a protein's exhibition of the properties of interest often influence its isolation by a screen. This occurs in most screens reported in the literature (e.g. 138, 147, and 170). A related technology, two-hybrid systems, has proven useful in the latter two areas mentioned above, namely, interaction studies (60, 102, 116) and epitope mapping (77, 138). However, two hybrid systems place a very strict limit on the kinds of interaction that may be studied: both components of the interaction must be proteins, and they must be readily transported into the nucleus.

It was an object of the present invention to provide a method of polypeptide combinatorial chemistry that resolves or circumnavigates many of the difficulties encountered by other technologies. (The term polypeptide is used herein to comprise proteins, glycoproteins, and proteoglycans.) In this invention, a library of exogenous polypeptides (i.e., proteins, glycoproteins, or proteoglycans) is exhibited in one or more extracellular domains of an outer membrane polypeptide expressed by a cell population. Selective pressures exerted by toxic agents eliminate those cells whose exogenous polypeptide sequences fail to bind with high affinity to a preselected target compound. Thus, from a cell population exhibiting a vast diversity of exogenous polypeptide sequences, clones whose exogenous sequences bind the target compound are selected and amplified by growth. We refer to the process of randomization of an extracellular domain followed by selection for clones exhibiting specific properties as "imprinting".

With this invention, a wide variety of prokaryotic and eukaryotic polypeptides may be imprinted, serving any of the three areas of application mentioned above, namely, creation or discovery of polypeptide sequences with high affinity for a predetermined target, interaction studies, or epitope mapping. In more sophisticated embodiments of the invention, polypeptides may be imprinted to exhibit the following specific properties: multivalent binding to a predetermined target; high affinity for a target and low affinity for one or more molecules closely related to the target; inhibitory activity in a biological pathway (antagonism); excitatory activity in a biological pathway (agonism); or enzymatic activity.

### BACKGROUND OF THE INVENTION

### i. INTRODUCTION

This invention is the product of the confluence of three distinct areas of research: i. the study of prokaryotic and eukaryotic cellular membranes and their complex constituents; ii. the study of the evolution of microbial populations under the selective pressures of toxins, bacteriocins, or viral parasites; and iii. the design and screening of polypeptide combinatorial libraries. To comprehensively review these three areas of research is beyond the scope of this background. Instead, the background is intended to provide a fundamental understanding of each area of research that underpins this invention, while also offering a thorough list of references, and treating specific details and examples when they are directly related to or involved in specific embodiments of this invention.

### ii. THE COMPLEX CONSTITUENTS OF THE CELLULAR SURFACE

The membrane bounding any prokaryotic or eukaryotic cell, referred to here as the outer membrane, presents an extremely complex surface to the extracellular environment. The complexity of this surface is due largely to proteins, glycoproteins, and proteoglycans associated with the outer membrane. These molecules-referred to here as outer membrane polypeptides-exhibit a vast range of distinctive conformational, chemical, and electrical properties. Surveyed elsewhere in greater detail (24, 139), the outer membrane polypeptides are here categorized into several structural families. The conformational and functional differences between these families are relevant to this invention because outer membrane polypeptides provide the underlying structures that are adapted by our method to exhibit specific properties. Moreover, this invention often capitalizes on the intrinsic properties of outer membrane polypeptides by imprinting them to exhibit properties or perform functions that are similar to their natural ones. Finally, the deliniation of structural families presented here should also provide a profile of the vast range of properties exhibited by polypeptides on the cell surface, for it is in part this range that makes the capabilities of this invention so broad.

### a) Bitopic protein types Ia, Ib, and II

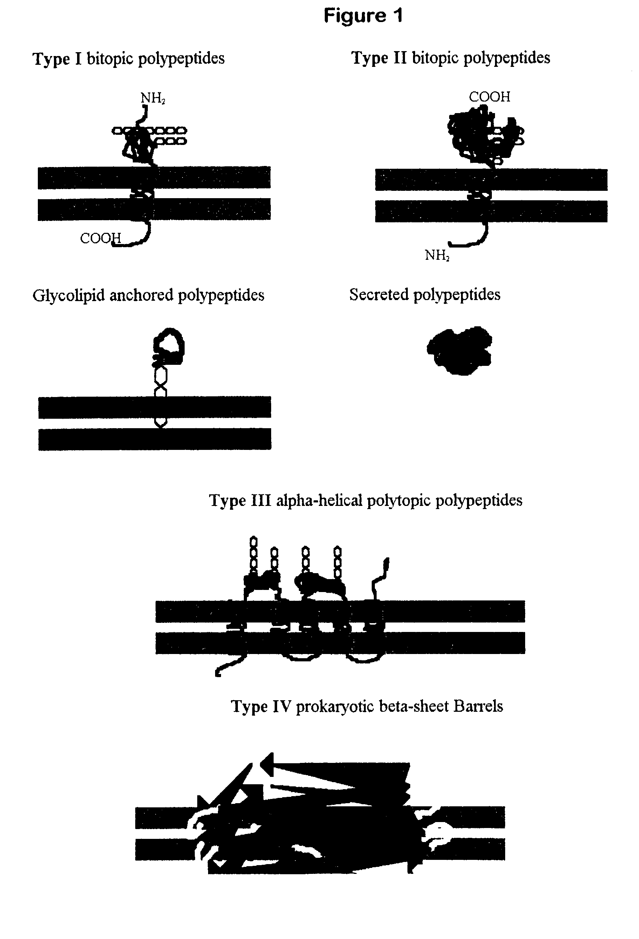

Single-pass transmembrane polypeptides (reviewed in 24, 139, and 153), also called bitopic polypeptides by Blobel (17), have one intra- and one extra-cellular domain, and a midsection that passes through the membrane. The terminal domains fold into tertiary structures, while the transmembrane section often forms a hydropathic α-helix, in which no more than 2 of the 20 transmembrane residues may be ionic. Singer (153) divides bitopic polypeptides into types Ia, Ib, and II (figure 1). Types Ia and Ib exhibit the amino terminus on the extracellular side of the outer membrane. Distinguishing type Ia is an amino terminal signal sequence that directs export to the membrane, and is cleaved before final membrane insertion. Type Ia polypeptides are ubiquitous, and in eukaryotes they are the predominant type. Because the transmembrane sequence of type Ia polypeptides is typically closer to the carboxyl terminus, the extracellular domain is often large. Consequently, type Ia polypeptides are competent for functions involving high-affinity adhesion or highly specific binding to extracellular targets. Among type Ia polypeptides are receptors for growth factors (46) and for peptide hormones (56); immune system antigen recognition molecules (103, 140); and molecules responsible for cell adhesion (156, 157). Proclivity for adhesion or highly specific binding make type Ia polypeptides suitable for use in many embodiments of this invention.

Type Ib polypeptides do not have a cleavable signal sequence. The transmembrane stretch is typically close to the amino terminus, and therefore there are few extracellular residues (153). This makes type Ib polypeptides suitable for sophisticated cytoplasmic functions, but poorly suited for use in embodiments of this invention.

Type II bitopic polypeptides are oriented with their carboxyl terminus on the exterior side of the membrane (figure 1). As in type Ia bitopic polypeptides, the bulk of type II molecules is usually on the exterior of the cell. Type II polypeptides are typically enzymes with extracellular active sites, such as glycosyl transferases (107), or highly specific extracellular receptors, such as the asialoglycoprotein receptor (151). Competence for extracellular enzymatic or receptor activity makes type II bitopic polypeptides suitable for use in certain embodiments of this invention.

### b) Polytopic polypeptide type III and β-barrels

Multipass transmembrane polypeptides (reviewed in 24, 139, and 153), also called polytopic polypeptides (17), traverse the lipid bilayer in two to fifteen or more passes. Like thread stitched into cloth, the polypeptide begins on one side of the membrane, passes through it, loops into the space on the other side of the membrane, then once again traverses the membrane to expose either its end or yet another loop leading to more transmembrane passes (figure 1). Although referred to and often illustrated as "a loop", each exposed region of a multipass polypeptide exhibits not only a unique sequence of amino acids, but also a distinctive tertiary structure. Like single-pass transmembrane polypeptides, multipass ones are suspended in the lipid bilayer by the hydrophobicity of transmembrane strands. However, in multipass polypeptides, these strands do not invariably twist into α-helices, because when multiple strands neighbor one another, they can adopt an alternative strategy for hydrogen bonding between peptide bonds: if each strand adopts the secondary structure of a β-strand, these strands can curl together into a closed cylinder, allowing hydrogen bonds to form between the peptide bonds on separate strands. The resultant tertiary structure is called a β-barrel (figure 1). It is extremely stable, and can be released from the outer memberane without denaturing tertiary structure. In several preferred embodiment of this invention, the extremely stable, polytopic β-barrel OmpA, is imprinted to bind to various targets.

Polytopic polypeptides that traverse the membrane in α-helical conformation often function as specific receptors for messengers or hormones, such as yeast mating pheromone (88) and many others (129). These and other α-helical polytopic receptors are categorized by Singer (153) as type III polypeptides. Their capacity to bind target molecules specifically, and sometimes multivalently, makes α-helical polytopic receptors ideal for use in many embodiments of this invention. Moreover, CISTEMs are easiest to construct when the polypeptides that are to be adapted are polytopic.

### c) Glycolipid-anchored polypeptides

Many prokaryotic and eukaryotic cell-surface polypeptides are fixed to the lipid bilayer not by a hydrophobic transmembrane strand, but rather by a glycolipid anchor (e.g. 180, reviewed in 54 and 113) (figure 1). An anchor consists of a lipid molecule, such as Dimyristyl glycerol or Alkylacyl glycerol, adorned by a multi-residue sugar complex, which is, in turn, covalently linked to the outer membrane polypeptide. Among eukaryotes, two signaling elements of a protein direct the addition of a glycolipid anchor: a well-defined amino terminal signal sequence, which enables transport into the lumen of the endoplasmic reticulum, and a carboxy terminal glycolipid-addition sequence, which is not precisely defined but generally consists of a short hydrophobic domain located 10-12 residues carboxy terminal to a pair of small residues (125). Once appended to the carboxy terminus of a protein, the glycolipid anchor itself can direct polypeptide insertion in a targeted region of the outer membrane (112).

Lacking an intracellular domain, glycolipid-anchored outer membrane proteins cannot perform cytosolic or periplasmic functions. Instead, they provide protection from the extracellular environment, or function as enzymes or adhesins recognizing exclusively extracellular substrates. At least six leukocyte surface proteins, many other eukaryotic proteins belonging to the immunoglobulin superfamily (54), and the trypanosome variable surface glycoprotein (the protective protein that the trypanosome sequentially alters to avoid detection by the host immune system (113), are anchored by glycolipids. These examples illustrate the characteristics of glycolipid-anchored proteins that are important to their use in embodiments of this invention: capacity for highly specific adhesion and plasticity that allows variation of the anchored protein without disturbing a robust system for export to and insertion in the outer membrane.

### d) Glycosylated proteins

Among eukaryotic cells, the diverse array of surface-exposed proteins is adorned by yet another tier of complex molecules with distinctive properties, namely, the saccharides (structure and function reviewed in 61 and 111, laboratory analysis and relavent techniques reviewed in 62). The oligosaccharides adorning glycoproteins generally consist of less than 15 residues. However, owing to the diversity of residues and covalent linkages that permit assembly into branched as well as linear structures, oligosaccharides exhibit thousands of distinct structures. The polysaccharides of integral membrane proteoglycans are larger than oligosaccharides, and can contain dozens of residues.

The oligo- or polysaccharide molecule attached to a particular site in a polypeptide is not a simple function of the local amino acid sequence. The same polypeptide sequence receives distinct saccharide chains when expressed in different cells, and separate copies of a polypeptide expressed in a single cell type can have different sugars attached at a given site. Because sugar residues are sequentially appended, each by a particular enzyme to which the protein is exposed in the course of the processes of translation, folding, and export to the outer membrane, the glycosylation at a site is largely a product of the extent and duration of the site's exposure to each enzyme. Exposure of a site is, in turn, affected by the way a protein folds and the rate at which it moves through the steps from translation to insertion. Consequently, the glycosylation at a site can be altered in unpredictable ways by changes to the local sequence or to the sequence elsewhere in the polypeptide (61,111).

Diversity of molecular structure and relatively complete exposure to the extracellular environment make the saccharides appended to proteins effective receptors of specific substrates outside the cell. Glycoproteins and proteoglycans are involved in the intercellular recognition and adhesion underpinning sperm-egg interactions, blood clotting, and inflammation (111,137). The roles they play, via adhesion, in these important processes make the glycoproteins and proteoglycans ideal for use in certain embodiments of this invention.

### iii. MECHANISM AND PROCESS OF MEMBRANE POLYPEPTIDE INSERTION

It is significant to some embodiments of this invention that insertion of polypeptides into the outer membrane and translocation of secreted polypeptides across the outer membrane are performed by the same cellular machinery (153). Among eukaryotes, integral membrane polypeptides inserted into the endoplasmic reticulum (ER) have the same cleavable or noncleavable amino terminal signal sequences as completely translocated polyeptides. This is consistent with the fact that insertion and secretion depend on the same cytoplasmic signal recognition protein (SRP), which transfers both inserted and secreted polypeptides to the SRP receptor on the ER membrane. Similarly, among bacteria, membrane insertion and translocation seem to require the same gene products, namely secA and secY (5, 6, 145). Among prokaryotes and eukaryotes alike, there are many examples of secreted and membrane-bound polypeptide isoforms. Often the only difference between the isoforms is the existence of a hydrophobic stretch of residues or a glycolipid attachment site near the carboxy terminus of the membrane-bound polypeptide (79, 128).

The process of type III polypeptide intercalation into the outer membrane also has implications that are significant to this invention. The cellular machinery that inserts bitopic polypeptides and translocates secreted ones is also responsible for intercalating α-helical multipass polypeptides into the outer membrane. This is clear from studies that show that i) type III polypeptides sometimes have the same signal sequences as bitopic and secreted polypeptides, and ii) membrane intercalation of type III polypeptides requiresthe same gene products as insertion of bitopic polypeptides. Several experimental approaches, including gene fusion, which is discussed below (III.iv.a), have shown that the cellular machinery for inserting polypeptides in the outer membrane intercalates most type III polypeptides by translocating one hydrophilic extracellular loop at a time. The polypeptide is thus intercalated as a series of independently inserted segments (5, 6, 117, 153, 172, 173).

### iv. ENGINEERING COMPONENTS OF THE OUTER MEMBRANE

### a) Insertion of exogenous sequences into extracellular polypeptide domains

All embodiments of this invention require a topological map of the imprinted polypeptide, and a technique for introducing new sequences to specified extracellular domains of the imprinted polypeptide. Both of these needs are met by gene fusion techniques.

Most of the polypeptides reviewed in the previous sections (III.ii.a-d) have already been topologically mapped using gene-fusion techniques. In these studies, the gene for a membrane polypeptide was joined to a gene coding for a "reporter", a protein that is functionally active only when it is located on a particular side of the cell membrane. This genetic construct coded for a hybrid polypeptide in which the membrane polypeptide bore the reporter moiety as a mid-sequence insertion or as a C-terminal extension. Provided the foreign moiety disrupted neither sorting of the polypeptide to the appropriate membrane, nor insertion of the polypeptide into the membrane, the reporter was localized in the same way as the immediately adjacent region of the membrane polypeptide. This region's location on the exterior or interior of the membrane was then inferred from the reporter's activity, or lack thereof. The entire polypeptide's topology was deduced by assaying reporter activity in a set of hybrid polypeptides, each of which bore the reporter at a different place in the membrane polypeptide's sequence (these gene-fusion mapping techniques are reviewed and described in 21, 22, 23). Not only has this approach provided topological maps of most of the polypeptides described in the previous section; it can also be used to determine the topology of any membrane polypeptide that has not yet been mapped, but is a candidate for imprinting by this invention.

In addition to a topological map of the imprinted polypeptide, each embodiment of this invention also requires a technique for inserting exogenous peptide sequences into one or more extracellular sites in the imprinted polypeptide. Alternatively, exogenous peptide sequences may replace sections of the extracellular domains of the membrane proteins that are exposed at the cell surface and thus accessible for a target compound.

Because gene-fusion topology studies used a set of hybrid polypeptides, each bearing the reporter in a region whose location relative to the membrane was to be mapped, several techniques were developed to enable insertion of the reporter gene at precisely targeted sites in the membrane protein gene. These techniques include the following: i. the use of vectors with restriction sites that occur at unique locations in the membrane protein gene (described in 28,141); ii. an oligonucleotide-directed deletion mutagenesis method (described in 21); and iii. polymerase chain reaction methods (described in 22). Each of these techniques can be used in this invention to insert exogenous sequences to a target site in the imprinted polypeptide; an extension of these techniques, which is used in this invention to introduce new variation to extracellular domains, is described below (V. iv. c).

Besides providing topological maps and techniques for inserting exogenous peptide sequences, gene-fusion studies have collectively demonstrated that introduction of an exogenous sequence of peptides to an extracellular domain of a membrane polypeptide does not interfere with the sorting of the polypeptide to its appropriate membrane, or with the polypeptide's normal insertion into the membrane. This capacity of extracellular domains to accommodate foreign oligopeptides is critical to the construction of combinatorial libraries of exogenous oligopeptides in the extracellular domains of membrane polypeptides. This capacity is also, therefore, critical to all embodiments of this invention. It has been observed in various bacterial, yeast, and mammalian polypeptides, including the following examples.

An extracellular domain of lamB, an outer membrane porin of *E. coli* (thoroughly characterized in 38 and 39), has been shown to accept exogenous polypeptides up to 165 amino acids in length without disturbing normal sorting or insertion of the protein (28, 38, 39). Foreign sequences have also been inserted into extracellular domains of *E. coli* PhoE (phosphate-limitation-inducible outer membrane pore, described in 4) and FhuA (ferrichrome uptake receptor, described in 105), and the insertions had no effect on protein sorting or insertion. In the specific case of *E. coli* OmpA, one of the most abundant polypeptides of the bacterial outer membrane (sequenced in 14 and 42, structure and function reviewed in 115, 127, and 141), oligonucleotides rich in restriction endonuclease sites were inserted into the *omp*A gene at sites corresponding to protein loops II and IV, and the altered protein was expressed and exhibited normally (76). Subsequently, influenza haemaglutinin, beta-galactosidase, foot and mouth disease viral protein 1 (FMDV-VP1), malaria antigens, and several other antigenic polypeptides up to 56 amino acids in length were successfully displayed in OmpA loops III and IV (96, 144). Furthermore, two lines of evidence demonstrated that these exogenous polypeptides adopted stable folded structures. First, ompA fusion proteins exhibiting viral antigens were shown to elicit intense virus-specific immune response in rabbits (144). Second, Cex exoglucanase displayed on an extracellular domain of OmpA hydrolyzed cellulose as effectively as conventionally immobilized enzymes (70, 71).

In yeast, extracellular domains of arginine permease (87), uracil permease (152), ste2 protein, and HMG CoA reductase (23), all accept exogenous peptide sequences without exhibiting disturbance to normal sorting and insertion. Higher eukaryotic membrane polypeptides have also exhibited the capacity to accept foreign inserts at extracellular domains. When a polypeptide is expressed in eukaryotic cells, gene fusion studies may insert extra glycosylation sites to function as reporters: only polypeptide domains on the luminal side of the ER are exposed to glycosylating enzymes, and luminal domains become extracellular domains. Extra glycosylation sites were inserted into extramembranous regions of mouse muscle nicotinic acetycholine receptor: the inserted sites did not disrupt normal sorting or insertion of the outer membrane polypeptide; and, sites inserted in extracellular receptor domains were in fact glycosylated (41).

In summary, gene fusion mapping techniques contribute to this invention in four ways. First, these techniques have been widely used to determine the topology of countless outer membrane polypeptides, many of which are described in the previous section (III.ii.a-d) and may be imprinted by this invention. Second, these techniques may be used to provide topological maps for polypeptides that have not yet been mapped, but may be candidates for imprinting by this invention. Third, gene fusion techniques provide this invention with commonly practiced methods for inserting an exogenous peptide sequence into a targeted region of a membrane polypeptide; the use of these techniques in the invention, as well as the straightforward extension that enables the insertion of variable sequences, is described below (V.iv.b). Fourth, gene fusion topology studies have demonstrated that the introduction of an exogenous sequence to an extracellular domain of many mapped polypeptides does not interfere with the polypeptides' export to or insertion in the membrane.

### b) Alteration of mode of attachment

Certain embodiments of this invention involve alteration of a polypeptide's mode of attachment to the outer membrane. Because insertion into the membrane and translocation across the membrane are handled by the same cellular machinery (III.iii), secreted, transmembrane, and glycolipid anchored isomorphs of a single polypeptide are often interconvertible. Moreover, secreted and membrane-bound isomorphs can use the same amino terminal signal sequence, so in many cases, the only alteration required for conversion of a polypeptide to its isomorph is a change at the polypeptide's carboxy terminal. Attachment of a 20 residue hydrophobic sequence to the carboxy terminal of a secreted polypeptide is often sufficient to construct an isomorphic polypeptide that is anchored by a transmembrane span; this technique for converting a secreted polypeptide into a membrane-bound one is described at length in the literature (examples of isomorphic membrane-bound and secreted forms of polypeptides, and techniques for their interconversion, are described in 79,128,142,149). Alternatively, transmembrane or secreted polypeptides can be converted to glycolipid anchored variants by addition of carboxy terminal signal sequences from the precursors of glycolipid anchored polypeptides; this technique is also described in the literature (addition of glycolipid anchors described in 36, 37, 53, 164). In some cases, a glycolipid anchor not only attaches an exogenous polypeptide to a cell membrane, but also directs sorting of the anchored alien polypeptide (112). In such cases, a single glycolipid anchor sequence is all that is required to direct an exogenous polypeptide to the membrane, translocate it, and anchor it.

### c) Fusion of membrane polypeptides

In certain embodiments of this invention, two or more outer membrane polypeptides are fused to construct a chimeric multipass polypeptide comprising extracellular domains of both constituent polypeptides. This manipulation has been successfully performed with a variety of prokaryotic and eukaryotic polypeptides. For example, in *E. coli* ompF-ompC chimeric protein 3601 (69), the region of ompF comprising extracellular loops 1 through 5 is fused to the region of ompC comprising extracellular loops 6 and 7. When expressed in *E. coli* SM1005 (F⁻ Δ*lac*U169 *rps*L *rel*A *thi*A *fib*B *gyr*A *omp*C *omp*F), this chimeric polypeptide is properly inserted in the outer membrane (69). In a striking example of the construction of eukaryotic chimeric polypeptides, novel transmembrane polypeptides were created as follows. A region of the human asialoglycoprotein receptor H1, which included the polypeptide's transmembrane domain (20 hydrophobic residues, 40-61 in the wild-type protein), was tandemly repeated two, three, or four times. In each case the H1 repeats were joined by relatively hydrophilic linkers, such as the hexapeptide HRPSLG. These constructs were normally inserted into membrane, and luminal domains were normally glycosylated (172).

The general principle that often makes fusion of outer membrane proteins possible has already been outlined: the above description of the process of membrane protein insertion emphasizes that α-helical transmembrane strands of polytopic membrane proteins are inserted sequentially and independently of one another. An implication of this mechanism of intercalation of transmembrane strands is that almost any chimeric polypeptide that is constructed of an amino-terminal signal sequence followed by multiple ∼20-residue-long hydrophobic stretches, alternating with stretches of more hydrophilic residues, can be normally inserted, provided each hydrophobic stretch is independently stable in the membrane (172).

### v. TOXIC AGENTS

Many of the polypeptides of the outer membrane are ports of adhesion for an array of toxic agents, in particular pathogenic agents, that kill or retard multiplication of the cells they enter. The following sections review the requirements for adhesion and the nature of pathogenic action of three distinct categories of toxic agents. First, however, three general concepts should be introduced: the adhesion domain; resistance; and immunity. These concepts are important for understanding how this invention selects rare cells exhibiting specific polypeptides from a highly variable cell population. For the purpose of the present invention, the term "toxic agent" denotes any agent that impairs or inhibits growth of the cells, in particular, a pathogenic agent.

Many toxic agents recognize a specific region (the adhesion domain) of a specific polypeptide (the receptor) on the surface of a specific cell type (the host). Binding by the agent to the adhesion domain is the first step in a process that culminates in cell lysis or inhibition of cell growth and multiplication. If this first step is impeded by mutations in the adhesion domain, by repression of the receptor, or by the interference of an inhibitor occluding the adhesion domain, a toxic agent's invasion of the cell is blocked. When all of an agent's possible receptor molecules on a cell are, for whatever reason, inaccessible or dysfunctional for agent entry, the cell is rendered immune. When some but not all receptors are inaccessible or dysfunctional, the cell may become resistant but not immune: reduction of the number of receptors may slow the entry of agents, and thus reduce the rapidity or intensity of the agent's harmful effects.
For the purpose of the present invention, a toxic, in particular a pathogenic agent may be employed that has been alterered such that it is different from the corresponding naturally occuring agent with regard to specificity and mode of action on the cell. For example, pathogenic agents like bacteriocins and bacteriophages can be altered such that they change their preference for a specific adhesion domain. Thus it is possible to design strategies to make pathogens adhere to a great variety of adhesion domains.

### a) Bacteriocins

Bacteriocins are bacterially produced proteins that are harmless to the strains that express and secrete them, but fatal to other, often closely related, bacteria (bacteriocins are reviewed in 106 and 135). The most extensively studied of the bacteriocins are the colicins, which are expressed by *E. coli*, and are encoded by plasmids that also code for the proteins that confer colicin immunity upon colicin-producing cells. The colicins are composed of three structural domains, each of which is responsible for one of three consecutive steps of colicin activity: i. recognition of an adhesion domain; ii. translocation across the cellular membrane; and iii. cell destruction (13, 135).

The colicins kill by two principal mechanisms: i. the formation of a pore in the cytoplasmic membrane, resulting in membrane permeabilization and de-energization (colicins E1, A, B, Ia, Ib, and K); ii. enzymatic cleavage of DNA or 16S ribosomal RNA (colicins E2 and E3) (106, 135). The kinetics of cell death when bacteria are exposed to colicins suggest that the toxic molecules enter the cell in discrete, fatal doses, which may comprise only a single molecule, but may also consist of a number of active molecules imported in one concerted step (135). The important point here is that a single dose kills a single cell.

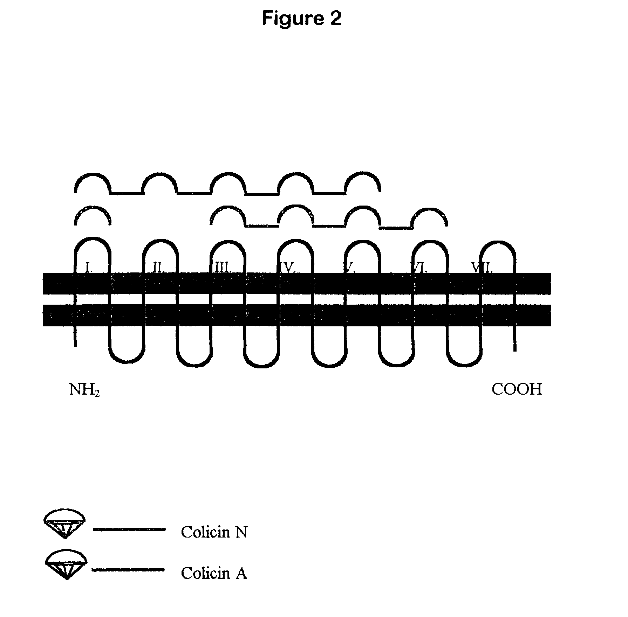

Almost every major outer membrane protein of *E. coli* is the receptor for at least one colicin. When multiple colicins recognize a single receptor, their adhesion domains may overlap partially. Two colicins that adhere to OmpF, a major outer membrane porin, furnish an example. As is illustrated in figure 2, OmpF exhibits seven extra-cellular loops, L1 through L7, where L1 is amino proximal and L7 is carboxy proximal. The adhesion domain of colicin N includes L1, L3, L4, L5, and L6, but not L2 or L7. Thus, alterations in the peptide sequence of L1 result in total immunity to colicin N, whereas alterations to L2 do not confer even slight resistance. The adhesion domain of colicin A, another colicin that recognizes ompF, is somewhat different. Loops 1 through 5 are required for colicinA to bind its receptor, while L6 and L7 are superfluous (69). The chimeric ompF-ompC protein 3601, which was described above (III.iv.c), comprises loops 1-5 from ompF and loops 6 and 7 from the distinct ompC. As would be expected, *E. coli* SM1005 expressing 3601 are totally susceptible to colicin A, but totally immune to colicin N.

The number of receptors per cell is important to this invention's selection of specific cells from a highly variable population. The functional receptors of colicins are generally present on the surface of the cell in thousands of copies. However, it is important to the function of this invention that the level of expression of most receptors is easily manipulated: the gene encoding the receptor has already been, or may easily be, cloned into a plasmid with an inducible heterologous promoter, permitting external regulation of expression. Protocols for the construction and manipulation of plasmids with inducible promoters, permitting regulation of expression, are described in the literature (74, 76, 29, 146).

### b) Bacteriophages

Bacteriophages are a ubiquitous and extraordinarily diverse group of viral parasites of bacteria. There are twelve families of phage, comprising 3500 distinct isolates of known morphology, and infecting over 100 bacterial genera. Their vast variation is evident even at the level of nucleic acid: depending on species, phage contain single stranded DNA, double stranded DNA, single stranded RNA, or double stranded RNA. A wide range of morphologies is usually divided into four categories: tailed; cubic; filamentous; and pleomorphic. Extensive reviews of morphology, genetics, ecology, and life-cycle are available in the literature (3, 34).

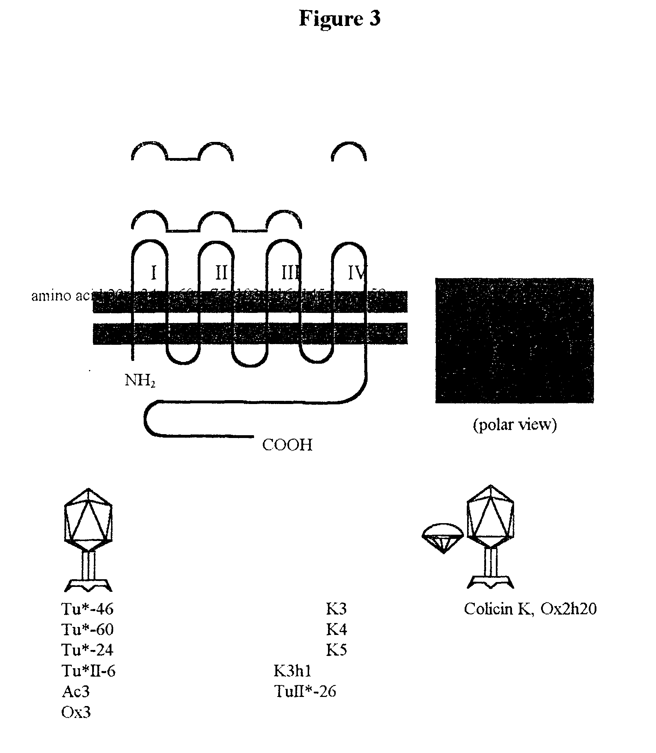

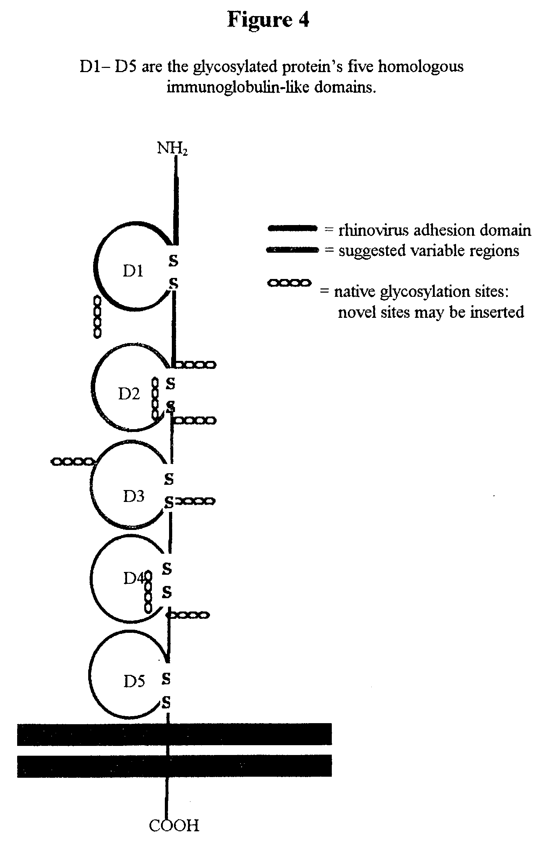

Very much like bacteriocins, phages bind to specific adhesion domains of receptors on the host surface. A single outer membrane protein may be the receptor for multiple phage species, whose adhesion domains overlap to varying extents. Figure 3 shows the adhesion domains of various phages, as well as one of the colicins, that utilize *E. coli* outer membrane protein A as their receptor. The figure also shows the receptor domains that are manifestly not utilized by the phages: alterations to these regions of the receptor do not affect phage adsorption, and thus do not confer phage-resistance to the bacterial cell. Phage receptors, like those of bacteriocins, are often present in 100s to 1000s of copies per cell, but this number can be manipulated through use of a plasmid-encoded receptor regulated by an inducible heterologous promoter. As was stressed above, regulation of the level of expression of the imprinted polypeptide is important to this invention. Protocols for the construction and manipulation of plasmids with inducible promoters are described in the literature (74, 76, 29, 146).

Following binding to the receptor, the viral nucleic acid enters the bacterial host, and phage multiplication commences. The strategies for multiplication, and the effects of multiplication upon the host, vary widely across phage species. Immediately following infection, some but not all species of phage confer immunity to subsequent infections by other individuals of the same phage species. If the infecting phage is a virulent one, it quickly modifies normal bacterial syntheses in order to appropriate them to its own multiplication and release into the extracellular space. Distinct strategies for release have very different effects upon the host. The *Inoviridae* (a family of filamentous phages) are liberated by extrusion: new phage are secreted through the bacterial membrane (122). While this process is not lethal to the bacterium, it is metabolically taxing, and therefore infected bacteria divide at a slower rate than uninfected bacteria (30). Transmission also takes place vertically, as daughter cells inherit the infection from their parents. A strategy for release similar to extrusion is exhibited by the *Plasmaviridae* (a family of pleomorphic phages). These phages, which consist only of a double stranded DNA molecule surrounded by a lipoprotein envelope, are liberated from the host by budding from the cellular membrane. Again, the process is not lethal but retards multiplication. By contrast, lysis, a means of release for tailed and cubic phages, destroys the host. After intracellular phage multiply to numbers of 20-1000, they weaken the bacterial cell from within, causing it to burst and liberate the infective particles.

The bacteriophages exert such a wide range of effects upon their hosts that even among phage species using the same receptor there may be several different consequences of infection. Moreover, the effects of a phage upon its host can be manipulated. Mutagenesis followed by artificial selection for phage virulence or benevolence can intensify or mitigate a phage's deleterious effect on its host (31). Insertion or deletion of phage genes, including insertion of genes that confer metabolic capabilities or antibiotic resistance upon infected bacteria, are commonly practiced means of manipulating the impact that phage have upon their bacterial hosts. Another way to modify a phage's effects upon its host is to alter the host. For example, certain groEL mutations in *E. coli* produce bacterial strains that are susceptible to T4, T5, lambda, and 186 phage infection, but do not permit the intracellular assembly of mature phage particles (177). Thus, infected bacteria are retarded, or even killed, but they do not yield infectious phage particles; in short, these bacteria are a dead-end for the phage that infect them. Finally, the effects of phage upon their hosts can be modified by the preparation of phage ghosts (phage depleted of DNA), by methods described in the literature (20, 174). Because they do not carry a genome, phage ghosts do not infect cells. They do, however, adhere to cells, pierce the cytoplasmic membrane, and form ion channels, which would normally serve to transport the phage genomes. Whereas normal phage close these channels behind them, phage ghosts leave the channels open indefinitely. The rate ion efflux through these channels in linearly related to the number of ghosts that bind to the cell; gradually, the efflux depolarizes the cell, which eventually dies.

### c) Toxins and viruses of eukaryotic cells

These toxic, in particular pathogenic agents recognize specific adhesion domains of their receptors on eukaryotic cell-surfaces. The receptors of at least thirty different species of virus of eukaryotic cell lines, representing eleven different viral families, are described in the literature, and for most of these pathogens, the viral adhesion domains, which may comprise oligosaccharides, glycosylated peptide sequences, or sequences unadorned by sugars, have been characterized (165). The receptors of eukaryotic pathogenic agents, like those of bacteriocins and bacteriophage, are commonly cloned onto vectors and expressed from inducible promoters, allowing manipulation of the number of receptors expressed per cell (159, 146, 120, 67).

Major group human rhinoviruses (HRVs) provide an example of the pathogenic agents that affect eukaryotic cells and are suitable for use in this invention. HRVs are members of *Picornaviridae*, small protein-encapsidated viruses with RNA genomes. Of 100 serotypes of rhinovirus, 89 use an extremely well-characterized glycoprotein, ICAM-1 (CD-54) as their receptor (158, 52). HRV serotype 14 is well studied , easily propagated in the lab, and therefore suitable for use in this invention (159, 52). Major group HRVs grow efficiently in many different lines of human cell culture. The most commonly used tissues are WI-38 diploid fibroblast, fetal tonsil, MRC, HeLa and COS cell lines. Optimal conditions and protocols for growth are described in the literature (52). HRVs form plaques on monolayers of a variety of cell lines exposed by any one of the described techniques for exposure to viral agents. Infected cells yield 10 to 200 plaque forming units per cell after 10 to 12 hours of incubation (52).

The structure of HRV14 has been solved by X-ray crystallography (143). The capsid is 30 nm in diameter, exhibiting icosahedral symmetery and a canyon, 120-300nm (12-30Å) wide and 250nm (25Å) deep, that runs along the 5-fold axis and contains the pathogen's receptor binding site. The receptor is the well-characterized member of the immunoglobulin superfamily, ICAM-1 (CD-54), a 532 residue, 19nm x 2-3 nm rod comprising 5 immunoglobulin-like domains arranged in a line with a hinge between domains 2 and 3 (figure 4). Viral binding involves insertion of the distal immunoglobulin domain (D1) into the viral canyon. This mode of interaction is extremely vulnerable to disturbance by steric inhibition (159). Thus, although the lower three immunoglobulin-like domains (D3-D5) are not directly involved in viral binding, and mutations within them do not disturb adhesion, their complete deletion does inhibit adhesion by reducing accessibility of D1.

ICAM-1 bears multiple N-linked oligosaccharides, and variation in glycosylation between cell-types results in ICAM-1's molecular mass heterogeneity of 76 to 114 kD. It functions as the cell-surface ligand for lymphocyte function-associated antigen 1 (LFA-1), and binding between ICAM-1 and LFA-1 contibutes to leukocyte adhesion in several immunological and inflammatory processes (50). Cytokines secreted by T-lymphocytes and monocytes, including IFN-γ,TNF-α and β and IL-α and β induce expression of 3.5 x 10⁶ copies per endothelial cell. However, uninduced cells exhibit much lower levels of ICAM-1, and in the CDM8 expression system, ICAM-1 can be exogenously regulated through control of an inducible promoter (148,159) as is important to the function of this invention.

### vi. COMBINATORIAL CHEMISTRY

Combinatorial chemistry consists of two or three steps, often performed in iterative cycles. The first step is the generation of a diverse population of molecules, the so-called combinatorial library. In the second step, the molecules in the combinatorial library are partitioned according to their exhibition of properties sought by researchers, such as affinity for a particular compound or enzymatic activity. In a third step that is practiced in some but not all embodiments of combinatorial chemistry, the molecules found to exhibit the desired properties are replicated or resynthesized. The cycle may then be repeated, beginning either at the first step, with the introduction of new variation to the molecular population, or at the second step, with the partitioning of the population according to the exhibition of desired properties. When a combinatorial approach is successful, the cycle of generating molecular diversity, whittling down diversity in favor of the few molecules that display the desired properties, and, in some cases, amplifying the numbers of these molecules, ultimately yields molecules exhibiting the desired specific activities.

The immune system is essentially a genetically coded combinatorial library, complete with steps of variation, selection, and replication: A vast diversity of antibodies is displayed on the surface of B-cells (variation), and specific recognition of antigen by any given cell triggers that cell's proliferation (partitioning and replication). It is not surprising, then, that the first in vitro genetically-coded combinatorial libraries were directly appropriated from the immune system: B-cells were immortalized through hybridization to mouse myeloma lines (175). Later, in order to circumnavigate difficulties of hybridization, and to utilize a greater part of the antibody repertoire, *E. coli* were engineered to express and secrete the variable chains of antibodies (98). However, because secreted antibodies were "untagged" (99)-*viz*. not physically linked to their genetic codes-screens for antibodies that bound antigen had to be performed according to techniques that allowed researchers to trace from each selected antibody to the gene that encoded it. This severely limited the number of clones that could feasibly be screened (7, 98). The advent of phage-display, or "fusion-phage", libraries made it possible to screen variable proteins that were "tagged" (99), and thus competent for immediate replication.

Combinatorial libraries of fusion-phage (134, 147) are composed of filamentous bacteriophage or phagemid that have been engineered to bear semi-random exogenous proteins as sandwich fusions near the end of the adsorption protein pIII. The method of screening these libraries for phage that exhibit affinity for a target compound is known as "affinity purification": the target is immobilized on a solid surface or column, over which the phage library is washed; phage bound to the target are eluted, amplified by growth in bacterial culture, and then subjected to yet another round of partitioning. This cycle is usually iterated until a tractable number of clones are isolated. Phage libraries have been used for epitope mapping (51, 138)-*viz*. pinpointing the region of a larger protein that is involved in binding a target compound of interest-as well as for deriving novel ligands to targets. Entire single-chain variable-fragment antibodies (scFv's) have been inserted into phagemid pIII (118), and libraries of scFv's or semi-random oligopeptides have been screened for binding to a variety of medically significant polypeptides (8, 12), nucleic acids (33), and molecules not previously known to bind proteins (58).

However, while fusion phage technology has been responsible for significant strides forward in combinatorial chemistry-such as the recognition that "tagged" libraries would permit screening of a vastly increased number of sequences-phage libraries themselves have not escaped several besetting difficulties. First, in affinity purification of phage libraries, factors besides affinity for the target compound contribute significantly to each clone's selection. For example, the collection of clones selected from a single library by affinity purification can vary dramatically with the technique used for eluting phage from the solid substrate of target compound (170). Second, phage adsorption protein, pIII, may fail to provide exhibited proteins with a sufficiently stable platform. Many polypeptides acquire structural rigidity only in certain constraining conformations; otherwise they remain extremely plastic (24, 83). Therefore, to be functionally active structures, many polypeptides will require a foundation other than pIII (e.g. see 7, 8).

A promising solution to problems with the platform for polypeptide display was introduced when a combinatorial library of proteins was displayed in an extracellular loop of a bacterial outer membrane protein (25). To construct this library, between 1 and 20 semi-random oligonucleotides, each one 33 bases long, were inserted into the genetic site encoding an extracellular loop of the outer membrane porin LamB. Like many membrane proteins that have been the subjects of topological (21-23) and antigen presentation (96, 144) studies (III.iv.a), LamB intercalates the outer membrane even when exogenous polypeptide sequences have been inserted into one or more of the protein's extracellular domains (35, 36). Thus, when *lam*B genes bearing insertions were used to transform *E. coli*, each transformant expressed LamB in which one extracellular domain exhibited a semi-random, exogenous oligopeptide between 11 and 220 amino acids long.

To screen the library for proteins exhibiting affinity for the target compound iron oxide, the *E. coli* population was placed in a suspension of iron oxide particles, binding was allowed to occur, and a magnet was used to recover bacteria that had adhered to metal. The selected portion of the population was amplified by bacterial growth, and the partitioning procedure was repeated. After four cycles of selection and amplification, fresh variation was introduced according to a strategy that would not erase sequence-information accrued thus far (see (25) for a detailed description of this method of semi-conservative introduction of sequence-variation). Another four cycles of selection and amplification then yielded four clones, three of which bore a consensus sequence for binding iron oxide.

While the combinatorial library in LamB introduced the promising possibility of using bacterial outer membrane proteins as platforms for presenting libraries of diverse polypeptides, the selection procedure applied to the library was labor-intensive and very limited in its applicability. The target compound, iron oxide, was conveniently chosen to allow screening for magnetism-a procedure that could not be applied to screening for the majority of interesting interactions that polypeptides exhibit. The present invention offers a user-friendly, efficient, and precise method of selection, capable of screening combinatorial libraries of proteins, glycoproteins, or proteoglycans presented on the stable platform of an outer membrane polypeptide.

### SUMMARY OF THE INVENTION

This invention provides a method for determining the interaction between a (poly)peptide of interest and a target compound, which interaction reflects, *inter alia* specific, prescribed properties of the (poly)peptide of interest, such as affinity of the (poly)peptide for a predetermined target, enzymatic activity, or inhibition of a biologically active molecule. In this interaction, at least one of the interactants is unknown. The method allows for identifying the unknown interaction partner, i.e. a (poly)peptide of interest and/or the target compound. For some embodiments, both interactants may be unknown, e.g. when an unknown ligand, upon binding at a receptor site, exerts a certain effect on the cell which needs to be interfered with and both the ligand and the receptor domain to which it binds need to be identified. (The words "identify" and "identification" are used here, as in the rest of the specification, to denote both the discovery of(poly)peptides of interest that exist in nature, and the design of polypeptides that have no natural occurrence.) Embodiments of this method are called CISTEMs, an acronym for Cellular Imprinting by Selection: Teleological Evolution of Molecules. A CISTEM applies selective pressure to a population of genetically engineered cells in order to amplify the frequency of cells in which an outer membrane polypeptide shows prescribed properties. This process is termed "imprinting" the polypeptide. The range of polypeptides that may be imprinted by this invention is broad, comprising proteins, glycoproteins, and proteoglycans, presented in a wide range of sizes, on a diverse array of underlying structures, and by any one of several prokaryotic or eukaryotic cell cultures (figure 1, III.ii).

The invention is directed to method for determining the interaction between a (poly)peptide of interest and a target compound wherein at least one of the interactants is unknown, comprising the steps
a) constructing a prokaryotic or eukaryotic cell population by engineering it to express a membrane polypeptide which has the (poly)peptide of interest in its extracellular domain, said extracellular domain being exhibited in one or more copies on each cell's surface immediately adjacent to an adhesion domain for a toxic agent, said adhesion domain and said extracellular domain belonging to the same membrane polypeptide, such that upon interaction of the target compound and the (poly)peptide of interest the toxic agent is occluded from the adhesion domain,
b) exposing the cell population to a target compound or a population of target compounds for a period of time sufficient for the target compound to interact with the (poly)peptide of interest,
c) exposing the cell population, simultaneously with or after step b), to the toxic agent for a period of time sufficient for the agent to attach to its adhesion domain,
d) selecting for the clones which are at a growth advantage due to the interaction of the (poly)peptide of interest with the target compound,
e) allowing the selected clones to multiply,
f) identifying the unknown interaction partner and isolating it.

The terms "interaction" or "interact" for the purpose of the present invention denote any action of the target compound upon the extracellular domain that carries (poly)peptide of interest (variable domain) and that results in occluding the toxic agent from binding to its adhesion domain. This action may comprise binding, adhesion, structural changes or chemical modifications of the variable domain which has an impact on the adhesion domain. Such impact on the adhesion domain may, inter alia, be a conformational change of the adhesion domain that prevents the harmful action of the toxic agent.

The invention is useful in at least three general areas. First, the invention may be employed to identify (poly)peptides with properties that are useful in medical diagnostics, therapeutics, research, laboratory proceedures, or other applications. Among the polypeptides that may be identified by the method disclosed here are polypeptides that interact with, in particular bind with high affinity and specificity to, virtually any given target compound, polypeptides that adhere to their respective target compounds at multiple epitopes, polypeptides that bind their respective target compounds but show no affinity for compounds closely related to the targets, enzymatic polypeptides, and polypeptides that show biological activities, such as antagonism or agonism of a receptor. Second, by identifying polypeptide sequences that interact specifically with a target that is a known compound of significant interest, this invention may assist in the identification of the known compound's native interactants. Third, this invention may be used to analyse the interaction between a known polypeptide of interest and a known target molecule. By identifying which sequences in the polypeptide of interest interact with the target molecule, the invention assists in mapping the polypeptide epitopes that play a role in the interaction. In a further embodiment, the method of the invention may be used to identify a target compound from a population of target compounds that binds to a known (poly)peptide. In this embodiment, cells displaying a given section in the extracellular domain of the membrane are used in a screening assay as test cells to screen for pharmaceutically active compounds that interact with the (poly)peptide of interest.

Hence, in a preferred embodiment, the invention comprises a method in the form of a high-throughput screening assay wherein the cells are exposed to a multiplicity of target compounds from which an unknown interaction partner for a (poly)peptide of interest is to be identified.

This invention may also be used to identify polypeptides with more sophisticated properties of interaction with the target compound than prescribed binding affinity. Among the molecules that may be identified by this method are polypeptides that exhibit multivalent binding; polypeptides that bind tightly to one molecule but show no affinity for a second, closely related molecule; polypeptides that affect the functional activity of the target compound to which they bind; and enzymatic polypeptides.

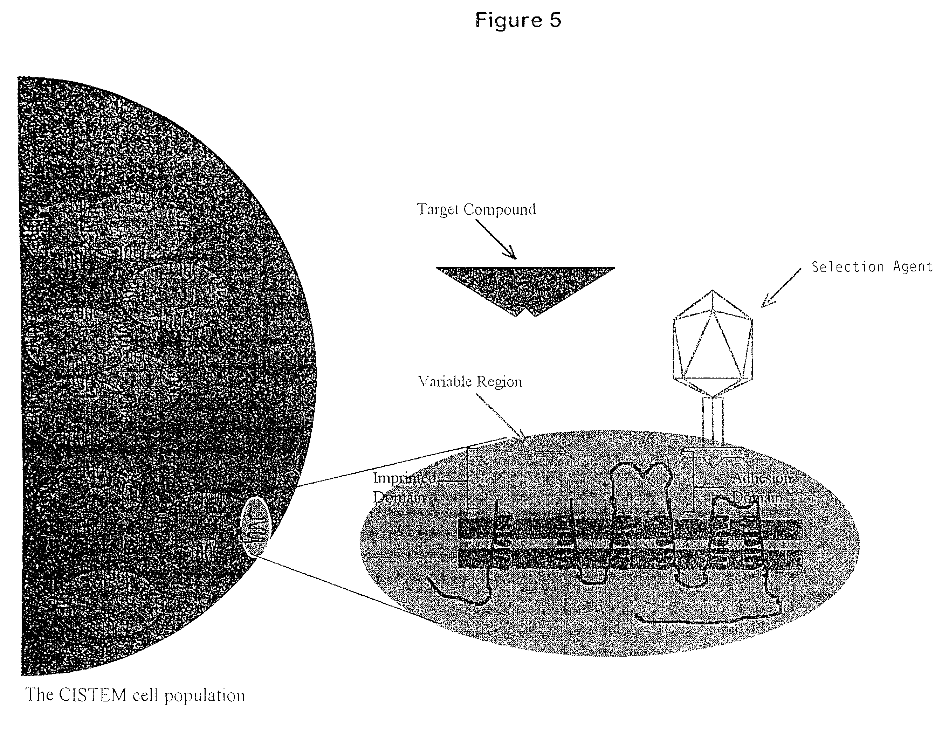

The invention is based on the interaction of three CISTEM components: (1) a genetically engineered population of prokaryotic or eukaryotic cells; (2) a target compound; and (3) a toxic agent (figure 5). On the surface of each cell in the population, there are two polypeptide domains involved in the operation of the CISTEM: the cellular domain carrying either a given (poly)peptide whose interaction with an unknown target compound is to be determined or, in the case of an unknown (poly)peptide, the imprinted domain, and the adhesion domain. The imprinted domain is highly variable, because the CISTEM cell population is engineered to display up to 10¹² distinct oligopeptides at the imprinted domain. (If the population is eukaryotic, the oligopeptides may be adorned by diverse oligosaccharides.) Neighboring the imprinted domain on each cell's surface is the adhesion domain, the structure to which the CISTEM's toxic agent must bind in order to exert its harmful effects upon the cell. The pathogenic agent may be an organism or molecule, and it may be fatal to cells or merely retardative of their growth, but in all cases the first step in the agent's destruction or retardation of a cell is binding between the agent and its adhesion domain. The two important domains - namely, the imprinted domain and the adhesion domain-are expressed in multiple copies on the surface of each cell, but they are always adjacent to each other: more specifically, they must be sufficiently close to each other that the target compound, when interacting with the imprinted domain, extends far enough over the adhesion domain that adhesion of the toxic agent is at least partially impeded.

The target compound is the molecule or set of molecules with which the polypeptide sequences identified by the CISTEM must interact in the prescribed manner in order to exhibit the properties or perform the functions desired of them. In other words, the CISTEM identifies polypeptides that interact with, e.g. bind to, and optionally also structurally or functionally alter, the target compound. A target compound can be a protein, carbohydrate, saccharide, glycoprotein, proteoglycan, hormone, receptor, antigen, antibody, pathogen, substrate, metabolite, transition state analog, cofactor, inhibitor, drug, dye, nutrient, small molecular compound, etc., without limitation. Each CISTEM places only one requirement on the target compound: it must be sufficiently large that it's attachment to the imprinted domain results in occlusion of the adjacent adhesion domain.

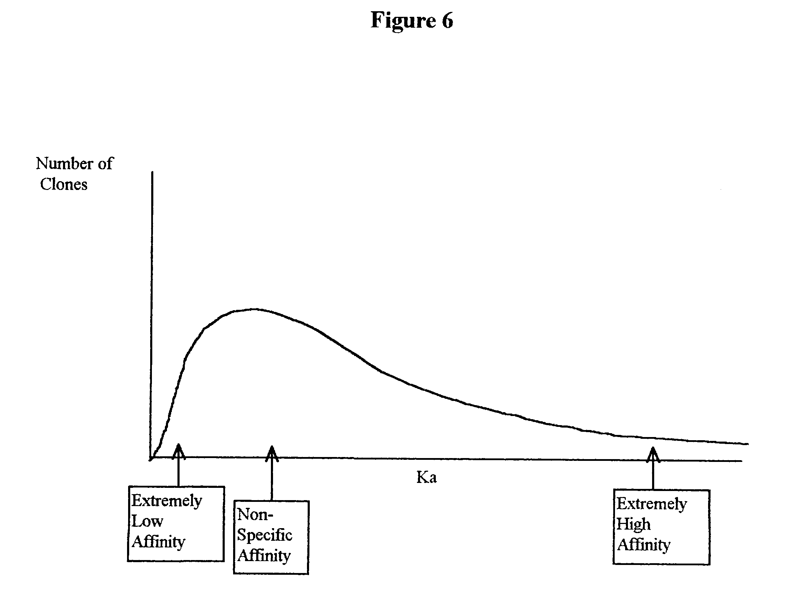

To assemble the most basic CISTEM (exemplified for the embodiment wherein the unknown interactant is the (poly)peptide) and initiate selection, the following steps are performed. First, CISTEM component (1), the cell population, is incubated with CISTEM component (2), a solution or suspension of the target compound. During exposure of the CISTEM cell population to the target compound, the structural diversity of the imprinted polypeptides' variable regions results in a wide range of interactions between imprinted domains and target molecules. That is, the distinct structures displayed at the imprinted domains of separate cells exhibit characteristic on-rates, off-rates, and dissociation constants in their binding reactions with the target (figure 6). After or during incubation, CISTEM component (3) is introduced: a population of bacteriocin, bacteriophage, virus, or toxin particles is added to the cells and target compound. With this step, assembly of the basic CISTEM is completed, and selection commences.

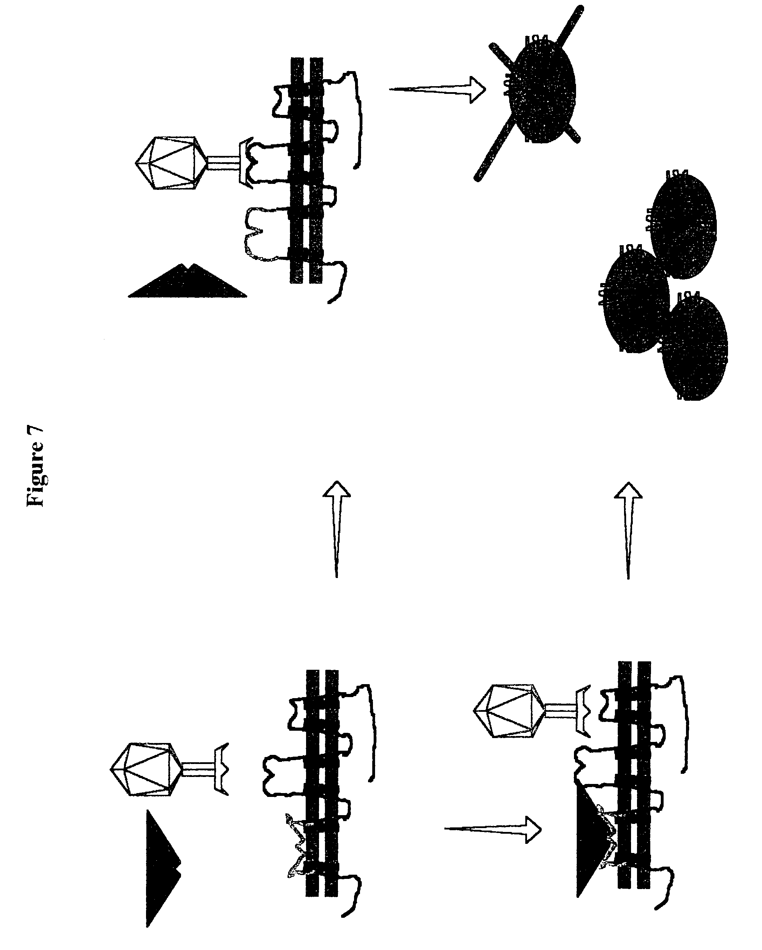

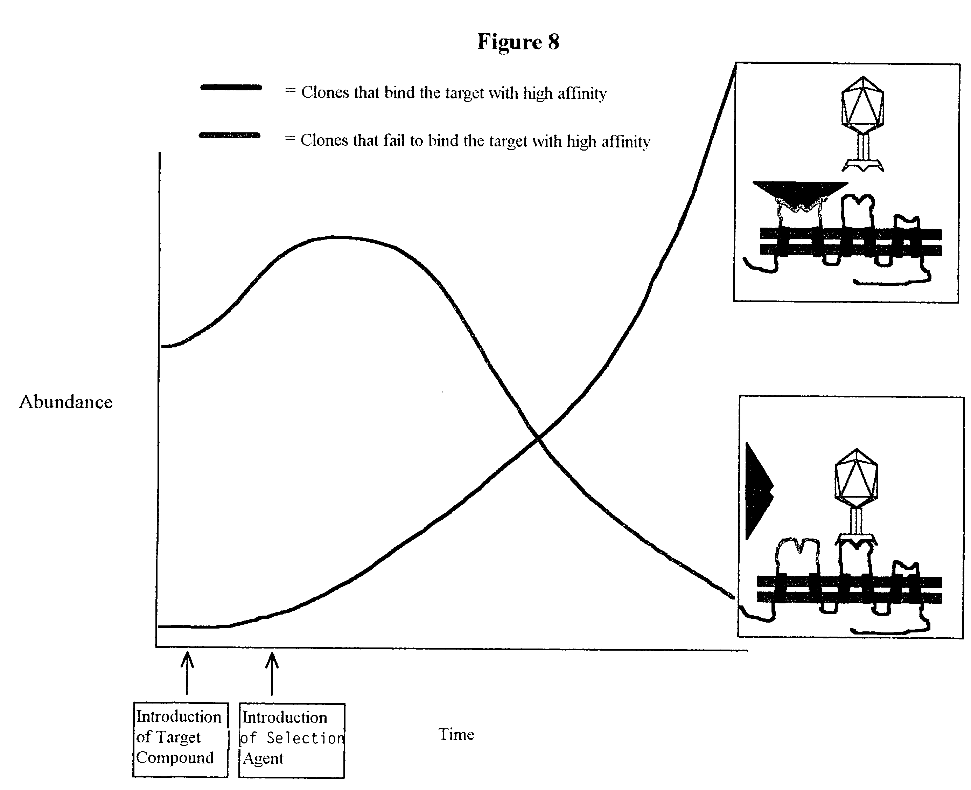

Interaction among the three CISTEM components confers a selective advantage upon cells whose imprinted domains bind the target compound (figure 7). This takes place as follows. As was stressed above, the imprinted domain (in the following, when describing structural features of the invention, the term "imprinted domain" is also used for the domain carrying a known (poly)peptide of interest whose interaction with an unknown target compound is to be determined. Hence, the term "imprinted polypeptide" may also denote proteins carrying such known (poly)peptide of interest) and the toxic agent's adhesion domain are immediate neighbors on the cell surface. Consequently, when the target compound binds to a structure located at the imprinted domain, the compound overlaps the surface of the adjacent adhesion domain. This overlap interferes with the toxic agent's attachment to the adhesion domain, thus protecting the cell, and allowing it to continue dividing (figure 7). By contrast, cells whose imprinted domains do not bind the target compound with adequate affinity remain unprotected, and are therefore killed or retarded by the toxic agent (figure 7). Thus, each selected clone increases in frequency and in number as it continues to divide, while unprotected clones are afflicted by the toxic agent. From the cell population displaying a great diversity of structures at the imprinted domain, the CISTEM selects and amplifies clones expressing structures that bind the target compound (figure 8).

Modifications and elaborations of the basic CISTEM permit selection for a prescribed affinity or nature of interaction of the imprinted domain with the target. Multivalent binding may be selected, as may highly specific binding, binding at predetermined sites, or binding by specified structural motifs. Moreover, CISTEMs may be extended to select not only for binding, but also for functional activity; these extended CISTEMs may be used to create enzymes and other functionally active molecules.

### DETAILED DESCRIPTION OF THE INVENTION

### i. INTRODUCTION

This invention provides a method for determining the interaction of a (poly)peptide and a target compound, wherein one of the interactants is unknown, thereby identifying (poly)peptides that exhibit prescribed properties, such as high affinity for a predetermined target compound, enzymatic activity, or the capacity to alter a target compound's activity, or identifying unknown target compounds that exert prescribe effects upon a known (poly)peptide of interest. The method is based on a convergence of three distinct areas of biological research: i. the study of prokaryotic and eukaryotic cellular membranes and their complex constituents; ii. the study of the evolution of microbial populations under the selective pressures of toxins, bacteriocins, or viral parasites; and iii. the design and screening of oligonucleotide-encoded combinatorial libraries of polypeptides.

The embodiments of the invention are called CISTEMs, for Cellular Imprinting by Selection: Teleological Evolution of Molecules. The next section (V.ii) describes the basic structure and function of CISTEMs. This basic function involves the interaction of three "CISTEM components", and in the following sections (V.iii - V.v), each component is considered in greater detail: the proceedures for properly manipulating each component and integrating it into a whole CISTEM are described in full or referenced. The final section (V.vi) describes modifications and elaborations of the invention that permit several significant extensions of its capabilities.

### ii. BASIC STRUCTURE OF THE INVENTION

The invention consists of three components. They are: (1) an engineered population of cells; (2) a target compound; and (3) a toxic agent (figure 5). The CISTEM cell population may be prokaryotic or eukaryotic. In case the interactant is the (poly)peptide of interest this population, one extracellular domain (the "imprinted domain") of an outer membrane polypeptide (the "imprinted polypeptide") is engineered to exhibit highly variable peptide sequences. If the CISTEM cell population is eukaryotic, glycosylation sites within the imprinted domain are adorned by variable complex saccharides. High sequence variability at the imprinted domain is achieved through the insertion of variable oligonucleotides at the sites in the polypeptide gene that correspond to translated regions in which variation is desired. Techniques for inserting a gene for an exogenous oligopeptide at a targeted site in an outer membane protein gene were described in the background (III.iv.a), and an extension of these techniques, which allows the insertion of variable oligonucleotides (10⁵ to 10¹² different sequences, instead of a single, stereotyped nucleotide sequence), will be described in detail below (V.iv.c).

As was described in the background (III.iv), many pathogenic agents can kill or retard the growth of cells only if they can first bind to a specific domain on the cell surface. A CISTEM's toxic agent binds to an adhesion domain that is located immediately adjacent to the imprinted domain on the surface of the cells in the CISTEM population (figure 5). The adjacent extracellular domains in most of the outer membrane proteins used in this invention are separated by 3 - 30 Å. (The approximate inter-loop distance for each of the imprinted polypeptides that are mentioned in this document as specific examples can be found in the literature (each of the following references estimates the interloop distance for one or more of the polypeptides mentioned here: 4, 23, 28, 38, 39, 41, 76, 105, 126, 127, 141).) In the preferred embodiments of this invention, which are described in greater detail below, this arrangement of domains is the result of carefully choosing an imprinted polypeptide and a toxic agent such that the imprinted domain is immediately adjacent to the adhesion domain. (As was stressed in the background (III.v), almost every outer membrane polypeptide serves as the receptor for multiple toxic agents, each of which utilizes a distinctive adhesion domain.) However, in other embodiments of this invention, outer membrane proteins are genetically altered in order to construct a hybrid polypeptide in which the imprinted domain is placed beside the adhesion domain. The techniques for engineering outer membrane proteins, including the fusion of two separate polypeptides to form a chimeric polytopic polypeptide, were described in the background (III.iv.c); their specific applications to embodiments of this invention are described below (V.iv).

The target compound is any molecule or set of multiple, distinct molecules of interest to which the polypeptide sequences identified by this invention must bind in order to perform the functions required of them. In other words, this invention identifies polypeptide sequences that bind the target compound, or some subset of molecules included in the target compound, and this binding event is necessary to the functions to which the polypeptides are ultimately applied, such as enzymatic activity or alteration of the target compound's biological activity. In the application of this invention to the analysis of molecular interactions, the target compound is a known compound of interest that is thought to have important interactions with unknown polypeptides. Identification of these unknown polypeptides is assisted by this invention's identification of sequences with which the target compound binds. In epitope mapping, the target compound is a molecule of interest (not necessarily a polypeptide), which is known to be involved in an interaction with a polypeptide of interest. By identifying polypeptide sequences that bind the target, this invention assists in delineating the regions of the known polypeptide that are directly involved in its interaction with the target compound. For each of the three areas of applications that have been mentioned here-identification of sequences with useful functions; interaction analysis; and epitope mapping-specific target compounds are described in the next section (V.iii).

The third component of the invention is the toxic agent. The most important requirement of the toxic agent has already been stated: the agent's effects upon cells must be entirely contingent upon the agent's ability to bind to an adhesion domain that is adjacent to the imprinted domain. Other attributes of the toxic agent, e.g. an pathogenic agent, such as virulence and concentration, may be altered, if necessary, by common laboratory procedures. These attributes and their adjustment are described in the section on toxic agents (V.v).

There are two steps in the execution of selection for polypeptide sequences that bind the target compound. In most embodiments of this invention, these steps are performed sequentially, though they may in some cases be simultaneous. First, the CISTEM cell population, component (1) of the invention, is incubated with a suspension or solution of the target compound, component (2) of the invention. The solution of incubation is a standard medium for cell growth, described in more detail in the section on cell lines and media (V.iv.g). During incubation, the structural diversity of the variable regions results in a wide range of binding interactions between imprinted domains and target molecules: the distinct structures displayed at the imprinted domains of separate cells exhibit characteristic on-rates, off-rates, and dissociation constants in their binding reactions with the target (figure 6). Incubation continues until the binding between imprinted domains and target compounds approaches equilibrium levels. For most embodiments of the invention, one hour is sufficient. In certain embodiments of the invention, the period of incubation is cut short in order to favor polypeptides that exhibit not only a high affinity for the target, but also exceptionally high on-rates; these CISTEMs are described in the section on elaborations of the invention (V.vi).

After incubation, the toxic agent, component (3) of the invention, is added to the suspension of cells and target compound. As is described in the section on toxic agents (V.v), exposure of the cell population is performed by a method appropriate to the agent. For example, if the agent is a bacteriophage, the suspension of cells and target compound is exposed to classical phage infection. Alternatively, if the agent is a colicin, exposure of the cell population to the agent is performed simply by adding the colicin to the medium in which the cells are incubated with the target compound. When toxic agent in the form of a pathogenic agent is a reproducing organism, such as a bacteriophage, it may be necessary to stop its action after a period of exposure. The appropriate concentration of pathogenic agent, period of exposure, and procedures for halting the action of the agent are described in the section on toxic agents (V.v).

Addition of the toxic agent to the suspension of cells and target compound completes assembly of the CISTEM, and selection commences. Interaction among the three CISTEM components confers a selective advantage upon cells whose imprinted domains bind the target compound: when a structure located at the imprinted domain binds the target compound, the compound overlaps the surface of the adjacent adhesion domain. Because the distance between adjacent extracellular domains of the polypeptides used in this invention is only 30-300nm (3-30Å)even a small peptide hormone is a sufficiently large target to reach from the imprinted domain, where it is bound, to overlap the abutting adhesion domain. This overlap interferes with the toxic agent's attachment, protecting the cell and allowing it to multiply (figure 7). Cells whose imprinted domains do not bind the target compound with adequate affinity remain unprotected, and, consequently, they are killed or retarded by the pathogenic (toxic) agent (figure 7).

Each cell the CISTEM population expresses multiple copies of its imprinted polypeptide, and binding is a dynamic process balancing on-rate and off-rate. Therefore even when a cell's imprinted polypeptide exhibits high affinity for the target compound, some copies of the imprinted polypeptide are left unprotected some of the time. Consequently, every cell in a CISTEM population, no matter how high its affinity for the target compound, has some probability of being hit by a toxic agent. Nonetheless, this probability is lower when a cell's affinity for the target compound is higher. Furthermore, because each clone in a CISTEM is present in multiple copies (the CISTEM cell population is redundant) the probability that a single cell will be hit is also the fraction of that cell's clonal population that will be hit, and the differential elimination of clonal populations is the very basis of selection. Thus, each selected clone increases in frequency and in number as it continues to divide, while unprotected clones are destroyed. In short, the CISTEM selects and amplifies clones expressing structures that bind the target compound (figure 8).

### iii. THE TARGET COMPOUND

The target compound can be a protein, carbohydrate, saccharide, glycoprotein, proteoglycan, hormone, receptor, antigen, antibody, pathogen, substrate, metabolite, transition state analog, cofactor, inhibitor, drug, dye, nutrient, etc., without limitation. The effect of the target compound relevant for the invention is that, due to its size, it extends, upon interaction with the imprinted domain, from the imprinted domain to the adhesion domain. This is 30-300nm (3-30Å) for most imprinted polypeptides imprinted by this invention. Alternatively, independent of its size, the interaction between the target compound and the imprinted domain may lead to a conformational and/or chemical change of the variable domain and/or the adhesion domain which prevents the toxic agent from attachment. The target compounds, their source, preparation, and use in CISTEMs with particular applications are described in this section. While the concentration of target compound in a CISTEM is also important to its function, this concentration depends on the nature of other CISTEM components, and is therefore discussed in a later section (V.v).

In CISTEMs designed to imprint polypeptides to serve as highly specific markers or partitioning agents in diagnostic assays, the target compound is simply the molecule that is to be tagged and/or partitioned by the polypeptide. For example, in an embodiment of the invention that is used in the design of a diagnostic polypeptide that binds tightly and specifically to Human Immunodeficiency Virus (HIV), the target compound is HIV gpl20, the extracellular subunit of a major HIV glycoprotein (114). The production of recombinant gp120 in a soluble form that is suitable for use in CISTEMs is described in the literature (59, 66). Another embodiment of the invention is used to design a diagnostic polypeptide for the direct detection of a different virus, Human T-cell Leukemia Virus type I (HTLV-1); here the target compound is the HTLV-1 surface glycoprotein gp46 (35). The production of authentic, glycosylated HTLV-1 gp46 in a recombinant vaccinia virus/T7 polymerase sytem is described in the literature (9). Diagnostic assays for detecting human viral infection often bind not to the virus itself, but rather to the antibodies raised against the infectious agent. One preferred embodiment of this invention imprints a polypeptide to exhibit high affinity for a monoclonal antibody, MAbT7. The target compound in this CISTEM, MAbT7 itself, is commercially available (Novagene), and is described in the literature (43).

The invention is also used to imprint polypeptides to serve as antagonists, agonists, or modulators of the functional action of biologically active molecules. When this invention is used to imprint a polypeptide to serve as an antagonist, the target compound may be either of the molecules involved in the interaction to be antagonized. HIV gp120 is the viral glycoprotein that binds to the CD4 receptor, and thus mediates viral invasion of the human cell (114). The embodiment of this invention that imprints a polypeptide (ICAM-1, which is described in III.v.c) to bind specifically to HIVgp120, provides a polypeptide that could serve as an antagonist of the interaction between the viral glycoprotein and its host cell receptor.

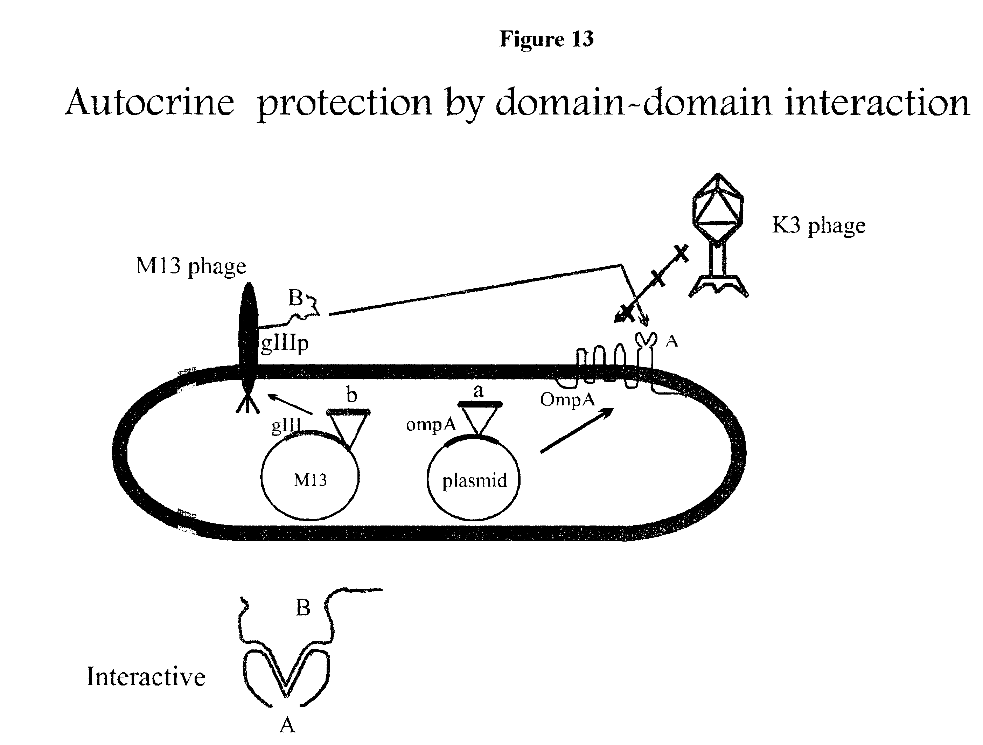

The polypeptides selected by a CISTEM are more likely to functionally antagonize a given molecular interaction when the CISTEM's target compound is pared down, so far as is possible, to one interactant's active site. Structures on either interactant that are distinct and topologically distant from the active site are less promising as target compounds because a polypeptide that binds such structures may not interfere with the normal interaction of the active molecules. For example, one embodiment of this invention imprints a polypeptide (*E. coli* OmpA, which is depicted in figure 13, and described further in III.iv.a, III.v.b, and VI) to function as an antagonist of the interaction between the eukaryotic cell-signalling proteins Ras and Raf (119). (Activation of Raf, a protein kinase, by the immediately upstream protein, Ras, (169) initiates pathways leading to the phosphorylation of transcription factors, the stimulation of numerous genes (100), and, ultimately, to cell proliferation and differentiation (124). Constitutively active oncogenic mutants of Ras or Raf occur in a large number of human tumors (19, 57).) In this CISTEM, the target compound is the domain of Raf(amino acids 51-131) that is known to be directly involved in binding to Ras (Raf's so-called Ras-binding domain, or Raf-RBD) (18). The construction of a recombinant expression vector for Raf-RBD, expression in E. coli, and purification of the protein are described in the literature (94). Using Raf-RBD, rather than Raf in its entirety, as the target compound ensures that the polypeptides selected by the CISTEM bind to precisely that domain of Raf which interacts with Ras, rather than to Raf epitopes that are extranneous to the important cell-signalling interaction.

When this invention is used to imprint polypeptides to serve as agonists, rather than antagonists, of a receptor or biological pathway, the target compound is the receptor or biomolecule that the imprinted polypeptide is intended to activate. For example, in an embodiment of this invention that imprints a polypeptide (*E. coli* OmpA) to mimic interleukin 6 and function as an agonist of the interleukin 6 receptor (IL-6R), the target compound is a solubilized form of IL-6R: the expression of soluble human IL-6R in CHO cells, and the protein's purification, are described in the literature (176). After this CISTEM selects polypeptides with affinity for IL-6R, a secondary screen is used to isolate polypeptides whose binding to the receptor results in transduction of the signal that is normally initiated by IL-6. This secondary screen is described in the section on extensions of the basic CISTEM (V.vi).

When this invention is used to create agonists, the target compound is pared, so far as is possible, to the active site of the receptor or biomolecule that is to be agonized. This is done because removal of extraneous epitopes from the target compound increases the probability that an imprinted polypeptide that binds the target will also be active as an agonist.

In another example of this invention's application to the creation of agonists, a polypeptide, such as *E. coli* OmpA, is imprinted to serve as a mimic of certain microbial antigens, and thus provoke a specific immune response. In these embodiments of the invention, the target compound is composed of more than one molecule: multiple monoclonal antibodies or polyclonal antisera derived from individuals exposed to a specific antigen, such as meningicoccal Opa proteins (1). This composite target is used as an impression-a "negative", or "footprint", of the antigen-and from this footprint *it casts* a positive likeness of the antigen: when a polypeptide (in this case, OmpA) is imprinted to bind the entire set of antibodies composing the sera raised against an antigen, then this imprinted polypeptide is likely to elicit an immune response very similar to the one provoked by the antigen (136). Antibodies suitable for use as the target compound in a CISTEM for imprinting *E. coli* OmpA to mimic Neisserial protein Opa 5c include mAb B306, mAb A222/5b, and others described in the literature (2, 130).

In order to create catalysts of chemical reactions, such as ester hydrolysis, polypeptides are imprinted to bind to transition state analogs. The preparation and structure of these transition state analogs are described in the literature (40, 162). In one embodiment of this invention, a phosphonate transition-state analog (structure and preparation described in 40) is used as the target compound for imprinting *E. coli* OmpA to function as a catalyst of the hydrolysis of p-nitrophenyl esters.

In other embodiments of this invention that are used to create polypeptides with specific enzymatic activity, the target compound is not a transition state analog, but rather a modified substrate. The target is a molecule that is structurally very similar, but not identical, to the substrate upon which the imprinted polypeptide will ultimately act. The reason for using a slightly modified structure, rather than the substrate itself, is the following: if the substrate itself were used as the target compound, imprinted polypeptides that not only bound the substrate, but also performed the desired enzymatic activity, would alter the substrate and then release it, rendering the underlying cell vulnerable to the toxic agent. Thus, if the substrate itself were used as the target compound, the CISTEM would select against clones expressing polypeptides with the desired enzymatic activity. Therefore, the target compound must be an effective structural mimic of the substrate (i.e. polypeptides that bind the target must also be able to bind the substrate), but it must be invulnerable to the prescribed enzymatic activity (i.e. polypeptides that act enzymatically on the substrate must be unable to act on the target compound).

For example, in an embodiment of this invention that imprints *E. coli* OmpA to function as a sequence-specific protease that hydrolyzes the bond between two methionines, the target compound is the oligopeptide NH2-R1-R2-R3-M1=M2-R4-R5-COOH. M1 and M2 represent methionine residues; the bond between them, which is represented by "=", is a nonhydrolyzable methylene-group bond. Normal amide bonds are repesented by "-". R1...5 are variable residues, which are randomly chosen from the 20 amino acids. These residues are varied so that only imprinted polypeptides that bind to the consistent M1=M2 motif of the target compound are selected by the CISTEM. Imprinted polypeptides that show high affinity for a different peptide motif bind at most only a tiny fraction of the target polypeptides, and are therefore left vulnerable to the toxic agent. Several methods for the synthesis of this oligopeptide target compound are described in the literature (99).

After the CISTEM has selected clones expressing imprinted polypeptides that bind the modified substrate, a secondary screen is used to isolate imprinted polypeptides that not only bind the target, but also perform the desired enzymatic reaction. Secondary screens, including the one used in the CISTEM that imprints polypeptides to hydrolize the bond between two methionines, are described in the section on elaborations of the CISTEM (V.vi).

In addition to the creation of functionally active polypeptides, such as antagonists and enzymes, this invention's applications include interaction analysis (current techniques reviewed in 138, exemplified in 179, and described above in III.vi). By screening a diverse polypeptide library for sequences that interact specifically with a target compound of significant interest, this invention assists in the identification of the compound's native interactants. This capacity of the invention is illustrated by the CISTEM that imprints *E. coli* OmpA to bind to Raf-RBD (see above for a more detailed description of this target compound). Although the structural interaction between Ras and Raf-RBD has been well-characterized through crystallography and mutational analysis (18), the activation of Raf by Ras is a complex process that is not yet completely understood, and probably involves interaction with other molecules. The CISTEM that imprints OmpA to bind Raf-RBD assists in the identification of polypeptides involved in Raf activation. The CISTEM selects multiple peptide sequences exhibiting affinity for the target, Raf-RBD. These sequences, or the oligonucleotides encoding them, are then used in commonly practiced procedures for searching sequence libraries and/or cDNA libraries from Ras/Raf expressing cells.

This invention is also used for epitope mapping (32, 51, 138), serving to elucidate the surface of structural interaction between two molecules. In CISTEMs for epitope mapping, the target compound is one of the two molecules whose interaction is to be mapped. Usually it is the interactant that is most easily prepared in soluble form that serves as the target compound. Thus, if one of the two interactants is a membrane protein, it is often the other molecule that serves as the target. In a CISTEM used to map the epitopes of interaction between Ras and Raf-RBD, Raf-RBD serves as the target compound. As noted above, it is readily prepared in soluble form (94). Thus, one of the interacting proteins is dissected into multiple smaller domains which may be probed in routine assays for their capability to bind to the other interactant.

In certain embodiments of this invention, multiple separate molecules compose the target compound. Each molecule must meet the CISTEM's size requirement and must be present in the CISTEM at a concentration sufficient to confer protection upon clones that bind to it with high affinity. (Selection by CISTEMs with multiple distinct target molecules strongly favors binding by imprinted domains to multiple distinct molecules, because the effective concentration of target compound is significantly higher for a cell whose imprinted domain is capable of binding either of two distinct molecules than it is for a cell whose imprinted domain is capable of binding only one molecule.) When the target compound is a library of molecules, the CISTEM assists in the identification of interacting pairs of molecules: polypeptides expressed in the imprinted domain are selected for their affinity for particular members of the library of molecules that composes the target compound. Those cells that express an imprinted polypeptide that binds to one or more molecules in the target compound are selected, while cells expressing an imprinted polypeptide that fails to bind any component of the target compound are eliminated by the CISTEM's toxic agent. In certain embodiments of this invention, which are described below (V.vi), the target compound consists of a library of polypeptides or phage-displayed polypeptides that are secreted by the CISTEM cell population itself.

In an embodiment of the invention, the target compound is the interaction partner that is to be identified due to its interaction with a given (poly)peptide of interest, e.g. a section of a naturally occuring cell surface receptor which is displayed in the extracellular domain. In this case, the target compound is the unknown interactant. In this example, the cells presenting the receptor domain of interest may be incubated with chemical compounds from a pool of substances. Surviving cells are indicators that the compound that has interacted with these cells is a candidate substance for interaction with the receptor domain.
In a further embodiment, the invention is directed to a method wherein the imprinted domain binds in an autocrine fashion. In this embodiment, the target compound is secreted by the cell. Such an application of CISTEM permits to select cells from a population of cells that express various domains for interaction with a particular target secreted by the cells or to select cells from a population of cells that secret various targets and present a particular domain for interaction with a particular target. In both cases cells from the population are selected that are protected against the action of the toxic agent by the proper match of the secreted target compound and the imprinted domain displayed by the same cell.

### iv. THE IMPRINTED POLYPEPTIDE

As was noted above (II), the term "polypeptide" is used in this document to comprise proteins, glycoproteins, and proteoglycans. To be imprinted by this invention, a polypeptide must comprise at least one extracellular domain that accepts exogenous oligopeptide insertions without disturbing translation or export of the polypeptide to the cell surface. This capacity to accept insertions has been observed in numerous secreted polypeptides, as well as in each one of the dozens of bacterial, yeast, and mammalian membrane polypeptides that have been topologically mapped by gene fusion techniques. Several of these polypeptides, such as *E. coli* OmpA, *E. coli* LamB, and mammalian ICAM-1, their properties, and their capacities to exhibit exogenous sequences in extracellular domains, are described in more detail in the background (III.iv.a).

The membrane polypeptide may be autologous or heterologous for the cell.

Preferably, the membrane polypeptide is heterologous. By use of a cell population lacking the autologous protein, a competition between a binding site which may be present in the naturally occuring membrane protein and the (poly)peptide sequence of interest for interaction with the target compound is excluded. In case the cell contains the autologous membrane polypeptide but otherwise has the desired properties for its use in the present invention, the gene encoding the membrane protein is inactivated, e.g. by deletion. Standard molecular methods are available to the person skilled in the art.

Exogenous insertions at an extracellular domain are involved in two stages of each CISTEM. First, an oligopeptide tag is inserted into the imprinted domain, and cell populations exhibiting this tag are used in preliminary trials to optimize the CISTEM. The oligopeptide tag, its insertion into the imprinted domain, and construction of cell populations exhibiting the tag, are all described in this section. Second, once preliminary trials with the tagged cell population have been performed, the actual CISTEM cell population is constructed: in this population, variable peptide sequences, rather than an oligopeptide tag, are inserted into the imprinted domain. The steps in the construction of CISTEM cell populations are also described here.

### a) Cloning of the Imprinted Polypeptide Gene onto a Suitable Vector

By conventional methods described in the literature (146), the gene encoding the imprinted polypeptide is cloned onto an appropriate vector. The vector's level of expression is important to CISTEM function. In most CISTEM cell populations, roughly 1000 copies of the imprinted polypeptide are exhibited per cell. However, as is described below, in some cases expression is adjusted to achieve proper CISTEM selection. In preferred embodiments of this invention, regulation of the level of expression of the imprinted polypeptide is made possible through the use of a vector comprising an inducible promoter, such as the prokaryotic lac regulatory elements (76), which are inducible with isopropyl-β-D-thiogalactopyranoside (IPTG), or the eukaryotic interferon promoter (67). The vector also contains a selectable marker, such as a gene for antibiotic resistance, to facillitate transformation (selectable markers and their use in cloning vectors are extensively described in the literature, including 146 and 109).

For prokaryotic proteins, common expression plasmids *are* used, and the polypeptides imprinted in preferred embodiments of this invention have already been placed on suitable plasmids. For example, on pEV218 (76), the plasmid used in a particularly preferred embodiment of this invention, *E. coli* OmpA (*E. coli* Outer membrane protein A is described further in III.iv.a, III.v.b, and VI) has been placed under the inducible control of the lac regulatory elements residing on the common, high-copy-number plasmid pUC9 (74). Expression of ompA on pEV218 is further controlled by the presence of a TAG stop codon corrosponding to amino acid 7 of the polypeptide: when the plasmid is used to transform an amber-supressing host, such as *E. coli* UH203 (*sup*F), expression is regulated not only by induction with IPTG, but also by the concentration of the suppresor tRNA. The plasmid also comprises a gene for ampicillin resistance. Similarly, on pSB1649 (28), *E. coli lam*B is regulated by the inducible *lac* promoter on the common plasmid pACl. For eukaryotic polypeptides, common viral vectors are suitable, and many polypeptides have been cloned into appropriate vectors. For example, for the polytopic mammalian glycoprotein (ICAM-1) (Intercellular Adhesion Molecule-1 is described further in III.v.c), which is imprinted in a preferred eukaryotic embodiment of this invention, the CDM8 cDNA expression vector pCDl.8 may be used in conjunction with the COS cell expression system (148, 159).

The vectors bearing the gene for the imprinted polypeptide are replicated and isolated. They may be replicated by a cell population (146), by PCR techniques (146), or by any other commonly employed methods described in the literature. In preferred embodiments of the invention, 10⁸ to 10¹⁰ copies of the vector are isolated. The exact number depends on the number of distinct clones that will compose the CISTEM cell population, and on the technique used in step c, below, to insert the variable oligonucleotides into the vectors.

### b) Insertion of an Oligopeptide Tag into the Imprinted Domain

The gene for a well-characterized oligopeptide tag is inserted into the vector at a genetic site corresponding to the imprinted domain of the polypeptide. Alternatively, the oligonucleotide tag may replace a nucleotide sequence of the naturally occuring sequnce. Replacement is accomplished by excising a natural coding region and by inserting the oligonucleotide tag in place thereof. The tag may be any peptide sequence that is known to bind with high affinity and specificity to a ligand that is commercially available or easily produced in the laboratory by techniques described in the literature. One example of a commercially available tag/ligand pair is the T7 sequence (*Met-Ala-Ser-Met-Thr-Gly-Gly-Gln-Gln-Met-Gly*)*,* which binds to the T7 antibody (available from Novagene), as is described in the literature (43, Novagene's TB15 protocol). An example of a tag/ligand pair that is described in the literature is amino acid sequence 36-41 of the eukaryotic polypeptide Ras and the Ras-binding domain (amino acid sequence 51-131) of Raf (18). The production and purification of Ras-RBD by commonly practiced techniques is described in the literature (94). Many other tag/ligand pairs, such as hexapeptides that bind to specific antibodies, are also described in the literature (e.g. 58 and 147). To insert the gene for the oligopeptide tag at a genetic site encoding the imprinted domain, any common technique for inserting an oligonucleotide at a specific site in a vector can be used (e.g 23 and 146). Several such techniques are referenced above (III.iv.a), and one preferred procedure is described here in more detail.

Many outer membrane polypeptides have been topologically mapped, and these maps are availiable in the literature (III.iv.a). Using a topological map of a membrane polypeptide's sequence, two restriction endonuclease sites (site R1 and site R2) each of which is unique in the vector and is located in the genetic region coding for the imprinted domain, are identified. If two unique restriction sites do not already exist at the desired site of insertion, they may be engineered using any one of a number of procedures described in the literature (examples of unique restriction sites being engineered at a desired site of insertion are described in 28, 29, 141, and 146). The vector is cleaved at the two designated sites by digestion with restriction endonucleases R1 and R2, following the protocols and under the conditions specified by the suppliers of the enzymes. This yields a linearized vector with non-complementary sticky ends (RS1 and RS2) and an excized fragment. The linearized vector is isolated by standard electrophoretic size fractionation (146).

An oligonucleotide template comprising the gene for the tag is synthesized using conventional technology, such as phosphoramitide chemistry, or can be obtained from commercial laboratories offering custom oligonucleotides (methods for synthesizing single-stranded oligonucleotides are described and referenced in 29 and 82). The oligonucleotide is synthesized to read 5'-P--R1--tag gene--R2--3', in which P is the primer sequence for a proof-reading polymerase, such as Vent® polymerase (New England Biolabs), R1 and R2 are the restriction endonuclease sites mentioned above, and --tag gene-- is the genetic sequence encoding the oligopeptide tag. (It may be necessary to include one or two extra-oligonucleotides flanking the tag gene so that the tag is translated in frame with respect to the imprinted polypeptide.) Using the polymerase whose primer is P, the oligonucleotide template is used to generate a double stranded oligonucleotide. This oligonucleotide is digested with restriction endonucleases R1 and R2 under conditions described by the enzyme suppliers, yielding an oligonucleotide with sticky ends RS1' and RS2'. This oligonucleotide, which has sticky-ends complementary to the linearized vector, is ligated into the vector by a common ligation procedure (146). The result is that the vector's gene for the imprinted polypeptide bears the oligopeptide tag at a genetic site corresponding to the imprinted domain.

An alternative procedure for the construction of the tag gene with sticky ends RS1' and RS2' is used in some preferred embodiments of this invention. In this procedure, two semi-complementary single stranded oligonucleotides are synthesized by conventional technology (29, 82) or acquired from a laboratory offering custom oligonucleotides. The two single stranded oligonucleotides are synthesized such that, when they are annealed, they form a double-stranded oligonucleotide with sequence RS1'--tag gene--RS2'. This double stranded oligonucleotide is then ligated into the linearized vector described above.

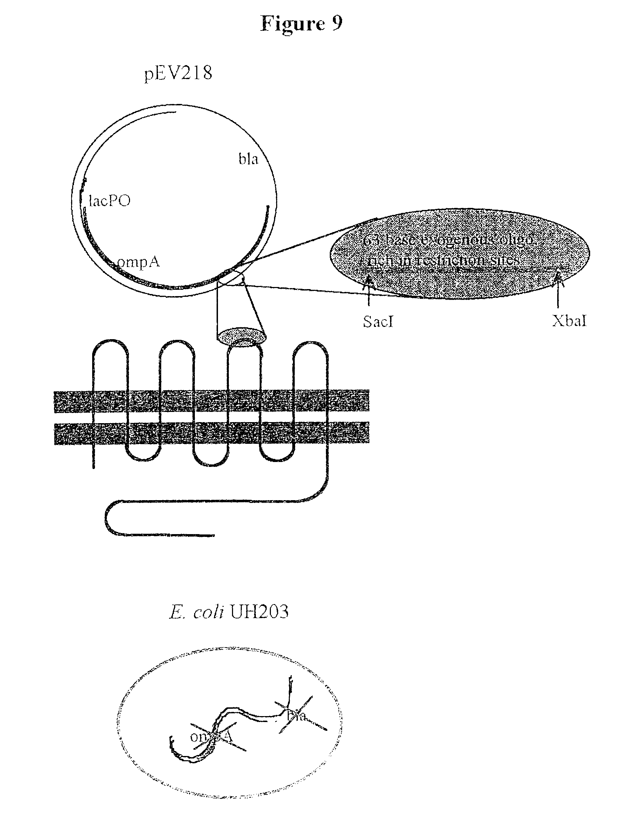

An example of this procedure is provided by a preferred embodiment of this invention, which involved the construction of an *E. coli* population displaying the T7 tag (figure 9) at loop IV of OmpA. In pEV218 (76), the ompA gene has been engineered to bear a string of restriction endonuclease sites at a genetic region corresponding to extracellular loopIV of the polypeptide (figure 9). This plasmid was digested with SacI (enzyme and reaction buffer from New England Biolabs) and XbaI (enzyme and reaction buffer from MBI Fermentas), yielding a fragment of the restriction site polylinker and the linearized plasmid. The linearized plasmid was isolated by standard gel electrophoresis (146). Two single stranded oligonucleotides were synthesized by conventional technology to read as follows:

Oligonucleotide 1:
5' - CG[ATGGCTAGCATGACTGGTGGACAGCAAATGGGT] - 3'

Oligonucleotide 2:
5' - CTAG[ACCCATTTGCTGTCCACCAGTCATGCTAGCCAT]CGAGCT - 3'

The sequence in brackets comprises the gene segment encoding the oligopeptide that is recognized by the T7 antibody. These two single-stranded oligonucleotides were annealed in MBI Fermentas Klenow buffer, forming the double stranded T7-tag gene segment with sticky ends complementary to the sticky ends on pEV218, linearized by SacI and XbaI, as described in the previous paragraph. When the double-stranded oligonucleotide was ligated into the linearized plasmid, the T7 tag gene segment was in frame with the ompA gene on pEV218. The new construct was designated pEV218T7.

After the tag gene has been inserted into the vector, the vector is used to transform a population of cells lacking a chromosomal copy of the gene for the imprinted polypeptide. Strains lacking chromosomal genes for outer membrane polypeptides are described in the literature (e.g., 28, 74, and 159), as well as below, in the section on cell lines and media (V.iv.g). Conventional methods of transformation (89, 109) are used, and transformants are selected by their expression of the vector-borne selectable marker, such as a gene for ampicillin resistance. In a particularly preferred embodiment of this invention, in which *E. coli* OmpA was engineered to display the T7 polypeptide tag at loopIV, pEV218T7 was used to transform *E. coli* strain UH203 (*lac, sup*F, *omp*A, *rec*A, *pro*AB, *rps*L) (74), according to a common method of electrotransformation, as follows.

25 ml of overnight culture of UH203 (Luria broth, 37°C) was suspended in 11 of LB. Cells were cultivated at 37°C, with shaking. When the culture reached OD₆₀₀= 0.5, the cells were chilled for 15 min. on ice, and then centrifuged in a cold rotor at 4000 x g for 15 min at 4°C. Supernatant was removed. Sediment was gentlry resuspended in 11 of ice-cold 10% glycerol and centrifuged as before. This washing procedure was repeated three times and cells were resuspended in 1 ml of ice-cold 10% glycerol. 42µl of the cell suspension was mixed with 2µl of DNA (pEV218T7 described above) solution (1pg dissolved in TE) and incubated 10 min on ice. 40µl of this suspension was pipetted to an electroporation cuvette (BIO-RAD Gene Pulser® with Pulse Controller® was used. Apparatus settings were as follows: 2,5 kV; 200Ω; 25µF; time constant = 4,9 ms. Immediately following electroporation, cells were washed in 600µl of SOC medium and transferred to a cultivation tube. Cells were then allowed to recover for 1 hour at 37°C in a shaking flask. Finally, cells were plated on LB plates with 100µg ampicillin/ml.

### c) Insertion of Variable Oligopeptides into the Imprinted Domain

There are several ways to generate the variable oligonucleotides that are inserted into the vector, and, ultimately, translated into the variable regions exhibited by the CISTEM cell population. To facilitate insertion into the vectors, the variable oligonucleotides must have conserved sequences at their termini, just as the tag gene described in the previous section was given defined termini RS1 and RS2. Because the nucleic acids composing SELEX libraries (162) also consist of random or nearly random sequences flanked by stereotyped termini, the methods used to generate SELEX libraries (these methods are reviewed and described in detail in 82) may also be used to construct the oligonucleotide inserts that are required here. For example, in preferred embodiments of the invention, variable oligonucleotides with defined sequences at their termini are easily synthesized using solid-phase phosphoramidite chemistry (29, 82): at defined terminal positions, only one of the four nucleotides is made available for addition to the strands being synthesized, whereas at variable positions, a mixture of multiple nucleotides is presented to the strands (82). Alternatively, end addition of bases, catalyzed by terminal transferase in the presence of nonlimiting concentrations of multiple nucleotides, can append a highly variable sequence to a defined terminal (82). Highly variable sequences may also be obtained by selecting fragments of defined length from digestions of natural cellular DNA or RNA preparations (146).

In a CISTEM for screening the polypeptides encoded by a cDNA library, the variable oligonucleotides are the cDNA library genes, with defined sequences ligated to the termini (82) in order to facilitate insertion of the genes into the vectors. The size of the cDNA library that is screened by such a CISTEM is in some embodiments of this invention reduced by limiting the screen only to genes of interest, as determined by comparison of the cDNA libray genes to sequences catalogued in genomic libraries. In a CISTEM for analyzing the interaction between a known polypeptide and a second known compound of interest, the variable oligonucleotides are sections of the gene encoding the known polypeptide. For example, in a preferred embodiment of this invention, in which the interaction between the eukaryotic cell-signalling proteins Ras and Raf is analyzed, the oligonucleotides inserted into pEV218 are sections of the sequence encoding Ras.

To prevent truncation of the imprinted polypeptide, stop codons are excluded from variable oligonucleotide inserts. This is achieved by synthesizing oligonucleotides to read D1(NNP)ₙD2, where D1 and D2 represent the defined sequences that facilitate insertion of oligonucleotides into vectors; n represents the number of triplet codons that compose the variable section of each oligonucleotide; N represents all four nucleotides, each with equal probability; and P represents a subset of nucleotides, each with equal probability. NNP encodes all 20 amino acids, but precludes the occurrence of stop codons recognized by the CISTEM cell population. Thus, when P is allowed to be G, C, or T, the stop codons TAA and TGA are excluded from the oligonucleotide inserts. The synthesis of oligonucleotides reading D1(NNP)ₙD2 by solid-phase phosphoramidite chemistry or by end-addition terminal transferase is described in the literature (28, 82). It is not necessary to preclude the occurrence of all three stop codons in inserted oligonucleotides, because the CISTEM cell population may be composed of stop codon-suppressing mutants, which are capable of translating one or more of the stop codons. Numerous suppressor strains are described in the literature (e.g. 28, 76).

When the imprinted polypeptide is a glycoprotein or proteoglycan, the inserted oligonucleotides may include consensus sequences for N-linked glycosylation (41). These are synthesized as defined sequences interrupting the variable sequence of each oligonucleotide. In CISTEMs that incorporate an element of structural design, variable oligonucleotide inserts may include a mixture of defined and variable positions, sequences coding for fixed motifs, or statistical biases toward certain sequences or structures.

The variable oligonucleotides are inserted at a prescribed site in the vectors constructed and amplified by the methods described in step a. Two preferred procedures for precisely targeted insertion are described here-one in which the number of oligonucleotides inserted into each vector is defined, and another in which the number of inserts is variable. The available procedures for inserting oligonucleotides at specific vector sites are certainly not limited to the two described here. In fact, any of the gene fusion techniques used to insert a gene coding for a reporter moiety into the gene for a membrane protein (III.iv.a) may be used to the same effect as the procedures that will be described.

The site of insertion in the vector is located such that oligonucleotides inserted there are translated as part of the imprinted domain. The site of insertion may be further specified (i.e. within the sequence encoding the imprinted domain) according to structural analysis or prescriptions for a particular nature of interaction between the imprinted domain and the target compound. To insert oligonucleotides, both of the procedures outlined here make use of restriction endonuclease sites that are unique in the vector, and are located at or near the prescribed site of insertion. If a unique restriction site does not already exist at the site of insertion, one may be engineered using any one of a number of procedures described in the literature (e.g. 28, 29, 141, and 146)

To insert one and only one variable oligonucleotide per vector, two distinct restriction sites, each unique in the vector, must be located such that they flank a short restriction fragment comprising the prescribed site of insertion. If the sticky ends left by cleaving the vector with one endonuclease (R1) at its unique site are RS1 and RS1', while cleaving with the other endonuclease (R2) at its unique site leaves sticky ends RS2 and RS2', then the variable oligonucleotides generated at step 3 are given defined and conserved sticky ends RS1 and RS2'. The vectors are digested by both restriction nucleases (R1 and R2), and the digestion products are size-fractionated to isolate the vectors, each of which has non-complementary sticky ends RS1' and RS2. The population of restriction-digested vectors is combined with a slightly smaller population of variable oligonucleotide inserts (vectors/insert ≈ 1.5 - 2.0), and ligation is allowed to occur. Because the sticky ends of the vectors are non-complementary to each other, each vector can neither ligate shut, nor ligate to other vectors. Similarly, because oligonucleotide inserts have non-complementary sticky ends RS1 and RS2', they can neither self-ligate into loops, nor ligate to each other to form concatamers of multiple oligonucleotides. The only ligation that can occur is the insertion of one variable oligonucleotide into each vector. After ligation has taken place, the vector population is size-fractionated to discard vectors that did not receive an insert. In the resultant vector population, each vector has one variable oligonucleotide at the prescribed site in the region encoding the imprinted domain.

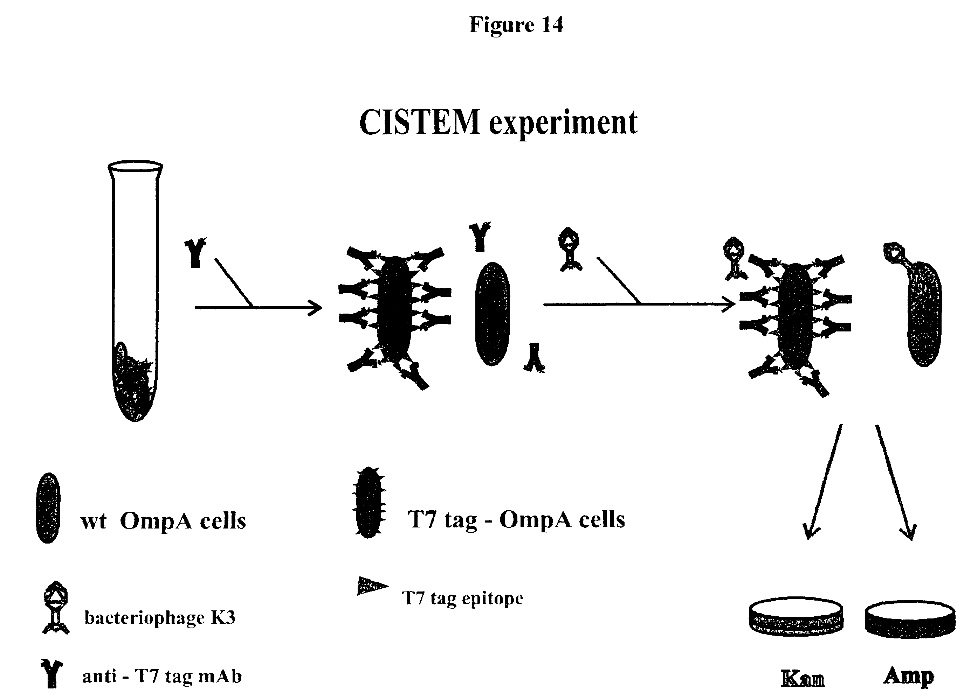

An example of this procedure for inserting one and only one variable oligonucleotide per vector is furnished by a preferred embodiment of this invention, in which a cell population is engineered to display variable oligopeptides at loop IV of *E. coli* ompA. In pEV218 (76), the ompA gene has been engineered to bear a polylinker rich in restriction endonuclease sites at a genetic region corresponding to extracellular loopIV of the polypeptide (figure 14). This plasmid is digested with SacI (enzyme and reaction buffer from New England Biolabs) and XbaI (enzyme and reaction buffer from MBI Fermentas), under conditions described by the suppliers, yielding a fragment of the restriction site polylinker and the linearized plasmid. The linearized plasmid is isolated by standard gel electrophoresis (146). Single stranded oligonucleotides are synthesized by conventional technology (29) to read 5'GGTCTAGA(VNN)ₙCGAGCTC3', in which N represents all four nucleotides, each with equal probability; and V represents C, G, or A, each with equal probability. Using common protocols described in the literature (29), a 5' primer and a proof reading polymerase, such as the Vent® polymerase, are used to generate double-stranded oligonucleotides from the single-stranded templates. These double-stranded, highly variable oligonucleotides are then digested with SacI and XbaI, under conditions described by the suppliers, yielding highly variable oligonucleotides with non-complementary sticky ends 5'-CTAG and AGCT-3'. The highly-variable oligonucleotides are then ligated into the linearized plasmid (MBI ligation buffer, conditions described by supplier); they ligate in only one oligo per plasmid, and in only one orientation. Conventional electrophoretic size fractionation (146) is used to isolate ligated plasmids containing an insert. The product of this proceedure is a population of plasmids (pEV218I) bearing, at the genetic site corresponding to loop IV of OmpA, a highly variable exogenous sequence free of TAA and TGA stop codons.

To construct a vector population in which each vector harbors between 0 and n variable oligonucleotides at a prescribed site, the following procedure may be used. First, a restriction site that is unique in the vector is located at the prescribed site of insertion, i.e., at a genetic site corresponding to the imprinted domain of the imprinted polypeptide. (If a unique restriction site does not already exist at the desired site, one may be engineered by conventional methods described in the literature, such as oligonucleotode-directed mutagenesis (e.g. 28 and 76). If the endonuclease (R) that cleaves at this unique site leaves complementary sticky ends RS and RS', then the variable oligonucleotides generated by the procedures described above are given defined and conserved complementary sticky ends RS and RS'. The vectors bearing the gene for the imprinted polypeptide are cleaved by R. The vectors are then combined with an excess of variable oligonucleotide inserts. Several distinct ligation events can occur. First, the vectors, which have complementary sticky ends RS and RS', can ligate to other vectors or self-ligate, closing without an oligonucleotide insert. Second, the variable oligonucleotides, which also have complementary sticky ends RS and RS' can i) self-ligate as a loop, ii) ligate into a vector with sticky ends RS and RS', iii) ligate to each other, forming a multi-oligonucleotide concatenation, which itself can perform ligations i, ii, or iii. After ligation, size fractionation can be used to discard all inserts that did not ligate into a vector and all vectors that did not receive inserts. This yields a population of vectors, each of which harbors between 1 and n variable oligonucleotides at the prescribed site in the gene encoding the imprinted polypeptide. A specific example of this method of constructing a vector population in which each vector harbors between 0 and n variable oligonucleotides at a prescribed site may be found in the literature (the construction of such a library of vectors is described in detail in 28 and 29).

The vectors bearing variable oligonucleotide inserts are used to transform a population of cells that lack a gene for the imprinted polypeptide. Conventional methods of transformation (89, 109) may be used. Transformants are selected by their expression of the vector-borne selectable marker, such as a gene for antibiotic resistance. (Efficient transformation of competent prokaryotic cells achieves up to 10⁹ transformants/ ml.) The product of transformation is a population of cells, each of which harbors a distinctive oligonucleotide insertion at a genetic site corresponding to a point in the imprinted domain. After transformation, the cell population is amplified by growth in a suitable medium (V.iv.g).

In a preferred embodiment of this invention, in which the CISTEM cell population is engineered to display variable oligopeptides at loop IV of *E. coli* OmpA, transformation is performed in the following manner. The variable plasmid population, pEV218I, whose construction was described above, is used to transform *E. coli* strain UH203 (*lac*, *sup*F, *omp*A, *rec*A, *pro*AB, *rps*L) (this strain, its propagation and its transformation, are described more completely in 74), according to a common method of transformation described in the literature (74, 76, 109). The UH203 population is grown in Luria broth supplemented with 100 µg Ap/ml, selecting for Ap resistance of transformants.

Expression of the imprinted polypeptide is induced with the compound appropriate to the vector's promoter. Thus, for example, in the preferred embodiment mentioned above, the population of UH203 transformed with pEV218I is induced with 1m IPTG). This yields a population of cells comprising as many clones as there were transformants in the previous step. Each clone displays a distinctive oligopeptide at the imprinted domain. If the imprinted polypeptide is a glycoprotein or a proteoglycan, glycosylation sites in the imprinted domain bear diverse and unpredictable oligosaccharides. (Commonly practiced procedures for verifying that an extracellular domain-in this case, the imprinted domain-has been glycosylated are described in the literature (62).) In short, the CISTEM cell population displays a vast combinatorial library of proteins, glycoproteins, or proteoglycans.

### d) Genetic Manipulations of the Imprinted Polypeptide

In a preferred embodiment, a CISTEM is is constructed wherein the imprinted polypeptide is a polytopic membrane protein in which the domain that accepts insertions is directly adjacent to the adhesion domain of a toxic agent suitable for use in the CISTEM. This preferred configuration is depicted in figure 5, and several of the polypeptide/toxic agent combinations that exhibit this configuration are listed in table 1, in the background (III.v), and in the literature (165). When these polypeptide/pathogen combinations, or others like them, are used, the CISTEM is assembled with a minimum of genetic manipulation to the imprinted polypeptide; the only necessary manipulation is the introduction of an oligopeptide tag, as well as variable peptide sequences, to the imprinted domain, as described above. However, the list of polypeptides that may be imprinted is by no means limited to polytopic proteins exhibiting adjacent adhesion and imprinted domains. To further expand the diversity of polypeptides that may be imprinted by CISTEMs, two genetic manipulations are employed.

When secreted proteins are imprinted, they are first converted to their glycolipid- or transmembrane-anchored isoforms. Because translocation of secreted proteins and membrane insertion of integral proteins are handled by the same cellular machinery (reviewed in 153, and above, in III.iii), many secreted proteins may be converted to membrane-bound isoforms simply by carboxyl-terminal attachment of a hydrophobic transmembrane span or a signal sequence for glycolipid addition. For example, the gene-fragment encoding the 37 carboxyl terminal amino acids of decay accelerating factor, a eukaryotic glycophospholipid-anchored protein, can be fused to the gene for a secreted protein, such as human growth hormone, in order to target and anchor the protein to the apical surface of human intestinal cell lines. This proceedure for converting a secreted protein to its glycolipid-anchored isoform is described in the literature (36, 37, 112). Conversion of secreted polypeptides to isoforms that are anchored by transmembrane spans is also described in the literature. For example, heparin-binding epidermal growth factor-like growth factor (HB-EGF), can be expressed as a longer, membrane-spanning isoform (HB-EGFTMII) comprising an extracellular domain that is structurally identical to the secreted polypeptide (132). Similarly, human lymphocyte differentiation antigen CD8 may be expressed either as a glycoprotein anchored to the membrane by a hydrophobic domain, or as a secreted polypeptide, in which a stretch of 38 amino acids, including the transmembrane span, is deleted (79). Other examples of and techniques for the conversion of secreted polypeptides into isoforms anchored by transmembrane spans are described in the literature (128, 142, 172).

A second genetic manipulation of the imprinted polypeptide is performed when the domain that is to be imprinted lacks an adjacent adhesion domain. This occurs when the polypeptide chosen for imprinting is not polytopic, or when the toxic agents that adhere to domains adjacent to the imprinted domain are unknown or poorly suited for use in the CISTEM. In either situation, the sequence for a functional adhesion domain is fused to the gene for the extracellular domain, creating a gene for a chimeric polypeptide comprising both the functional adhesion domain and the variable domain. For example, the colicin A adhesion domain from *E. coli* OmpF, when fused to separate domains from another outer membrane protein, OmpC, remains functional as a colicin A receptor (69). A group of eukaryotic viral systems, which are described in the literature (44, 101), provide further examples of the insertion of functional adhesion domains. In their application to embodiments of this invention, these systems operate as follows. The antigen-binding site of an antibody (scA) is displayed on the surface of a virus, such as spleen necrosis virus, as described in the literature (44, 101). The antigen that is recognized by the scA is then expressed as an exogenous insert in the extracellular domain that will serve as the adhesion domain of the imprinted polypeptide. (Techniques for inserting exogenous sequences at targetted sites in extracellular domains are described above (V.iv.b), and referenced extensively in the background (III.iv.a).) In this way, a functional adhesion domain for the virus is generated in the desired location, directly adjacent to the imprinted domain.

To briefly summarize the manipulations of the imprinted polypeptide that have been described thus far, a secreted polypeptide may be converted to a membrane-bound isoform, and a functional adhesion domain may be inserted beside the domain that is to be imprinted by this invention. Owing to these manipulations, each of the polypeptide families shown in figure 1 may be imprinted by this invention.

### e) Imprinting Polypeptides in Interaction Analysis or Epitope Mapping

When this invention is used to identify the polypeptides that interact with a given target compound, the CISTEM's imprinted polypeptide may be any outer membrane polypeptide suitable for imprinting. For example, in a CISTEM that identifies polypeptide sequences that interact with the eukaryotic cell-signalling polypeptide Raf (V.iii), the imprinted polypeptide is *E. coli* OmpA. OmpA extracellular loop IV is imprinted to exhibit high affinity for Raf-RBD (V.iii). The sequences selected for their affinity for Raf-RBD are then used to screen sequence libraries and cDNA libraries from mammalian cells with active Ras-Raf pathways.

Similarly, when this invention is applied to epitope mapping, the imprinted polypeptide may be any outer membrane polypeptide suitable for imprinting. For example, the CISTEM that imprints OmpA to bind to Raf-RBD is used to map the surface of interaction between the cell-signalling polypeptides Ras and Raf. Sections of the gene encoding Ras are inserted into the genetic site encoding *E. coli* OmpA loopIV. The CISTEM then selects clones that display sections of Ras that exhibit high affinity for Raf. In this way, the CISTEM identifies the epitopes on Ras that bind to Raf.

### f) Imprinting Polypeptides to Exhibit Functional Activity

Certain embodiments of this invention imprint polypeptides to exhibit functional activity. Among these, the preferred embodiments imprint polypeptides that are naturally well-suited to the performance of the functions desired of them. (The structural and functional categories delineated in the background (III.ii) provide an outline of the proclivities displayed by distinct families of outer membrane polypeptides.) For example, type Ia polypeptides are generally competent for functions involving high-affinity adhesion or specific binding to extracellular targets. They are therefore well-suited to be imprinted for use as highly specific markers or antagonists of small polypeptides, such as small peptide hormones. Type II polypeptides, on the other hand, are often enzymes with extracellular active sites, such as glycosyl transferases. Some type II polypeptides, then, are well suited to be imprinted to exhibit enzymatic activity. When this invention is used to imprint a polypeptide to serve as a functionally active effector, the preferable imprinted polypeptide is a membrane-bound isoform of a secreted protein, such as human growth hormone.

Furthermore, in preferred embodiments of this invention, the imprinted polypeptide is a molecule that naturally exhibits functional or structural homology with molecules that perform functions similar or identical to the one desired of the imprinted polypeptide. For example, structural homologues t*o E. coli* OmpA have been found in *Haemophilus, Shigella, Neisseria*, *Bordetella, and Streptococcus* (The sequences and functions of homologues to *E. coli* OmpA are described in 15, 25-27, 78, 84, 85, 108, 154, 160, and 167). In these virulent bacteria, the OmpA homologues play a role in adhesion to host tissues, and are often recognized by the host's specific immune response. The structural homology between OmpA and these antigenic adhesins makes OmpA well-suited for imprinting for use in applications requiring mimicry of homologous adhesins. Two such applications are competitive inhibition of microbial adhesion, and vaccination, described in greater detail below (VI).

Unlike many other forms of combinatorial chemistry (III.vi), this invention can screen libraries of polypeptides expressed by eukaryotic cells. This greatly expands the range of molecular properties accessible to this invention, for eukaryotic polypeptides are adorned by saccharides, a second a tier of extremely complex, chemically distinctive molecules (III.ii.d). The oligo- and polysaccharides adorning the imprinted domain are not varied in the same systematic manner as the underlying polypeptide sequence, because, as was described in the background (III.ii.d), the saccharide molecule attached to a particular site on a polypeptide is not a simple function of the local amino acid sequence. Instead, the glycosylation at a site is altered in complex and unpredictable ways by changes to the local sequence or to the sequence elsewhere in the polypeptide (III). Therefore, when the glycosylation sites within the imprinted domain are preserved while immediately flanking regions are varied by the method described above (V.iv.c), a semi-random diversity of sugar structures is appended to a systematic diversity of polypeptide sequences, creating a complex glycoprotein or proteoglycan library.

When a CISTEM's target compound naturally interacts with glycosylated polypeptides (i.e., in its natural setting, the target compound binds to glycoproteins or proteoglycans), it is preferred that the imprinted polypeptide be glycosylated. This requires that the CISTEM cell population be eukaryotic. The adhesions that underpin inflammation, blood clotting, sperm-egg interactions, and intercellular recognition are all mediated by glycoproteins or proteoglycans. Therefore, for the identification of molecules for use as antagonists or agonists in these processes, CISTEMs with eukaryotic cell populations and glycosylated imprinted domains are preferred.

The unpredictable effects of peptide sequence variation upon glycosylation throughout the polypeptide merit a cautionary note. Because changes to a peptide sequence can alter glycosylation on entirely different domains of a polypeptide, the CISTEM's toxic (pathogenic) agent must not require a specific glycosylation on its adhesion domain. Otherwise, unexpected changes in the sugars adorning the adhesion domain, renders some cells immune to the pathogenic agent even if they do not bind the target compound at their imprinted domains. As was noted above, the adhesion domain of a pathogenic agent used in a CISTEM must be well-characterized (III.v). In most cases, this characterization reveals whether or not the agent requires glycosylations for adhesion. This may be verified, moreover, by removing glycosylations from the receptor and testing for susceptibility to the agent; methods for removing glycosylations are described in the literature (62, 111).

For use as imprinted polypeptides in embodiments of this invention, polypeptides that exhibit structural integrity are preferred. It is particularly preferred that they not only maintain their properties when liberated from the cell surface, but also exhibit stability in the environmental-perhaps *in vivo*-conditions in which the imprinted molecules are ultimately applied. Among prokaryotic polypeptides, β-barrels are exceptionally stable; they can be denatured only by boiling in strong detergents (141). And in eukaryotic polypeptides, glycosylation often adds structural stability to otherwise malleable protein structures (111). In some cases, the structural integrity of imprinted domains may be heightened by genetic manipulations. Such manipulations may be performed before or after imprinting, and they may be used to increase the stability of structures that will be attached to, or liberated from, the outer membrane.

Genetic manipulations of the imprinted polypeptide may also be used to introduce an element of rational structural design to a CISTEM. Structural analysis of the target compound, its native ligands, or molecules that naturally perform functions similar to that desired of the imprinted polypeptide, may guide the insertion of predetermined motifs into the imprinted polypeptide. A tendency toward the formation of certain structural motifs, such as α-helices, may be incorporated into imprinted domains by inserting variable oligopeptides that are not entirely random, but instead are statistically biased toward sequences that form the desired motifs. Predetermined motifs may also be inserted into the conserved regions of imprinted domains. For example, polyproline or α-helices may be used to extend a variable region away from the cell surface, making it more accessible to a sunken epitope on the target compound. While genetic manipulation informed by structural analysis is by no means necessary to CISTEMs, it may be utilized in combination with CISTEMs to design polypeptides that are well-suited to the performance of desired functions.

### g) Cell Lines and Media

In a preferred embodiment, the CISTEM cell population consists of cells that lack a chromosomal copy of the gene for the imprinted polypeptide, but are capable, upon transfection with an episomal or vector-borne copy of this gene, of translating, exporting, and inserting the imprinted polypeptide in the outer membrane. Thus, for example, in several prefered CISTEMs, which imprint *E. coli* OmpA (III.iv.a) to bind to any one of several targets (V.iii. VI), *E. coli* strain UH203 (74), which is *omp*A⁻, composes the CISTEM cell population. Similarly, in CISTEMs that imprint *E. coli* LamB (III.iv.a) to bind to a target, *E. coli* strain S1755 (28), which lacks the *lam*B gene, composes the cell population. A eukaryotic example is provided by several preferred eukaryotic CISTEMs, which imprint human ICAM-1 to bind to any one of several targets. In these CISTEMs, COS-7 cells are used; this strain lacks ICAM-1, but is easily transfected with an ICAM-1 gene borne on the CDM8 expression vector (the COS-7/CDM8 expression system is described in 148 and 158).

The strains that compose CISTEM cell populations are preferably stop-codon suppressors; that is, they recognize one of the stop-codons, such as UAG, as a translatable codon. The reason for this is that any stop-codons recognized by the strain composing the CISTEM cell population must be excluded from the variable oligonucleotides that are inserted into the vector-borne gene for the imprinted polypeptide. (The synthesis of variable oligonucleotides free of certain stop-codons, and their insertion into the gene for the imprinted polypeptide, are described above (V.iv.c).) Therefore, the synthesis of variable oligonucleotides is simplified when the CISTEM cell population is able to recognize one of the stop-codons. For example, *E. coli* UH203, which is used in several preferred CISTEMs for imprinting OmpA, is *sup*F, i.e., capable of translating the UAG stop-codon. Consequently, variable oligonucleotides inserted into the OmpA vector (pEV218), must be free of the stop codons UAA and UGA, but not of UAG. This is easily achieved by the method of synthesis described above (V.iv.c). Similarly, S1755, the strains used in CISTEMs imprinting LamB, is *sup*E44, i.e., capable of translating UAG.

The medium in which the CISTEM cell population is propagated depends simply on the strain composing the population. For example, *E. coli* UH203 is grown in Luria broth or agar. When a strain contains a plasmid with an antibiotic resistance gene, the appropriate antibiotics are added to the medium or agar. Standard media for all commonly used strains of prokaryotic and eukaryotic cells are described in the literature (10). The conditions for transformation of CISTEM cells with the vector bearing the imprinted polypeptide also depend upon the cell type, and are described in more detail above (V.iv.a), as well as in the literature (109, 146). Induction of expression of the imprinted polypeptide requires incubation of the cell population with the compound that activates the imprinted polypeptide's inducible promoter (V.iv.a). As is described above, however, certain inducible promoters, such as the lac promoter/operator elements, are "leaky", allowing for the appropriate level of expression of the imprinted polypeptide without any induction.

When the CISTEM cell population is incubated with a suspension or solution of the target compound, the medium generally contains only the minimal requisites for cellular sustenance in addition to the target compound. The presence of abundant macromolecules other than the target compound can result in selection for clones whose imprinted domain bind not to the target, but rather to one of the constituents of the medium. Thus, for example, in preferred CISTEMs imprinting OmpA, *E. coli* UH203 are incubated in the presence of any one of several target compounds in ATCC Medium 52 (10). However, in some CISTEMs, Luria broth was used. In several preferred CISTEMs for imprinting ICAM-1, COS cells are incubated with target compound in TC Minimal Medium Eagle or Minimal TC Medium 99 (10). Minimal media for other commonly used prokaryotic and eukaryotic cell lines are described in the literature (10). The concentration of target compound and duration of incubation are important to CISTEM function, and are discussed in detail in a later section (V.v).

When the invention is used for the identification of functionally active polypeptides, such as enzymes or inhibitors of a target compound's biological activity, incubation with the target compound occurs under chemical and thermal conditions that are as similar as possible to those under which the imprinted polypeptide will ultimately be applied. For example, one preferred eukaryotic CISTEM imprints ICAM-1 to serve as an antagonist to HIVgp120's interaction with the receptor CD-4 (114). In this CISTEM, the cell population is incubated with the target compound, soluble HIVgp120, described above (V.iii), in temperature and ion concentrations approximating human extracellular fluid (table 2). In the application of the invention to interaction analysis or epitope mapping, the incubation occurs under chemical and thermal conditions that are as similar as possible to those in which the studied interaction naturally takes place.

### v. THE TOXIC AGENT AND CISTEM PARAMETERS

Many outer membrane polypeptides are ports of adhesion for multiple agents that kill, or retard the multiplication of, the cells they enter (III.v). Each of these agents recognizes a distinctive adhesion domain on the polypeptide receptor (figures 2 and 3). The adhesion domains of a wide range of toxic agents are thoroughly characterized in the literature (table 1, reviewed in 164). Using these characterizations, a toxic agent whose adhesion domain resides beside the imprinted domain (as shown in figure 5) is identified. In the event that the polypeptide that is to be imprinted is bitopic, or when a suitable toxic agent with an adhesion domain adjacent to the imprinted domain cannot be found, a functional adhesion domain is appended or inserted by genetic manipulations of the imprinted polypeptide; these manipulations are described in the previous section (V.iv) and in the background (III.iv).

Once a toxic agent with an adhesion domain beside the imprinted domain has been identified, the following procedures are performed to establish and optimize the agent's selection for cells in which the imprinted domain binds the target compound. This procedure serves two functions. First, it establishes that the toxic agent significantly impairs cells in which the imprinted domain fails to bind the target compound, while leaving unaffected those cells in which the imprinted domain effectively binds the target. In other words, the procedure demonstrates that the agent is competent to perform CISTEM selection.

Second, this procedure is used to determine the experimental conditions that will allow reliable CISTEM selection. To adjust CISTEM selection, multiple parameters may be varied, including the following: concentration of the target compound ([T]) with which the CISTEM cell population is incubated; duration of incubation with the target compound; initial multiplicity of infection (MoI) of the pathogenic (toxic) agent; duration of incubation with the pathogenic agent; temperature; level of expression of the imprinted polypeptide. Each of these parameters may be optimized by varying the parameter value over a number of CISTEM trials, as will be described here. However, every parameter need not be optimized for every embodiment of this invention. In preferred embodiments of the invention, at least two parameters are optimized by multiple trials, as described here, while other parameters are set by common laboratory procedure or precedents described here. In the optimization described here, concentration of target compound, [T], and initial multiplicity of infection, MoI, are the two parameters that are varied over a number of trials. In the examples below (VI), an optimization is described in which two different parameters-namely, level of expression of the imprinted polypeptide and duration of incubation with the toxic agent are varied.

The procedures for demonstrating that the toxic agent is competent to perform CISTEM selection, and for optimizing CISTEM parameters, make use of the cell population whose construction is described above (V.iv.b). In this cell population, an oligopeptide tag is exhibited in the imprinted domain. This tag binds with high affinity and specificity to a readily available ligand. (The T7 tag/T7 antibody is a preferred example of a tag/ligand pair; this pair and others are described above and in the literature (see V.iv.b).) In the optimization described here, expression of the imprinted polypeptide bearing the oligopeptide tag is regulated to the relatively low level estimated to be roughly 1000 copies per cell. As was noted above, however, in the optimization of some embodiments of this invention, such as that described in the examples (VI), the level of expression of the imprinted polypeptide is one of the varied parameters. The method by which regulation of the imprinted polypeptide is achieved depends upon which heterologous promoter controls the gene for the imprinted polypeptide. For example, on pEV218T7 (V.iv.a), the *omp*A plasmid used in a preferred embodiment of this invention, *omp*A is under the control of the *lac* regulatory elements, which are induced by isopropyl-β-D-thiogalactopyranoside (IPTG). In addition, the *omp*A gene on pEV218T7 comprises a TAG stop codon corresponding to amino acid 7 (76) of the polypeptide; therefore, when expressed in the amber-supressing (*sup*F) host *E. coli* UH203, *omp*A expression is regulated not only by the level of induction with IPTG, but also by the concentration of the suppresor tRNA, which is expressed from a plasmid. Low level expression of OmpA is achieved with 1.0 mM IPTG induction. On pSB1649, *lam*B is under the control of the *lac* regulatory elements, and expression is regulated as described in the literature (28). Regulation of the expression of ICAM-1 from pCDl.8, the CDM8 vector construct used in a preferred eukaryotic embodiment of this invention, is also described in the literature (148, 158, 159).

Two populations of cells (populations A and B) exhibiting the oligopeptide tag in the imprinted domain are grown in a medium appropriate to the cell type and the expression vector. (For instance, in a particularly preferred embodiment of the invention, *E. coli* UH203 transformed by pEV218T7 are grown at 37° C in Luria broth supplemented with 100µg/ml ampicillin, and 1.0 mM IPTG for induction of pEV218I expression; conditions for growth of other cell types are described above (V.iv.g) and in the literature (10).) A third population of cells (C) of the same strain as populations A and B, transformed by the same vector as populations A and B, but without the oligopeptide tag, is grown in the same medium. (Thus, for example, in the particularly preferred embodiment mentioned above, *E. coli* UH203 transformed by pEV218, rather than pEV218T7, are grown in the conditions described above.) Populations B and C are each grown to generate three clonal subpopulations of 10⁶ cells, B1, B2, B3, and C1, C2, C3. Population A is grown to generate nine clonal subpopulations, A1, A2...A9, each comprising 10⁶ cells.

Populations A1...9 and C1...3 are incubated with the target compound, while populations B1...3 are incubated under identical conditions, but without the target. Optimal conditions and time required for binding between the oligopeptide tag and the target are prescribed by the supplier of the tag/target system or in the literature (43,18,147,58). In the particularly preferred embodiment, in which the T7 tag is displayed at loop IV of *E. coli* OmpA, and the T7 antibody (Novagen) serves as the target compound, the cell population expressing pEV218T7 is incubated with mAbT7 for at least 30 minutes. The concentration of target compound with which cells are incubated is systematically varied across populations A1...9 and C1...3 in order to identify the concentration that is optimal for CISTEM selection (table 3). Populations A1...3 are each incubated with the lowest concentration of target compound. This minimum concentration varies, depending upon the properties of the agent and the availability of the target, but is generally approximately 10³ x concentration of cells x number of imprinted polypeptides per cell. Thus, for example, for a population of 10⁶ E. coli grown under the conditions described above to express roughly 1000 copies of OmpA per cell, 0.001 mg mAbT7 is used in 1 ml of medium. This is approximately 4 x 10³ target molecules (mAbs) per imprinted polypeptide. Populations A4...6 are incubated with a higher concentration of target compound, such as 10x the concentration used with populations A1....3. Populations A7...9 and C1...3 are incubated with the highest concentration of target, such as 100x the concentration used with populations A1...3.

Following incubation, populations A1...9, B1...3, and C1...3 are exposed to the toxic agent. The technique of exposure depends upon the nature of the agent, and, for all well-known agents, is described in the literature. Methods of exposing prokaryotic cell populations to bacteriophages are described by Callendar (34); methods of exposing prokaryotic cell populations to colicins are described by Pattus et al. (135); and methods of exposing eukaryotic cell populations to viruses are described by Fields et al. (68). Like the concentration of target compound, in the optimization described here, the concentration of agent is varied across the cell populations in order to identify optimal conditions for the CISTEM's selection (table 3). (As was noted above, however, not every parameter is varied in optimizing each embodiment of this invention. In the optimization described below, for example (VI), the concentration of target is not one of the parameters that is varied. Instead, this concentration is fixed, while two other parameters are varied.) Populations A1,4,7,B1, B1, and C1 are exposed to the lowest concentration of the toxic agent. This concentration will vary from one CISTEM to another, depending upon the properties of the agent, the method and duration of exposure, and the availability (and thus the concentration) of the target compound, but it will generally be roughly the minimum concentration required to affect every cell in a fully susceptible population during a CISTEM run. Thus, for many lytic bacteriophages, such as E. coli K3 or 1, when the method of exposure is classical phage infection (34), and the duration of incubation with the bacteriophage is 60 minutes at 37 °C , the concentration will be 2 - 5 x the cell concentration, or MoI∼ 2 - 5. Cell populations A2,5,8, B2, and C2 are exposed to a doubled concentration of the agent, and populations A3,6,9, B3, and C3 are exposed to yet another doubling of agent concentration. Table 3 summarizes the variation of target and agent concentrations over the cell populations in this procedure for optimizing CISTEM selection.

The concentrations in this procedure may vary depending on the nature of the toxic agent and the method of exposure, but several fundamental aspects of the proceedure do not change from one CISTEM to another. First, in the control populations, B and C, the toxic agent has unimpeded access to its receptor, and therefore affects every cell. Population B is susceptible to the agent because, although cells bear the tag at the imprinted domain, the population is not exposed to the tag's ligand. Therefore, the toxic agent's adhesion domain remains exposed and functional. Population C is susceptible because, although the cell population is exposed to the ligand, the ligand is not bound by the imprinted polypeptide, which lacks the ligand's tag. Because the toxic agent's adhesion domain remains exposed and functional in populations B and C alike, the results of exposing these populations to the agent are the same. Thus, in these control trials, exposure to the agent yields the same results for populations B1 and Cl; likewise for populations B2 and C2; and for populations B3 and C3. These controls confirm two conclusions critical to the CISTEM: 1. the presence of the target ligand does not affect the toxic agent's activity, provided the ligand is not bound to the imprinted polypeptide; 2. the presence of the tag in the imprinted domain does not affect the toxic agent's activity, provided the tag is not bound to a target ligand.

Whereas control populations B and C are entirely susceptible to the toxic agent, populations A1...9 exhibit a range of resistance, extending even to immunity. In the cells of population A, the imprinted domain harbors the oligopeptide tag, which binds the target ligand during incubation. As a result, the adjacent adhesion domain is occluded from the toxic agent, and the cells are protected. Populations A1...9 exhibit a range of resistance because they are exposed to a range of concentrations of target ligand. When the range of concentrations is broad enough, the range of resistance extends from partial susceptibility to complete immunity.

Based on these preliminary trials, the concentration of toxic agent that allows for functional CISTEM selection is found as follows. Ubiquitous pathogenic effect--*i.e.* every cell in a population is affected by the agent--is easily observed when cell populations are exposed to agents according to protocols described in the literature (34, 68, 135). For example, in exposing prokaryotic populations to virulent lytic phage, ubiquitous pathogenic effect results in confluent lysis of the population, with zero surviving colonies (34). The lowest concentration of toxic agent at which every cell in the control populations (B and C) is affected by the agent is an appropriate level for CISTEM selection. For example, if some cells from B1 and C1 (table 3) escape the toxic agent, but the cells of B2 and C2 are all affected by the agent, then the appropriate concentration of toxic agent for the CISTEM is MoI = 10. In this example, the minimum concentration required for ubiquitous effect could be more exactly determined by further trials with MoI's ranging from 5 to 10.

After the CISTEM's concentration of toxic agent is found, the optimal concentration of target compound is determined as follows. Each CISTEM has an approximate threshold affinity, Kₐ = t , such that clones whose imprinted domains bind the target with an affinity below this threshold (Kₐ < t) are eliminated from the population by the toxic agent, whereas clones whose imprinted domains exhibit an affinity above the threshold (Kₐ >t) are protected from the agent, and thus selected. The threshold for a CISTEM can be set by adjusting the concentration of the target compound. To set the threshold to approximately the affinity that the oligopeptide tag exhibited by populations A and B shows for its ligand, the target concentration used in the CISTEM is the lowest level that provides complete protection to population Ai in the presence of toxic agent at the CISTEM's MoI. (Complete immunity to the agent is easily observed when cell populations are exposed to agents according to common protocols described in the literature (34,68,135).) Thus, for example, if the CISTEM's concentration of toxic agent, determined as described above, is MoI = 10, and population A5 exhibits complete immunity to the toxic agent while population A2 is slightly reduced by the agent (table 3), then 10⁴ molecules of target compound per imprinted polypeptide is used in a CISTEM to select for cells whose imprinted polypeptides bind the target with an affinity equal to or greater than the affinity exhibited by the oligopeptide tag for its ligand. A slightly lower concentration of target compound, such as 10³ molecules per imprinted polypeptide, is used to select for cells with imprinted polypeptides that exhibit an affinity for the target that is significantly higher than the affinity exhibited by the oligopeptide tag for its ligand. A higher concentration of target compound, such as 10⁵ molecules per imprinted polypeptide is used to set the CISTEM's threshold affinity well below the affinity exhibited by the tag for its ligand.

### vi. Identification of the (poly)peptide of interest

The application of CISTEM to cell populations yields cell clones that survive the selection procedure by the virtue of interaction with the target(s). Thus, the hallmark of their survival is the (poly)peptide of interest that they display at the surface. The sequence of the polypeptide of interest is encoded by the DNA sequence which has been inserted into the display domain. The surviving cell population can be monoclonal or polyclonal. In the latter instance, surviving cells need to be plated out on agar plates for recovering colonies derived from single cells (state of the art bacterial genetics). Amplification of monoclonal cell populations and the subsequent preparation of their plasmid DNA is following standard protocols of recombinant DNA methodology. Since the plasmid codes for the display protein and its inserted polypeptide of interest, DNA sequence analysis of the inserted DNA tag (state of the art recombinant DNA technology) provides the DNA sequence of the insert and thereby allows to predict the amino acid sequence of the polypeptide of interest which has been expressed and displayed at the surface as integral part of the membrane protein. The inserted DNA has been either originated from a pool of randomly synthesized oligonucleotides or from DNA fragments derived from natural occuring mRNA (cDNA) or genomic DNA. The obtained DNA and cognate polypeptide sequences can be scrutinized to match sequences of known proteins and their cognate genes. Thus, sequence analysis can be employed to identify the polypeptide of interest and to attribute and/or relate its composition to known genes or DNA sequences and their predicted proteins.

### vii. MODIFICATIONS AND ELABORATIONS OF THE BASIC CISTEM

Modifications and elaborations of the basic CISTEM achieve several extensions of its capabilities, including the following: i. augmentation of the maximum affinity of selected polypeptides for the target compound; ii. specification of the number of binding sites joining a multivalent imprinted polypeptide to the target compound; iii. selection for imprinted polypeptides that exhibit both high affinity for the target compound and low affinity for a second compound; iv. selection not only for affinity, but also for functional activity; v. screening the polypeptides expressed from a cDNA library for binding to a target compound; vi. selection for imprinted polypeptides that exhibit affinity for one or more of a set of multiple, distinct target compounds.

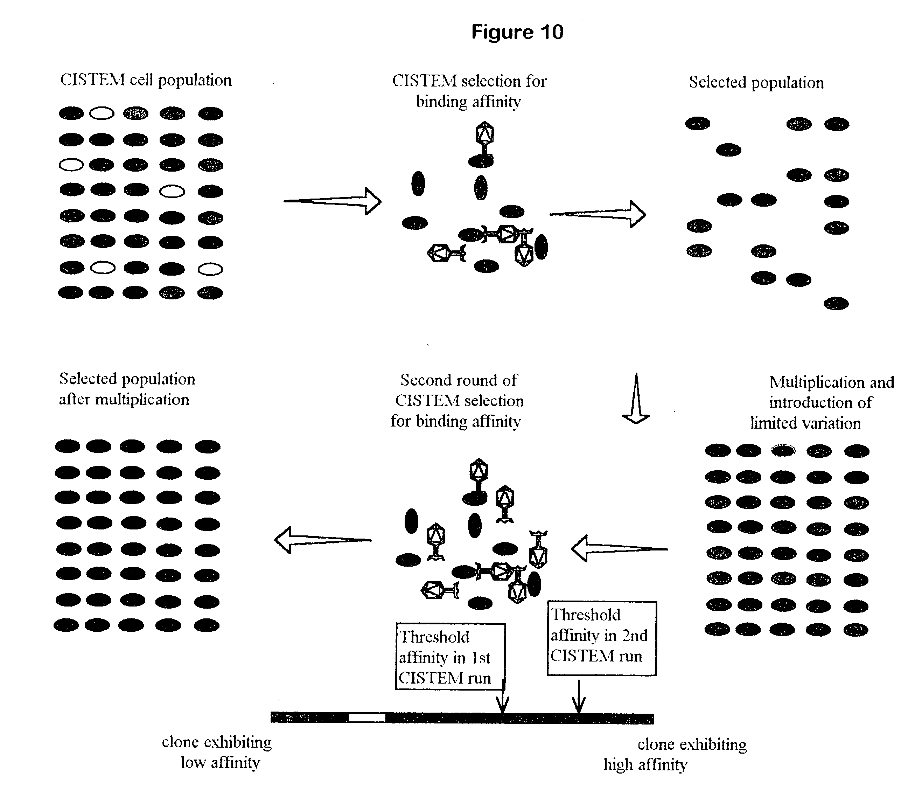

To increase the maximum affinity attainable in a CISTEM, iterative CISTEM runs are performed (figure 10). Once a population of clones exhibiting affinity for the target compound has been selected by a CISTEM, the vectors bearing the gene for the imprinted polypeptide are isolated from this population by common techniques appropriate to the vector, and described in the literature (146). New variable oligonucleotides, such as those described above (V.iv.c), are then inserted into the vectors alongside the original variable insertions. This is performed by the same procedure that was used to achieve the original insertion of variable oligonucleotides at a genetic site corresponding to the imprinted domain. (One such procedure is described above (V.iv.c).) Alternatively, variation is introduced not as an extension of the original oligonucleotide inserts, but rather as an oligomeric rearrangement of the original inserts (the procedure of oligomeric rearrangement of oligonucleotide inserts is described in 28) or a limited mutagenesis of the original inserts (techniques for the limited mutation of oligonucleotide inserts are described in 11 and 133; insertion of mutated oligonucleotdies is performed according to the procedure described above, V.iv.c, for insertion of variable oligonucleotides into a targeted site in a vector). In other words, oligopeptide "sequence space" (the sequence space of combinatorial libraries is defined in 63) may be searched by a number of strategies, some of which have been applied to screening other combinatorial libraries (11, 28, 29, 82,1 33, III.vi). The vectors bearing fresh variation at a site corresponding to the imprinted domain are then used to transform a population of cells, according to common protocols cited above (V.iv.a), creating a new CISTEM cell population. This population is then subjected to yet another round of selection by the CISTEM (figure 10). This cycle of introducing variation, selecting for affinity for the target compound, and allowing selected clones to multiply, is iterated until binding between the imprinted domain and the target compound attains the desired degree of affinity.

The number of oligopeptide sequences screened by a CISTEM for affinity for the target compound is increased, i.e., sequence space is increased, by performing CISTEMs in parallel, by combining CISTEMs with high-throughput robotics strategies, by performing CISTEMs on a large-scale, or by performing CISTEMs in a continuous-throughput chemostat.

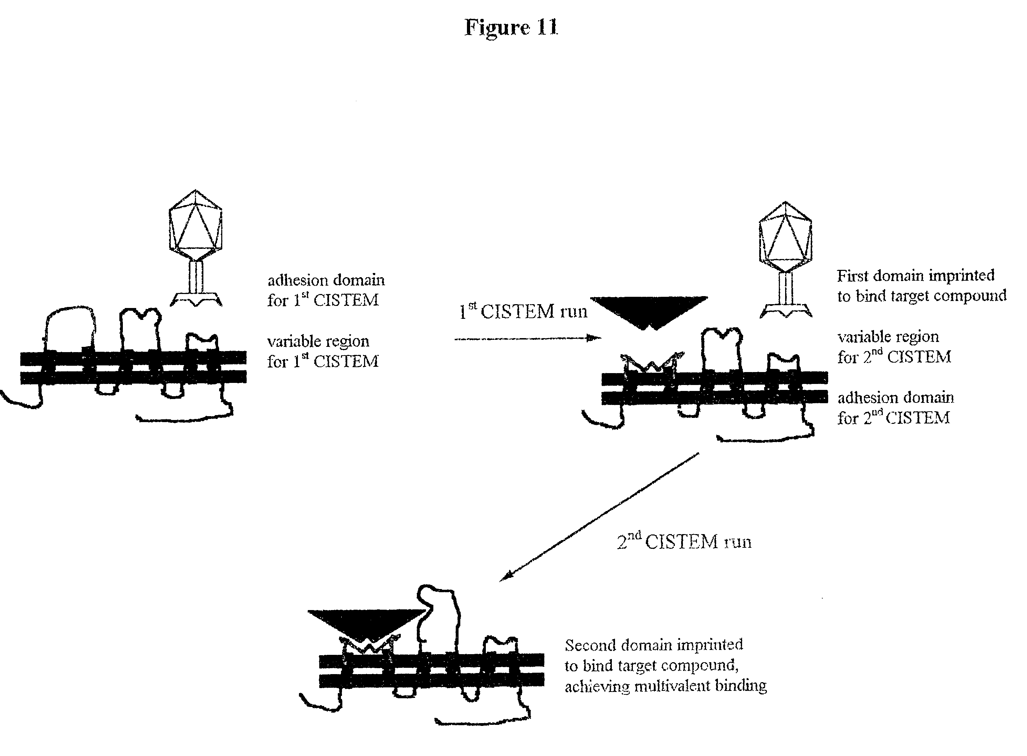

To create imprinted polypeptides that exhibit multivalent binding of the target compound, multiple variable regions may be imprinted by sequential CISTEM runs (figure 11). First, a single region or domain of the imprinted polypeptide is imprinted to bind the target compound. Next, the selected population of cells, which exhibit the previously-imprinted domain, is engineered to exhibit a variable region elsewhere in the imprinted polypeptide-either in an unimprinted region of the previously-imprinted domain, or in an altogether separate domain. This is achieved as follows. First, the vectors bearing the gene for the imprinted polypeptide are isolated from the selected population of cells by common techniques appropriate to the vector and described in the literature (146). Next, using one of the procedures described above (V.iv.c) for the insertion of variable oligonucleotides at a specific site in a gene, variable sequences are inserted at a genetic site correponding to an extracellular domain of the imprinted polypeptide. This site is distinct, however, from the site at which variable oligonucleotides were inserted for the first round of CISTEM selection. The vectors, which now bear selected sequences at one site and variable oligonucleotides at another site, are used to transform a population of cells (V.iv.a), creating a new CISTEM cell population. This population is subjected to another round of CISTEM selection. In this round, the concentration of target compound is lower than it was in the previous round, making the threshold affinity for selection, t (V.v), higher. The reason for this is that, after the first round of selection, every cell in the CISTEM population binds the target compound, and the CISTEM must now select only cells expressing imprinted polypeptides that bind the target at multiple distinct epitopes. With each new region of the imprinted polypeptide that is imprinted, a new epitope on the target compound is bound.

Certain embodiments of this invention select for imprinted polypeptides that exhibit high on-rate in their binding with the target compound. In these CISTEMs, the CISTEM cell population is not pre-incubated with the target compound prior to introduction of the toxic agent. Rather, the toxic agent is introduced simultaneously with the target compound. The concentrations of agent and target compound are varied over multiple trials, as is described in the optimization procedures detailed elsewhere in this document (V.v and VI), Concentrations are adjusted such that, upon simultaneous introduction of agent and target to the CISTEM cell population, clones whose imprinted polypeptides bind to the target relatively rapidly are protected, whereas clones whose imprinted polypeptide binds to the target more slowly are eliminated by the toxic agent.

A CISTEM that imprints a polypeptide to show high affinity for a target compound but extremely low affinity for a second compound, which is here called the "repelled compound", is constructed as follows. The size of the target compound, the size of the repelled compound, and the distance between the imprinted domain and the adhesion domain are altered, if necessary, to meet the following condition: when the target compound is bound to the imprinted domain, it occludes the adhesion domain, whereas when the repelled compound is bound to the imprinted domain, it is too small to occlude the adhesion domain. If this criterion is not met fortuitously (i.e. if the repelled compound does not happen to be extremely small), then the diameter of the target compound and the distance between the domains may be altered, as follows. The target compound may be enlarged by creating a multimer. (The procedure for creating a multimer depends upon the nature of the target compound, but many techniques for joining multiple copies of a compound are commonly practiced and described in the literature.) The distance between the imprinted domain and the adhesion domain may be increased by moving either domain to a site on the imprinted polypeptide that is topologically farther from the other domain. Transposing the imprinted domain simply involves inserting variable oligonucleotides, according to the procedure described above (V.iv.c), at a genetic site correponding to a different extracellular domain of the imprinted polypeptide. In many prokaryotic and eukaryotic embodiments of this invention, the adhesion domain may be transposed to a different extracellular domain of the imprinted polypeptide without affecting its function as a receptor for the toxic agent. The procedure for transposing an adhesion domain is described above (V.iv.d) as well as in the literature (44, 161). If, as a result of moving the adhesion domain and the imprinted domain farther away from each other, one or more extracellular domains that are not involved in CISTEM function occur between the two, topologically separated domains that are critical to CISTEM function, the superfluous domains may be replaced by polyproline linkers in order to ensure that extracellular structures do not impede proper occlusion of the adhesion domain by a target compound bound to the imprinted domain.

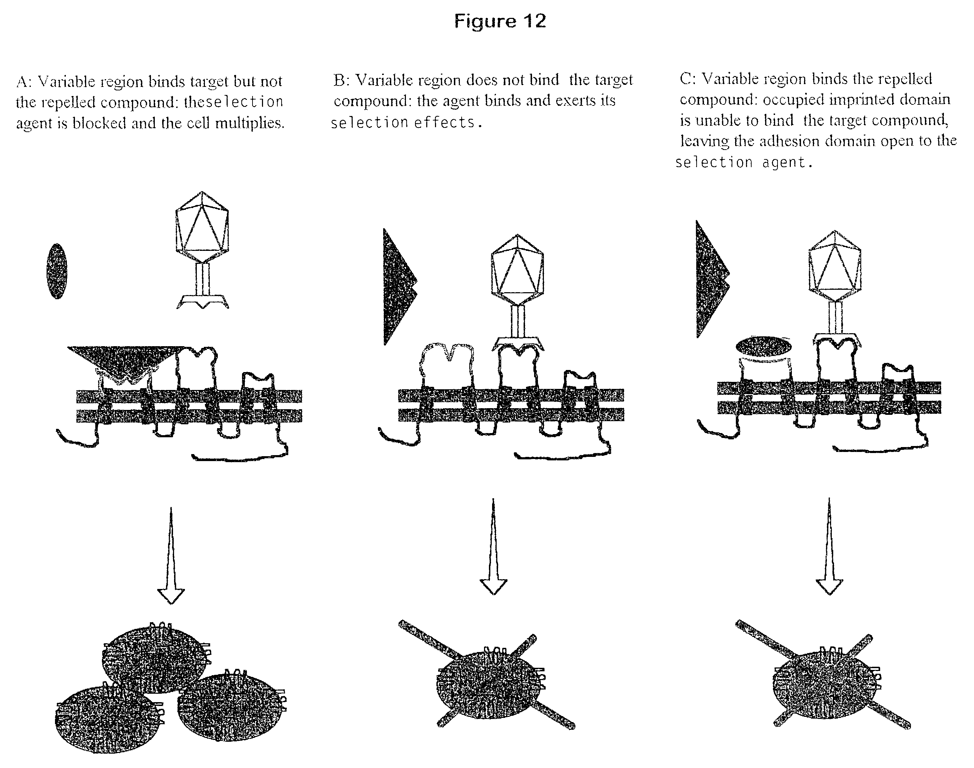

Once the three critical distances-the distance between the imprinted domain and the adhesion domain, the diameter of the target compound, and the diameter of the repelled compound-are in accordance with the criterion stated above, the CISTEM cell population is incubated with both the target compound and the repelled compound. Then the CISTEM's toxic agent is introduced, and this creates two selective pressures. One pressure favors cells expressing imprinted polypeptides with higher affinity for the target compound (figure 12): a target compound bound to an imprinted domain occludes the adjacent adhesion domain, and thus serves to protect the underlying cell from the toxic agent. Among cells that exhibit some affinity for the target compound, a second selective pressure favors cells with lower affinity for the repelled compound; a repelled compound bound to an imprinted domain not only fails to occlude the adjacent adhesion domain, but also prevents binding by a target compound (figure 12). In short, an imprinted polypeptide's affinity for the repelled compound effectively reduces its affinity for the target compound. Thus, selection favors cells that exhibit imprinted domains with high affinity for the target compound and low affinity for the repelled compound (figure 12).

To select not only for affinity but also for functional activity of imprinted polypeptides, a basic CISTEM may be coupled to a secondary screen for activity. In the first step of coupled selection, a CISTEM selects clones whose imprinted polypeptides bind the target compound in the prescribed fashion. From this population of clones, or from the imprinted domains extracted from such a population, a secondary screen selects the structures whose binding to the target compound exhibits the desired functional activity. Imprinted domains liberated from the cell surface by detergent or by expression as a secreted isomorph (isomorphic forms and their interconversion are described above, in III.iv.b) may be subjected to any commonly practiced screens or assays for activity or modification of the target compound's activity. For example, one CISTEM imprints *E. coli* OmpA to bind with high affinity to soluble IL-6-Receptor. (This target compound is described in more detail above (V.iii).) The imprinted polypeptides, freed from the cell membrane by a conventional gentle detergent, which is known not to denature OmpA, are then subjected to a second screen for their capacity to agonize the cell-bound, functional IL-6-Receptor. This screen for agonistic activity is described in the literature (95). Alternatively, extremely strong selection for functional activity may be exerted upon the CISTEM's selected set of cellular clones, and does not require liberation of the imprinted domains from the cellular membranes. In fact, precisely because they yield replicating cell populations, CISTEMs can be coupled to selection regimes very similar to genetic complementation. In these selection regimes, each cell's supply of a vital compound, such as a nutrient, is dependent upon the imprinted domain's performance of the function desired of it, such as enzymatic activity. In other words, the imprinted domain's functional activity either converts the target compound to a compound vital to the underlying cell, or secures the vital compound for the cell.

In certain embodiments of this invention, the target compound consists of a library of separate and distinct molecules, which is here called "the target library". A CISTEM in which a target library (rather than a single target compound) is used serves to select interacting pairs in which one member of each selected pair is the oligopeptide displayed at the imprinted domain of a protected clone, and the other member is a target library molecule that binds to this oligopeptide and thus serves to protect the clone. Provided each molecule in the target library is large enough to meet the size requirement for CISTEM function (V.iii), and is present in the medium at a concentration adequate to confer protection upon cells whose imprinted polypeptides bind to one or more molecules in the target library, the target library may comprise as many distinct molecules as is desired, and may be constructed according to any known procedure for making or isolating libraries of molecules. These procedures are partially reviewed in the background (III.vi), and include the following: phage-display technology (8, 12, 33, 58, 118, 134, 147); selex technology (82); and other technologies for library construction that are reviewed in the literature (99).

In CISTEMs involving target libraries, two strategies are used, if necessary, to increase the concentration of each molecule in the target library to the minimum level required for effective CISTEM selection, *i.e.*, to achieve the concentration required to confer protection from the toxic agent upon those cells whose imprinted polypeptides bind to one or more of the molecules in the target library. In one strategy, the CISTEM cell population and the target library are each amplified, divided into fractions, and exposed to one another in microtiter wells. This allows the concentration of target molecules to be sufficiently high to confer protection upon cells whose imprinted polypeptides bind to one or more molecules in the target library.

In the second strategy for increasing the concentration of each molecule in the target library, the target library is secreted by the CISTEM cell population itself. As was described in the background (III.vi), libraries of variable oligopeptides can be displayed as fusions to gene protein 3 of *E. coli* filamentous phage (M13, fl, or fd) (filamentous phage-display technology is described and demonstrated in 8, 12, 33, 58, 118, 134, and 147). Filamentous phage create relatively avirulent infections in which phage particles are liberated from the host cell by extrusion, leaving the cell in tact (122). Therefore, a CISTEM cell population that secretes its own target library can be engineered as follows.

As in the basic CISTEM described above, a cell population that lacks a gene for the imprinted polypeptide is transformed with a library of exogenous copies of this gene, in which variable oligonucleotides have been inserted at the genetic site corresponding to the imprinted domain. For example, in the construction of an *E. coli* CISTEM cell population described above (V.iv.c), *E. coli* UH203 is transformed with pEV218I, a library of ompA plasmids with variable oligonucleotides at the genetic site corresponding to extracellular loop IV of the membrane protein. The resultant cell population displays a library of exogenous oligopeptides at the imprinted domain. In an extension of the basic CISTEM, this cell population is then transformed with a second extra-chromosomal genetic element that encodes a secreted library of oligopeptides. The OmpA-imprinting cell population, for example, is transfected with a library of filamentous bacteriophage engineered to display exogenous oligopeptides at gene protein 3. (Many such libraries are described in the literature, specifically in 8, 12, 33, 58, 118, 134, and 147.) Each cell in the population now has two extra-chromosomal genetic elements: one encodes OmpA, in which an extracellular loop displays an exogenous oligopeptide; the other encodes a stably extruded M13 phage, in which gp3 displays a second exogenous oligopeptide, distinct from the one displayed on the cell surface (figure 14).

The CISTEM cell population expressing two extra-chromosomal genetic elements is then exposed to the CISTEM's toxic agent, following the same protocols for exposure as were described for the basic CISTEM (V.v and VI). Upon exposure to the toxic agent, a clone in the CISTEM's population is protected only if the target library molecule that is secreted by the clone binds specifically to the clone's imprinted domain. That is, a clone is protected by an autocrine loop. In the specific CISTEM in which *E. coli* displays an OmpA library and secretes an M13 library, K3 serves as the pathogenic (toxic) agent (figure 13). An *E. coli* clone is protected from K3 only if the exogenous polypeptide displayed at loop IV of OmpA binds specifically to the M13 that the clone itself secretes. The genes for both members of this interacting pair of oligopeptides are easily recovered from the protected cell. In sum, through autocrine protection of clones, a CISTEM with a target library selects for specifically interacting pairs of oligopeptides, as well as for the genes that encode them.

Certain embodiments of this invention are used to determine whether certain molecules interact specifically with an imprinted polypeptide that has not been altered by the insertion of variable oligopeptides. Any outer membrane polypeptide that is used as a receptor by a pathogenic agent, including polypeptides engineered by the manipulations described above (V.iv.d), may be used in these embodiments of the invention. Because the imprinted polypeptide is not engineered to display highly variable oligopeptides, there is no "imprinted domain", and the toxic agent's adhesion domain may be located anywhere on the imprinted polypeptide. As in the basic CISTEM cell populations described above, the imprinted polypeptide is expressed from an inducible promoter (V.iv.a). The cell population, induced to express the imprinted polypeptide, is exposed to a target library, according to one of the methods described above for constructing target libraries and introducing them to CISTEMs. Either microtiter wells or the autocrine loop methods may be used to increase the concentration of target library molecules. The CISTEM's toxic agent is then introduced according to procedures described for the basic CISTEM (V.v). Cells are protected only if molecules in the target library bind to the imprinted polypeptide, and thus prevent adhesion by the toxic agent. Thus, these embodiments of this invention provide an easy and straightforward method for assaying a set of molecules' affinity for the imprinted polypeptide.

### EXAMPLES

### i. EXAMPLE 1: AN E. COLI OMPA/COLIPHAGE K3/mAbT7 CISTEM

### a) E. coli Outer membrane protein A

*E. coli* Outer membrane protein A (nucleotide sequence described in 14, primary structure described in 42) is a 325-residue polypeptide comprising an amino-terminal, eight-stranded β-barrel (membrane insertion and structure described in 126 and 127) with four extra-cellular domains (surface-exposed regions identified in 48,126, and 127), three periplasmic loops (periplasmic exposure identified in 681, and a roughly 150-residue periplasmic carboxyl-terminal moiety (figure 3). The β-barrel conformation is extremely stable, and can be denatured only by boiling in strong detergents (III.ii.b). Naturally present in approximately 2-3 x 10⁵ copies per cell (92), OmpA is among the most abundant proteins in the *E. coli* outer membrane. It is also widely conserved in bacterial evolution (108).

As was described in the background (see III.iv.a), exogenous oligonucleotides, inserted into the ompA gene at sites corresponding to extracellular domains of the polypeptide, are normally translated as part of the polypeptide, and do not disturb the polypeptide's insertion in the outer membrane. Furthermore, the oligopeptides encoded by these exogenous genetic insertions are displayed on the surface of the cell, adopt stable folded structures, and can exhibit enzymatic, agonistic, or antagonistic activities.

### b) Construction of a cell population for testing and optimizing CISTEM selection

To construct a cell population for use in testing and optimizing CISTEM selection, an *E. coli* population lacking wild-type OmpA was engineered to express a plasmid-encoded copy of OmpA, in which extracellular loop IV of the polypeptide displayed the T7 tag oligopeptide (Met-Ala-Ser-Met-Thr-Gly-Gly-Gln-Gln-Met-Gly), which binds to the commercially available T7 antibody (Novagene). In pEV218 (76), the ompA gene, which is under the IPTG-inducible control of the lac promoter, has been engineered to bear a polylinker rich in restriction endonuclease sites at a genetic region corresponding to extracellular loopIV of the polypeptide (figure 9). This plasmid was digested with SacI (enzyme and reaction buffer from New England Biolabs) and XbaI (enzyme and reaction buffer from MBI Fermentas), yielding a fragment of the restriction site polylinker and the linearized plasmid with sticky ends left by the restriction enzymes. The linearized plasmid with sticky ends was isolated by standard gel electrophoresis (146). Two single stranded oligonucleotides were synthesized by conventional technology (custom oligonucleotides are available from commercial laboratories, and methods for synthesizing single-stranded oligonucleotides are described and referenced in 29 and 82) to read as follows:

Oligonucleotide 1:
5' - CG[ATGGCTAGCATGACTGGTGGACAGCAAATGGGT] - 3'

Oligonucleotide 2:
5' - CTAG[ACCCATTTGCTGTCCACCAGTCATGCTAGCCAT]CGAGCT - 3'

The sequence in brackets composes the T7 tag gene sequence. These two single-stranded oligonucleotides were annealed in Klenow reaction buffer (MBI catalogue), forming the double stranded T7-tag gene with sticky ends complementary to the sticky ends on pEV218, linearized by SacI and XbaI as described in the previous paragraph. When the double-stranded oligonucleotide was ligated (MBI Fermentas ligation buffer) into the linearized plasmid, the T7 tag gene was inserted, in frame, into the ompA gene on pEV218. The new construct was designated pEV218T7.

The plasmid pEV218T7 was used to transform a population of the strain UH203 (lac, supF, ompA, recA, proAB, rpsL) (74), according to common methods of transformation described in detail above (V. iv. b.).

### c) Preliminary optimization of CISTEM selection by coliphage K3

Coliphage K3 invades *E. coli* by binding to loops I, II, and III of OmpA. Loop IV is not used by the phage, and, indeed, exogenous oligopeptides at loopIV do not affect K3 susceptibility of the cell (the K3 adhesion domains are shown in figure 3; bacteriophages are reviewed briefly in the background, III.v.b, and bacteriophage K3, as well as its receptor requirements, are described in 76, 126, and 127). The following procedure was performed to fulfill two objectives: a) to conclusively demonstrate that cells whose imprinted polypeptides bind the target compound are protected from K3 attack, whereas cells whose imprinted polypeptides fail to bind the target are eliminated by the phage, and b) to achieve preliminary optimization of the duration of incubation of the CISTEM cell population with phage K3. The principle of this experiment is illustrated in figure 14.

*E. coli* MC4100 (pEGFP-N1), a kanamycin-resistant, ompA wild-type strain, which will here be referred to as U (for "Untagged"), was cultivated overnight in Luria broth (LB) supplemented with 25µg kanamycin/ml. *E. coli* UH203 (pEV218T7), which will here be referred to as T (for "Tagged"), is an ampicillin-resistant, ompA⁻ strain that has been transformed, as described above (V.iv.b.), by a plasmid encoding OmpA bearing the T7 tag sequence at the fourth extracellular loop of the polypeptide. This strain was cultivated overnight in LB supplemented with 100 µg/ml ampicillin/ml and 5 mM IPTG for induction of pEV218T7 expression. Each overnight culture was diluted with LB to OD₆₀₀ = 0.001. To create each of two mixed populations, 50µl aliquots of the U culture were combined with 50µl aliquots of the T culture. One mixed population was incubated for 20 min. at 37C with 20µl PBS alone, while the other incubated under the same conditions with 20µl PBS plus 0.1 µg anti-T7 tag mAb (Novagene mAbT7). (In table 4, the presence or absence of mAbT7 is indicated by a + or -.) Phage K3 diluted in 1 ml LB were then introduced to each mixed population at a Multiplicity of Infection (MoI) = 10. (Phage stock was prepared by the conventional method for preparation of T-even phages (34).) Sample aliquots of 100 µl were taken from each population at the appointed times shown in table 4, namely, immediately preceding introduction of phage (shown in the table as T = 0, Phage -), immediately following introduction of phage (shown in the table as T = 0, Phage +), and at 2 min., 10 min., 30 min., 60 min., 90 min., 120 min., 180 min., and 240 min. after the introduction of phage. Aliquots were plated on LB plates supplemented with 100 µg ampicillin/ml and on LB plates supplemented with 25 µg kanamycin/ml. Colonies were counted after incubation of the plates overnight at 37°C.

The results of this experiment demonstrated that interaction between mAbT7 and the T7 tag at loopIV of OmpA protected cells from phage K3. This conclusion is summarized most concisely in table 5. Cells exhibiting the T7 tag at loopIV of OmpA were protected from phage K3 infection, and this protection was dependent upon incubation of the cells with mAbT7. Cells that expressed wild-type OmpA, rather than the OmpA-T7 tag fusion were susceptible to phage K3, irrespective of the presence or absence of mAbT7. Thus, cells whose imprinted polypeptides bind the target compound are protected from K3 attack, whereas cells whose imprinted polypeptides fail to bind the target are eliminated by the phage.

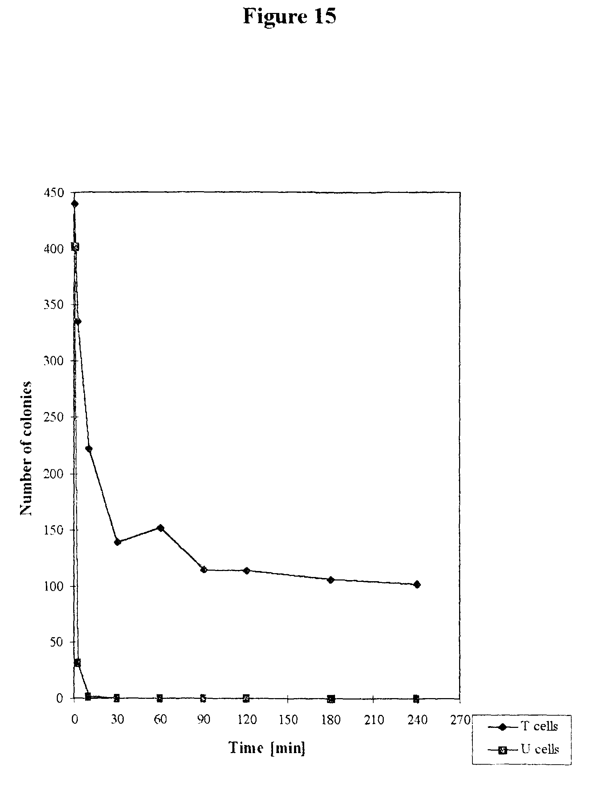

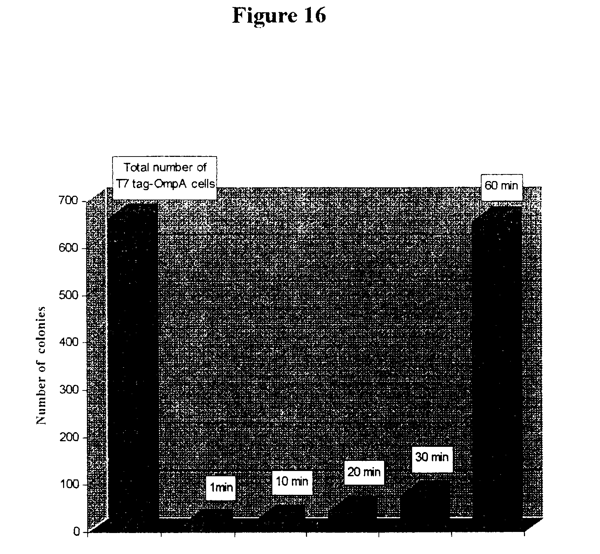

This experiment also serves in the preliminary optimization of CISTEM selection by phage K3. As is shown in table 4 and figure 15, in the mixed population of U and T cells that were incubated with mAbT7, U cells were completely eliminated from the population after 30 min. incubation with phage K3. T cells, by contrast, maintained a roughly stable population well beyond 30 min. of incubation with the phage. This suggested that when starting with MoI = 10, the minimum time period for phage K3 to eliminate all cells that failed to bind the target would be 30 - 60 min.

The initial precipitous drop in T cells followed by abrupt stabilization (see figure 15) suggested that the antibody may not yet have come to equilibrium in binding the T7 tag at the time the phage were introduced. Pre-incubation with the antibody was 20 min. in duration , and complete protection of T cells was apparently achieved at approximately 30 min. after the introduction of phage, suggesting that the time required for the antibody to come to equilibrium in binding the tag was approximately 50 min.. This suggestion was confirmed by the following preliminary optimization of the duration of the cell population's incubation with the target.

Strains U and T were cultivated overnight under the conditions described above. Each of five mixed populations of U and T cells was created by taking a 50µl aliquot of the U culture (OD₆₀₀ = 0.001) and combining it with a 50µl aliquot of the T culture (OD₆₀₀ = 0.001). As is shown in table 6, each mixed population was incubated at 37°C with 7nM mAbT7 diluted in PBS for a different time period: 1 min.; 10 min.; 20 min; 30 min; or 60 min.. Phage K3 stock, diluted with LB to 1 ml and MoI = 10, was added to each mixed cell population immediately following the population's incubation with the antibody. Each population was incubated with phage for 30 min. Aliquots were then taken and their composition of U and T cells determined as described above. As is shown in table 6, cells exhibiting wildtype OmpA remained unprotected from phage irrespective of the duration of incubation with mAbT7. By contrast, cells exhibiting the T7 tag at loop IV of OmpA received increasing protection from the antibody as the duration of incubation was lengthened. As was expected from the results the preliminary optimization of phage K3 incubation (described above) the antibody confered complete protection upon T cells only after an incubation time between 30 and 60 min.

### d) Initial test of CISTEM selection

The preliminary, single-parameter optimizations of incubation time with mAbT7 and the duration of K3 selection allowed an initial test of the CISTEM's capacity to select rare cells whose imprinted polypeptides bind the target from a background of cells whose imprinted polypeptides fail to bind the target. To demonstrate the selection of rare cells, the following procedure was performed.

*E. coli* strains U (Untagged) and T (Tagged) are described above. U was cultivated overnight in LB supplemented with 25µg kanamycin/ml. T was cultivated overnight in LB supplemented with 100µg ampicillin/ml and 1mM IPTG to induce expression of pEV218T7, the plasmid encoding OmpA with the T7 tag at loopIV of the polypeptide. Each overnight culture was diluted with LB to OD₆₀₀ = 0.005. A 50µl aliquot of the U culture was combined with a 50µl aliquot of the T culture to create a mixed population. As is shown in table 7, although the mixed population was created with equal sized aliquots of U and T cultures, it was composed predominantly of U cells. The reason for this was probably that the very low level of OmpA expression induced in the T strain (only 1mM IPTG was used) made this strain significantly less viable than the U strain under standard conditions of cultivation. The mixed population was incubated at 37C with 68nM mAbT7 (described above) diluted in PBS for 60 min.. Following incubation, 100 µl aliquots of the cell population were plated on LB plates supplemented with 25 µg kanamycin/ml or 100µg ampicillin/ml. Colonies were counted after overnight growth, showing that the population consisted of approximately 1 T cell for every 120 U cells (table 7). Phage K3 stock diluted in LB was then added to the cell population at MoI = 2, bringing the total volume to 1 ml. The cells were incubated with phage for 120 min. at 37C. Following incubation with phage, 100 µl aliquots of the cell population were plated on LB plates supplemented with 25 µg kanamycin/ml or 100µg ampicillin/ml. Colonies were counted after overnight growth.

Control experiments were performed according to the see protocol, except that PBS was added without mAbT7 and/or LB was added without phage K3, as shown in table 7. The results in table 7 demonstrated that rare cells (1 in >100) exhibiting an imprinted domain that bound the target were completely protected from phage K3 while all cells that failed to bind the target were quickly eliminated from the population.

A systematic test of the CISTEM's sensitivity in selecting rare cells that bind the target from a vast excess of cells that fail to bind the target is performed as follows. Overnight cultures of *E. coli* strains U (Untagged) and T (Tagged) are described above. 50 µl - 1 ml aliquots of the U and T overnight cultures are combined to create four populations, each representing a distinct ratio of U cells to T cells: 10⁸ U to 10⁴ T; 10⁸ U to 10³ T; 10⁸ U to 10² T; and 10⁹ U to 10² T. Each population is incubated for 60 min. at 37C with 10nM mAbT7. To monitor the population composition (i.e. the numbers of U and T cells), every 30 min. (i.e., at 30 min. and 60 min.) two aliquots of 5 µl are taken, one aliquot is plated on LB plates supplemented with 100µg Ap/ml, and the other aliquot is plated on LB supplemented with 25µg Km/ml. The plates are then incubated overnight and colonies counted to determine population composition. Immediately after removal of aliquots at 60 min., bacteriophage K3 (described above) are introduced to the cell population at MoI = 2. The admixture is incubated at 37°C for 180 min.. Again, to monitor the population composition, a sample aliquot of 5 µl is taken by micropipette every 20 min., plated, grown, and U and T cells counted as described above. After incubation with phage, a sample of approximately 1000 cells is removed, plated, grown, and its composition determined as described above.

Demonstration of the CISTEM's capacity to select rare cells that bind the target compound from a background of at least 100 times as many cells that fail to bind the target permits a final, two-parameter optimization of CISTEM performance. Each of five populations of T is induced for 15 min. by a distinct amount of IPTG in the medium: 0 mM; 0.25 mM; 0.5 mM; 1.0 mM; or 2.0 mM. Five mixed populations of 10⁵ U and 10³T, each representing a different level of induction of T cells, are created by combining aliquots, as described above. 10 nM mAbT7 is added to each population, and the populations are incubated for 60 min. at 37°C. During the 60 min. incubation, the population composition is monitored, as described above, by taking sample aliquots of 5 µl every 20 min., plating, growing, and counting the numbers of U and T cells. After the removal of an aliquot at 60 min., coliphage K3 is introduced at an MoI of 2. Over the next 180 min., the populations are monitored by 5 µl aliquot sampling every 20 min.. The [IPTG] and duration of incubation with K3 that result in the elimination of all U cells, with concomitant elimination of fewest T cells, are optimal parameter values for use in the CISTEM.

The optimization trials described here provide parameter-values ([IPTG], duration of incubation with K3, [mAbT7], duration of incubation with mAbT7) that allow the CISTEM to destroy all cells that fail to bind the target, while eliminating as few high-affinity cells as possible.

### e) Construction of an OmpA-imprinting CISTEM cell population

OmpA plasmid pEV218 (described above, shown in figure 9, construction described in 76), bears a polylinker rich in restriction endonuclease sites at a genetic region corresponding to extracellular loopIV of the polypeptide. This plasmid is digested with SacI (enzyme and reaction buffer from New England Biolabs) and XbaI (enzyme and reaction buffer from MBI Fermentas), under conditions described by the suppliers, yielding a fragment of the restriction site polylinker and the linearized plasmid. The linearized plasmid is isolated by standard gel electrophoresis (146). 10⁶ distinct, single-stranded oligonucleotides are synthesized by conventional technology (synthesis of variable oligonucleotides is described in depth in V.iv.c and 82) to read as follows: 5'NNGGTCTAGA(VNN)20CGAGCTCNN3' in which N represents all four nucleotides, each with equal probability; and V represents C, G, or A, each with equal probability. (*E. coli* strain UH203 is *sup*F, capable of translating the UAG stop-codon. Therefore, variable oligonucleotides inserted into the OmpA vector pEV218 must be free of the stop codons UAA and UGA, but not of UAG.) Using a standard protocol described in the literature (29), a 5' primer and a proof reading polymerase (such as the Vent® polymerase) are used to generate double-stranded oligonucleotides from the single-stranded templates. These double-stranded, highly variable oligonucleotides are then digested with SacI and XbaI, under conditions described by the suppliers (New England Biolabs and MBI Fermentas, respectively), yielding highly variable oligonucleotides with non-complementary sticky ends. The highly-variable oligonucleotides are then ligated (MBI ligation buffer, conditions described by supplier) into pEV218, linearized as described in the previous paragraph. Because the SacI and XbaI sticky ends are non-complementary, they ligate in only one oligonucleotide per plasmid, and in only one orientation. Conventional electrophoretic size fractionation (146) is used to isolate ligated plasmids containing an insert. The product of this procedure is a population of plasmids (collectively designated pEV218I) bearing, at the genetic site corresponding to loop IV of OmpA, a highly variable exogenous sequence free of UAA and UGA stop codons.

The variable plasmid population, pEV218I, is used to transform *E. coli* strain UH203 (*lac*, *sup*F, *omp*A, *rec*A, *pro*AB, *rps*L) (this strain, its propagation and its transformation, are described more completely above, and in 74 and 76). The UH203 population is grown in Luria broth supplemented with 100µg Ap/ml, yielding a population of 10⁸ cells comprising 10⁶ clones, each one of which carries a distinct member of the pEV218I population.

### f) CISTEM-selection for OmpA exhibiting high-affinity for mAbT7

The UH203 (pEV218I) population of 10⁸ cells plus approximately 100 UH203 (pEV218T7) cells is incubated at 37C for 15 min. in 100ml LB medium supplemented with 100 µg Ap/ml and IPTG between 0.25 and 2.0 mM, as determined in the optimization trial described above. This yields a population of 10⁸ clones, comprising 10⁶ distinct clones, each of which displays a distinct, exogenous, oligopeptide sequence at loop IV of OmpA. One of these clones displays the T7 tag sequence. After the initial 15 min. induction of pEV218I/pEV218T7 expression, 10nM mAbT7 is added to the 100ml of cells. The cells are incubated with the mAb for 60 min. at 37°C. Coliphage K3 is then introduced at MoI = 2 (the procedure for growth and handling of K3 is described above, and in 76, 126, and 127). Incubation with K3 proceeds for the minimum amount of time required, as determined in the optimization trial described above. Following incubation with phage, the cell population is centrifuged to separate cells from phage and the products of lysis, and cells are resuspended in 100ml NB, without 100mM NaCl supplement, to guarantee complete cessation of infection by K3 (incapacity of phage K3 to infect in NB without NaCl supplement is demonstrated in 126). This cell population represents the set of clones selected by the CISTEM for binding to the target compound mAbT7.

To amplify the numbers of selected clones, the selected cell population is grown to 10⁶ cells at 37°C in 100ml NB. Expression of OmpA is then heavily induced by 2.5 mM IPTG for 15 min.. Following induction, 10⁴ cells from the selected population are plated onto NB agar containing 0.5% sodium deoxycholate. (Sodium deoxycholate prevents growth of cells that fail to insert OmpA in the outer membrane; such cells are "false positives", selected by the CISTEM simply because they display no OmpA, and not because of their capacity to bind mAbT7 at loop IV of OmpA. The sodium deoxycholate supplement also prevents K3 infection, providing a further guarantee against continuing infection.) Plated cells are incubated at 37°C to grow colonies, which are composed of cells in which loop IV of OmpA contains an exogenous oligopeptide with high affinity for the target compound, mAbT7.

### ii. EXAMPLE 2: AN E. COLI OMPA/COLIPHAGE K3/NEISSERIA MENINGITIDIS ANTIBODY CISTEM

### a) E. coli Outer membrane protein A and Neisserial Opa proteins

Several recent findings show that one important OmpA function explaining such high levels of expression and evolutionary conservation of the polypeptide is adhesion to and invasion of host tissues. It has recently been demonstrated that *E. coli* OmpA contributes significantly to invasion of brain microvascular endothelial cells-a critical step in pathogenesis of neonatal *E. coli* meningitis. Similarly, OmpA homologs are directly involved in microbial adherence, invasion of host cells, and pathogenesis by *Haemophilus influenzae* (154 demonstrates virulence due directly to an OmpA homolog), *Neisseria meningitidis* (85 and 167 demonstrate virulence due directly to OmpA homologs), *Neisseria gonorrhoeae* (15, 84, 85, 117, and 160 demonstrate virulence due directly to OmpA homologs), and other virulent microbes (74, 81).

The structural homology between OmpA and a wide range of antigenic adhesins makes OmpA well-suited for imprinting for use in applications requiring mimicry of homologous adhesins. One such application is vaccination. When a polypeptide (in this case, OmpA) is imprinted to bind to antibodies composing the sera raised against an antigen, the imprinted polypeptide may elicit an immune response very similar to the one provoked by the antigen (97). Antibodies suitable for use as the target compound in a CISTEM for imprinting *E. coli Omp*A to mimic *Neisseria meningitidis* Opa proteins (described in 1, 2, 130, and 167) include mAb B33 (described in 167), which reacts with all Opa proteins, mAbs P322 and P514 (described in 2), which react with a broad subset of Opa proteins, and mAb B306 (described in 2), mAb A222/5b (described in 130), and others described in the literature.

### b) CISTEM-selection for OmpA exhibiting high-affinity for antibodies against Neisseria meningitidis Opa proteins

The construction of a cell population for testing and optimizing CISTEM selection, the optimization and demonstration of CISTEM function, and the construction of an OmpA-imprinting CISTEM cell population are performed exactly as described above (VI.i.a-e): this CISTEM, like the one described in Example 1, uses phage K3 to select from a cell population displaying variable inserts 20 amino acids in length at loop IV of OmpA. However, whereas Example 1 imprints OmpA to bind to mAbT7, this CISTEM imprints OmpA to bind to antibodies against *Neisseria meningitidis* Opa proteins. Therefore, where the protocol described in Example 1, part f, calls for 10 nM mAbT7, this CISTEM calls for 10 nM of each of the following antibodies: B33; P322; P514; B306; and A222/5b. Aside from this substitution, the protocol for imprinting OmpA to bind anti-Opa antibodies is as described in Example 1, part f.

### TABLES

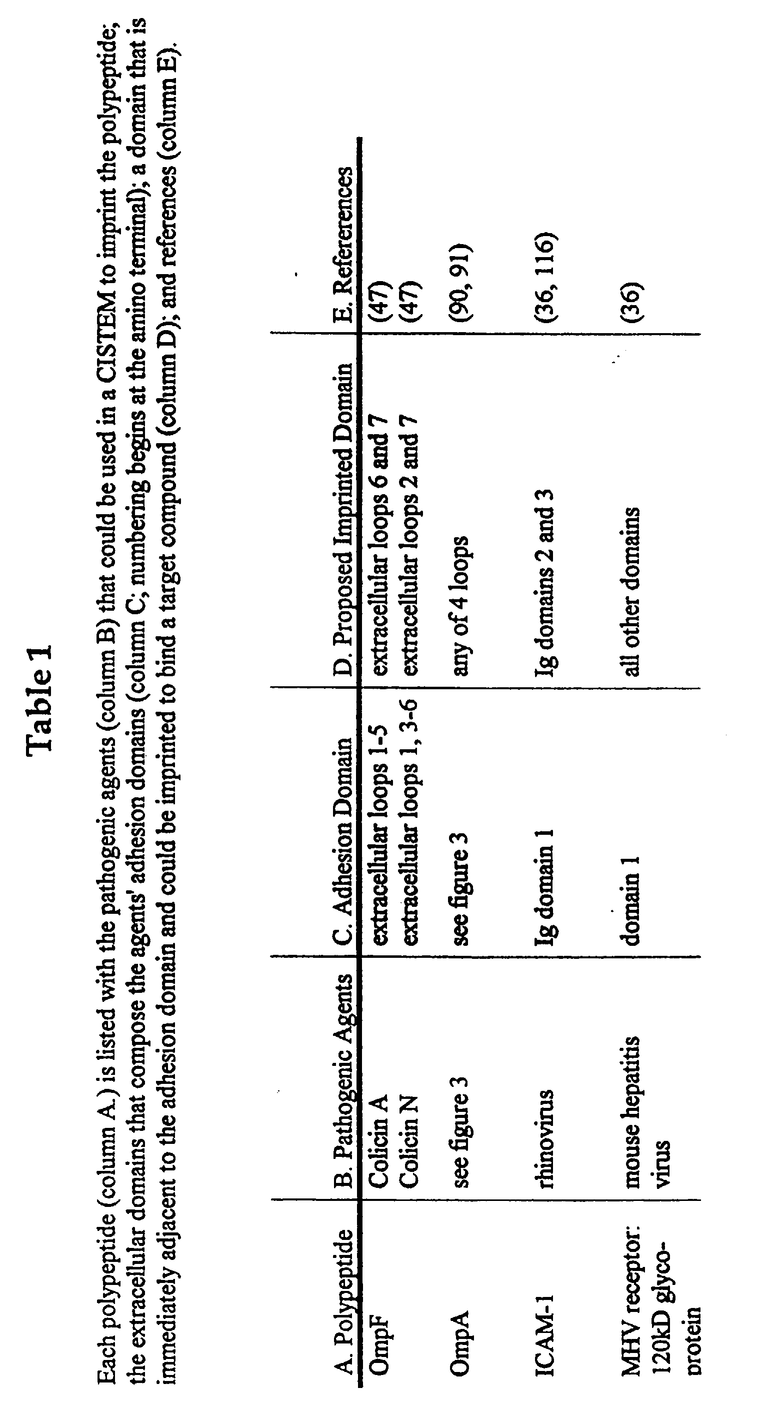

**Table 2**

| **Component** | **Intracellular Conc. (mM)** | **Extracellular Concentration (mM)** |
|---|---|---|
| ***Cations*** | | |
| **Na+** | **5-15** | **145** |
| **K+** | **140** | **5** |
| **Mg2+** | **30** | **1-2** |
| **Ca2+** | **1-2** | **2.5-5** |
| **H+** | **4x10**^{**-5**} **or pH 7.4** | **4x10**^{**-6**} **or pH 7.4** |

| ***Anions*** | | |
|---|---|---|
| **Cl-** | **4** | **110** |

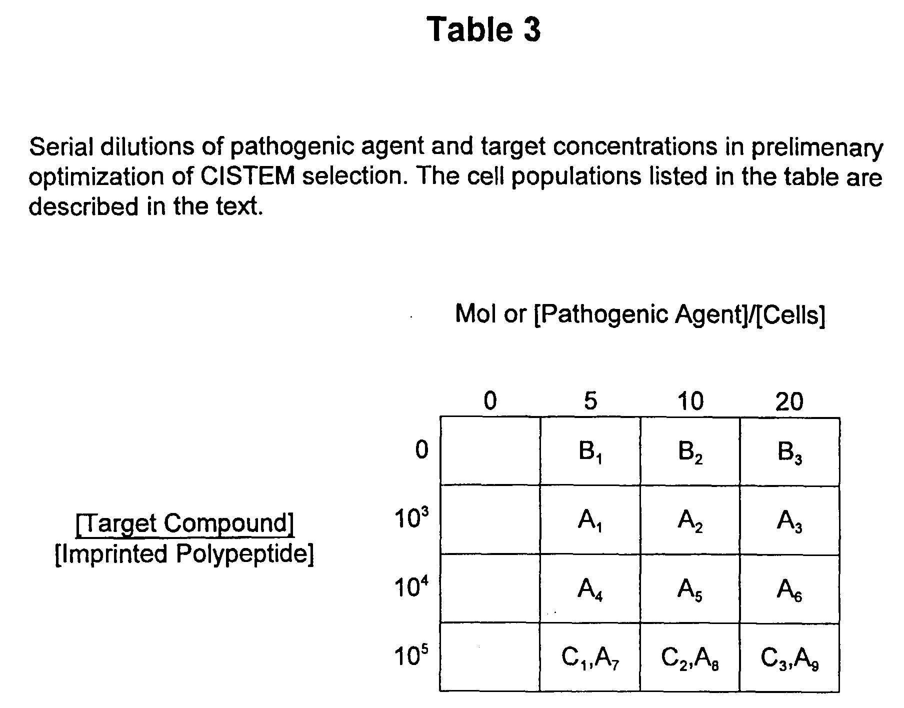

**Table 4**

| Time [min] | Phage | Antibody | Selection | Selected | Number of colonies |
|---|---|---|---|---|---|
| 0 | - | - | LB | both | 1085 |
| | - | - | Ap | T | 598 |
| | - | - | Km | U | 402 |
| | + | - | Ap | T | 204 |
| | + | - | Km | U | 34 |
| | + | + | Ap | T | 440 |
| | + | + | Km | U | 21 |
| 2 | + | - | Ap | T | 138 |
| | + | - | Km | U | 30 |
| | + | + | Ap | T | 336 |
| | + | + | Km | U | 31 |
| 10 | + | - | Ap | T | 67 |
| | + | - | Km | U | 3 |
| | + | + | Ap | T | 222 |
| | + | + | Km | U | 2 |
| 30 | + | - | Ap | T | 23 |
| | + | - | Km | U | 0 |
| | + | + | Ap | T | 139 |
| | + | + | Km | U | 0 |
| 60 | + | - | Ap | T | 3 |
| | + | - | Km | U | 0 |
| | + | + | Ap | T | 152 |
| | + | + | Km | U | 0 |
| 90 | + | - | Ap | T | 3 |
| | + | - | Km | U | 0 |
| | + | + | Ap | T | 114 |
| | + | + | Km | U | 0 |
| 120 | + | - | Ap | T | 0 |
| | + | - | Km | U | 0 |
| | + | + | Ap | T | 113 |
| | + | + | Km | U | 0 |
| 180 | + | - | Ap | T | 0 |
| | + | - | Km | U | 0 |
| | + | + | Ap | T | 106 |
| | + | + | Km | U | 0 |
| 240 | + | - | Ap | T | 0 |
| | + | - | Km | U | 0 |
| | + | + | Ap | T | 102 |
| | + | + | Km | U | 0 |

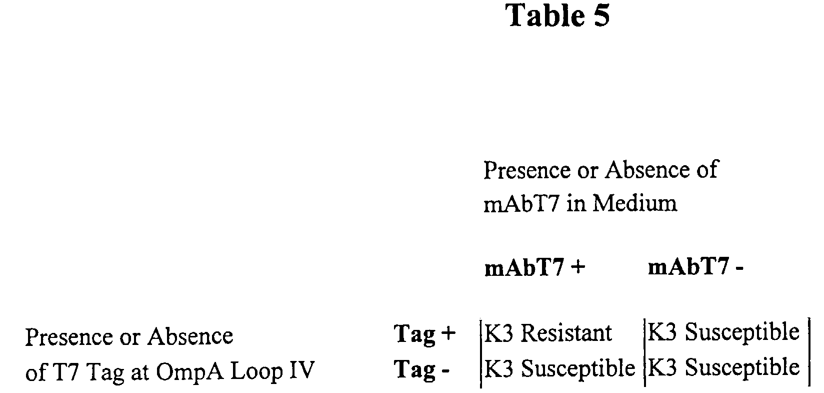

**Table 6**

| Time [min] | Phage | Antibody | Selection | Selected | Number of colonies |
|---|---|---|---|---|---|
| 0 | - | - | LB | both | 1188 |
| | - | - | Ap | T | 663 |
| | - | - | Km | U | 484 |
| | + | - | Ap | T | 12 |
| | + | - | Km | U | 0 |
| 1 | + | + | Ap | T | 18 |
| | + | + | Km | U | 0 |
| 10 | + | + | Ap | T | 24 |
| | + | + | Km | U | 1 |
| 20 | + | + | Ap | T | 43 |
| | + | + | Km | U | 0 |
| 30 | + | + | Ap | T | 75 |
| | + | + | Km | U | 0 |
| 60 | + | + | Ap | T | 655 |
| | + | + | Km | U | 0 |

**Table 7**

| Time [min] | Phage | Antibody | Selection | Selected | Number of cells |
|---|---|---|---|---|---|
| 0 | - | - | amp | T7 tag-OmpA | 616 |
| | - | - | kan | wt OmpA | 75400 |
| 120 | - | - | amp | T7tag-OmpA | 2867 |
| | - | - | kan | wt OmpA | 476000 |
| | + | - | amp | T7tag-OmpA | 0 |
| | + | - | kan | wt OmpA | 0 |
| | + | + | amp | T7 tag-OmpA | 2845 |
| | + | + | kan | wt OmpA | 0 |

### BIBLIOGRAPHY

1. Achtman, M. (1990) Molecular epidemiology of epidemic bacterial meningitis. Review of Medical Microbiology 1: 29-38.
2. Achtman, M. et al. (1992) A comparison of the variable antigens express by clone IV-1 and subgroup III of Neisseria meningitidis serogroup A. Journal of Infectious Disease 165: 53-68.
3. Ackerman, H-W. (1992) Bacteriophages. In Lederberg, J., ed. Encyclopedia of Microbiology vol 1. Academic Press, New York.
4. Agterberg, M, H. Adriaanse, A. van Bruggen, M. Karperin, and J. Tommassen (1990) Outer membrane PhoE protein of *Escherichia coli* K-12 as an exposure vector: possibilities and limitations. Gene 88: 37-45.
5. Akiyama, Y., and K. Ito (1987) Topology analysis of the SecY protein, an integral membrane protein involved in protein export in *Escherichia coli.* EMBO Journal 6: 3465-3470.
6. Akiyama, Y., and K. Ito (1989) Export of *E. coli* alkaline phosphatase attached to an integral membrane protein, SecY. Journal of Biological Chemistry 264: 437-42.
7. Ames, R.S. et al. (1995) Conversion of murine Fabs isolated from a combinatorial phage display library to full length immunoglobulins. Journal of Immunological Methods 184 (2): 177-186.
8. Ames, R.S. et al. (1995) Neutralizing murine monoclonal antibodies to human IL-5 isolated from hybridomas and a filamentous phage Fab display library. Journal of Immunology 154 (12): 6355-64.
9. Arp. J., et al. (1996) A source of glycosylated human T-cell lymphotropic virus type 1 envelope protein: expression of gp46 by the vaccinia virus/T7 polymerase system. Journal of Virology 70: 7349-7359.
10. Atlas, R. (1993) Handbook of Microbiological Media. CRC Press, Boca Raton, FL.
11. Balint, R.F. and J.W. Larrick (1993) Antibody engineering by parsimonious mutagenesis. Gene 137: 109-118.
12. Barry, M.A. et al. (1996) Toward cell-targeting gene therapy vectors: selection of cell-binding peptides from random peptide-presenting phage libraries. Nature Medecine 2(3): 299-305.
13. Baty, D., R. Lloubes, V. Geli, C. Lazdunski, S.P. Howard (1987) Extracellular release of colicin A is non-specific. EMBO Journal 6: 2463-2468.
14. Beck, E., and E. Bremer (1980) Nucleotide sequence of the gene *omp*A coding the outer membrane protein II∗ of *Escherichia coli* K-12. Nucleic Acids Research 8: 3011-3023.
15. Bessen, D., and E.C. Gotschlich (1986) Interactions of *gonococci* with HeLa cells: attachment, detachment, replication, penetration, and the role of protein II. Infection and Immunity 54: 154-160.
16. Bhat, K.S., C.P. Gibbs, O. Barrera, S.G. Morrison, F. Jahning, A. Stern, E.M. Kupsch, T.F. Meyer, and J. Swanson (1991) The opacity proteins of *Neisseria gonorrhoeae* strain MS11 are encoded by a family of 11 complete genes. Molecular Microbiology 5: 1889-1901.
17. Blobel, G. (1980) Intracellular protein topogenesis. Proceedings of the National Academy of Science USA 77: 1496-1500.
18. Block, C. et al. (1996) Quantitative structure-activity analysis correlating Ras/Raf interaction in vitro to Raf activation in vivo. Nature Structural Biology 3 (3): 244-251.
19. Bos, J.L. (1989) ras oncogenes in human cancer: a review. Cancer Research 49: 4682-4689.
20. Boulanger and Letellier (1988) Characterization of ion channels involved in the penetration of phage T4 into E. coli cells. Journal of Biological Chemistry 263 (20): 9767-9775.
21. Boyd, D., C. Maniol, and J. Beckwith (1987) Determinants of membrane protein topology. Proceedings of the National Academy of Science USA: 84: 8525-8529.
22. Boyd, D., B. Traxler, G. Jander, W. Prinz, and J. Beckwith (1993) Gene fusion approaches to membrane protein topology. Society of General Physiologists Series 48: 23-37.
23. Boyd, D. (1994) Use of gene fusion to determine membrane protein topology. pp. 144-163 in White, S.H., ed. Membrane Protein Structure: Experimental Approaches. Oxford, New York.
24. Branden, C., and J. Tooze (1991) Introduction to Protein Structure. Garland, New York.
25. Braun, G., and S.T. Cole (1982) The nucleotide sequence coding for major outer membrane protein *omp*A of *Shigella dysenteriae*. Nucleic Acids Research 10: 2367-2378.
26. Braun, G., and S.T. Cole (1983) Molecular characterization of the gene coding for major outer membrane protein ompA from Enterobacter aerogenes. European Journal of Biochemistry 137: 494-500.
27. Braun. G., and S.T. Cole (1984) DNA sequence analysis of the *Serratia marcescens omp*A gene: Implications for the organization of anenterobacterial outer membrane protein. Molecular General Genetics 195: 321-328.
28. Brown, S. (1992) Engineered iron oxide-adhesion mutants of the *Escherichia coli* phage 1 receptor. Proceedings of the National Academy of Science, USA 89: 8651-8655.
29. Brown, S. (1994) Method for identifying and expressing proteins that recognize and adhere to specific probes. United States Patent # 5,316,922.
30. Bull, J.J., I.J. Molineux, W.R. Rice (1991) Selection of benevolence in a host-parasite system. Evolution 45: 875-882.
31. Bull, J.J., and I.J. Molineux (1992) Molecular genetics of adaptation in an experimental model of cooperation. Evolution 46: 882-895.
32. Burnie, J.P. et al. (1996) Defining antibody targets in *Streptococcus oralis* infection. Infection and Immunity 64 (5): 1600-8.
33. Calcutt, M.J., M.T. Kremer, M.F. Giblin, T.P. Quinn, and S.L. Deutscher (1993) Isolation and characterization of nucleic acid-binding antibody fragments from autoimmune mice-derived bacteriophage display libraries. Gene 137 (1): 77-83.
34. Calendar, R. (1988) ed. The Bacteriophages vols. 1 and 2. Plenum, New York.
35. Cann, A.J. and I.S. Chen (1996) Human T-Cell Leukemia Virus Types I and II. in Fields, B.N. et al.:
36. Caras, I.W., and G.N. Weddell (1989) Signal peptide for protein secretion directs glycophospholipid membrane anchor attachment. Science 243: 1196-1198.
37. Caras, I.W., G.N. Weddell, and S.R. Williams (1989) Analysis of the signal for attachment of a glycophospholipid membrane anchor. Journal of Cell Biology 108: 1387-1396.
38. Charbit, A, E. Sobczak, M.L. Michel, A. Molla, P. Tiollais, and M. Hofnung (1987) Presentation of two epitopes of the preS2 region of hepatitis B virus on live recombinant bacteria. Journal of Immunology 139: 1658-1664.
39. Charbit, A., J. Ronco, M.L. Michel, C. Werts, and M. Hofnung (1991) Permissive sites and topology of an outer membrane protein with a reporter epitope. Journal of Bacteriology 173: 262-275.
40. Charbonnier, J.B. et al. (1995) Crystal structure of the complex of a catalytic antibody Fab fragment with a transition state analog: structural similarities in esterase-like catalytic antibodies. Proceedings of the National Academy of Sciences, USA 92(25): 11721-5.
41. Chavez, R.A., and Z.A. Hall (1991) The transmembrane topology of the animo terminus of the alpha subunit of the nicotine acetylcholine receptor. Journal of Biological Chemistry 266: 15532-15538.
42. Chen, R., W. Schmidmayr, C. Kr"mer, U. Chen-Schmeisser, and U. Henning (1980) Primary structure of major outer membrane protein II* (ompA protein) of *Escherichia coli* K-12. Proceedings of the National Academy of Science USA 77: 4592-4596.
43. Chen, W. et al. (1997) Expression and purification of human interleukin-1beta converting enzyme from Trichoplusia ni insect cells using a baculovirus expression system. Protein Expression and Purification 9: 69-75.
44. Chu, T.H., and R. Dornburg (1995) Retroviral vector particles displaying the antigen binding site of antibody enable cell-type specific gene transfer. Journal of Virology 69: 2659-2663.
45. Clackson, T., H.R. Hoogenboom, A.D. Griffiths, and G. Winter (1991) Making antibody fragments using phage display libraries. Nature: 352: 624-628.
46. Claesson-Welsh, L., A. Eriksson, B. Westermark, and C.-H. Helden (1989) cDNA cloning and expression of the human a-type platelet-derived growth factor (PDGF) receptor establishes structural similarity to the B-type PDGF receptor. Proceedings of the National Academy of Science USA 86: 4917-4921.
47. Clayton, L.K., M. Sieh, D.A. Pious, and E.L. Reinherz (1989) Identification of human CD4 residues affecting class II MHC versus HIV-1 gp120 binding. Nature 339: 548-551.
48. Cole, S.T., U. Chen-Schmeisser, I. Hindenach, and U. Henning (1983) Apparent bacteriophage-binding region of an *Escherichia coli* K-12 outer membrane protein. Journal of Bacteriology 153: 581-587.
49. Colonno, R.J., J.E. Tomassini, and P.L. Callahan (1987) Isolation and characterization of a monoclonal antibody which blocks attachment of human rhinoviruses. pp. 93-102 in Brinton, M.A. and R.R. Rueckert, eds. Positive Strand RNA Viruses. Alan R. Liss Inc., New York.
50. Colonno, R.J. (1992) Molecular interaction between human rhinoviruses and the adhesion receptor ICAM-1. pp. 32-41 in M. Hook and L. Switalski, eds. Microbial Adhesion and Invasion. Springer Verlag, New York.
51. Cortese, R. et al. (1994) Epitope discovery using peptide libraries displayed on phage. Trends in Biotechnology 12 (7): 262-7.
52. Couch, R.B. (1996) Rhinoviruses: propagation in cell culture. In Fields, B.N. et al.: 714-717
53. Crise, B., A. Ruusala, P. Zagouras, A. Shaw, and J.K. Rose (1989) Oligomerization of glycolipid-anchored and soluble forms of the Vesicular Stomatitis Virus glycoprotein. Journal of Virology 63: 5328-5333.
54. Cross, G.A. (1990) Glycolipid anchoring of plasma membrane proteins. Annual Review of Cell Biology 6: 1-39.
55. Cwirla, S.E., E.A. Peters, R.W. Barrett, and W.J. Dower (1990) Peptides on phage: a vast library of peptides for identifying ligands. Proceedings of the National Academy of Science USA 87: 6378-82.
56. D'Andrea, A.D., G.D. Fasman, and H.F. Lodish (1989) Erythropoietin receptor and interleukin-2 b-chain: A new receptor family. Cell 58: 1023-1024.
57. Daum, G. et al. (1994) The ins and outs of Raf kinases. Trends in Biochemical Science 19: 474-480.
58. Devlin, J.J., L.C. Panganiban, and P.E. Devlin (1990) Random peptide libraries: A source of specific binding molecules. Science 249: 404-406.
59. Ditzel HJ, J.M. Binley, J.P. Moore, et al. (1995) Neutralizing recombinant human antibodies to a conformational V2- and CD4-binding site-sensitive epitope of HIV-1 gp120 isolated by using an epitope-masking procedure. Journal of Immunology 154: 893-906.
60. Dorsey, M.J., H.J. Tae, K. G. sollenberger, N.T. Mascarenhas, L.M. Johansen, and E.J. Taparowsky (1995) B-ATF: a novel human bZIP protein that associates with members of the AP-1 transcription factor family. Oncogene 11 (11): 2255-65.
61. Drickamer, K. and M.E. Taylor (1993) Biology of animal lectins. Annual Review of Cell Biology 9: 237-264.
62. Dwek, R.A., C.J. Edge, D.J. Harvey, M.R. Wormald, and R.B. Parekh (1993) Analysis of glycoprotein-associated oligosaccharides. Annual Review of Biochemistry 62: 65-100.
63. Eigen, M. (1993) The origin of genetic information: viruses as models. Gene 135: 37-47.
64. Ellington, A.D., and J.W. Szostak (1990) In vitro selection of RNA molecules that bind specific ligands. Nature 346: 818-822.
65. Ellington, A.D., and J.W. Szostak (1992) Selection in vitro of single stranded DNA molecules that fold into specific ligand-binding structures. Nature 355: 850-852.
66. Fantini, J. et al. (1997) Synthetic soluble analogs of galactosylceramide (GalCer) bind to the V3 domain of HIV-1 gp120 and inhibit HIV-1-induced fusion and entry. Journal of Biological Chemistry 272: 7245-7252.
67. Ferran M.C. and J.M. Lucas-Lenard (1997) The vesicular stomatitis virus matrix protein inhibits transcription from the human beta interferon promoter. Journal of Virology 71: 371-377.
68. Fields, B.N., D.M. Knipe, D.M. Hawley, et al., eds. Virology, 3rd ed. Lippincott Raven, Philadelphia.
69. Fourel, D., C. Hikita, J.M. Bolla, S. Mizushima, and J.M. Pages (1990) Characterization of OmpF domains involved in *Escherichia coli* K-12 sensitivity to colicins A and N. Journal of Bacteriology 172: 3675-3680.
70. Francisco, J.A., et al. (1993) Specific adhesion and hydrolysis of cellulose by intact *Escherichia coli* expressing surface anchored cellulase or cellulose binding domains. Biotechnology 11: 491-495.
71. Francisco, J.A., et al. (1993) Production and fluorescence-activated cell sorting of *Escherichia coli* expressing a functional antibody fragment on the external surface. Proceedings of the National Academy of Science USA 90: 10444-8.
72. Frenette, M., H. Benedetti, A. Bernadac, D. Baty, and C. Lazdunzki (1991) Construction, Expression and Release of Hybrid Colicins. Journal of Molecular Biology 217: 421-428.
73. Freudl, R., and S.T. Cole (1983) Cloning and molecular characterization of the *omp*A gene from *Salmonella typhimurium*. European Journal of Biochemistry 134: 497-502.
74. Freudl, R. et al. (1985) Lethal mutations in the structural gene of an outer membrane protein (OmpA) of *E. coli* K-12. Molecular General Genetics 201: 76-81.
75. Freudl, R., S. MacIntyre, M. Degen and U. Henning (1986) Cell surface exposure of the outer membrane protein ompA of *Escherichia coli* K-12. Journal of Molecular Biology 188: 491-494.
76. Freudl, R. (1989) Insertion of peptides into cell-surface-exposed areas of the *Escherichia coli* OmpA protein does not interfere with export and membrane assembly. Gene 82: 229-236.
77. Fuchs, A. et al. (1995) Mapping the domains of interaction of p40phox with both p47phox and p67phox of the neutrophil oxidase complex using the two-hybrid system. Journal of Biological Chemistry (HIV) 270(11): 5695-7.
78. Gentry-Weeks, C.R., A-L. Hultsch, S.M. Kelly, J.M. Keith, and R. Curtiss III (1992) Cloning and sequencing of a gene encoding a 21-kilodalton outer membrane protein from *Bordetella avium* and expression of the gene in *Salmonella typhimurium*. Journal of Bacteriology 174: 7729-7742.
79. Giblin, P., J.A. Ledbetter, and P. Kavathas (1989) A secreted form of the human lymphocyte cell surface molecule CD8 arises from alternative splicing. Proceedings of the National Academy of Science, USA 86: 998-1002.
80. Giver, L., D.P. Bartel, M.L. Zapp, M.R. Green, and A.D. Ellington (1993) Selection and design of high affinity RNA ligands for HIV-1 Rev. Gene 137: 19-24.
81. Giver, L. D. Bartel, M. Zapp, A. Pawul, M. Green, and A.D. Ellington (1993) Selective optimization of the Rev-binding element of HIV-1. Nucleic Acids Research 21: 5509-5516.
82. Gold, L. and C. Tuerk (1993) Methods for identifying nucleic acid ligands. United States Patent #5,270,163.
83. Gold, L., B. Polisky, O. Uhlenbeck, and M. Yarus (1995) Diversity of oligonucleotide functions. Annual Reviews of Biochemistry 64: 763-797.
84. Gorby, G.L., and G.B. Schaefer (1992) Effect of attachment factors (pili plus opa) on *Neisseria gonorrhoeae* invasion of human fallopian tube tissue in vitro. Microbial Pathogenesis 13: 93-108.
85. Gotschlich, E.C. (1986) Conserved gonococcal surface antigens. in A. Tagliabue et al. (eds.) Bacterial Vaccines and Local Immunity. Ann. Sclavo, Siena, Italy.
86. Granellin, C.R. (1992) pp. 3-12 in Wermuth, C.G., N. Koga, H. Konig, and B.W. Metcalf, eds., Medicinal Chemistry for the 21st Century. Blackwell, London.
87. Green, R., R.A. Kramer, and D. Shields (1989) Misplacement of the amino-termina positive charge in the prepro-alpha-factor signal peptide disrupts membrane translocation in vivo. Journal of Biological Chemistry (HIV) 264: 2963-2968.
88. Hagen, D.C., G. McCaffrey, and G.F. Sprague Jr. (1986) Evidence the yeast STE3 gene encodes a receptor for the peptide pheromone A factor: Gene sequence and implications for the structure of the presumed receptor. Proceedings of the National Academy of Science USA 83: 1418-1422.
89. Hanahan, D. (1985) in DNA cloning. ed. D.M. Glover. IRL, Oxford: 109-135.
90. Hashemolhosseini, S., Z. Holmes, B. Mutschler, and U. Henning (1994) Alterations of receptor specificities of coliphages of the T2 family. Journal of Molecular Biology 240: 105-110.
91. Hayden, F.G., J.M. Gwaltney, Jr., and R.J. Colonno (1988) Modification of experimental rhinovirus colds by receptor blockade. Antiviral Research 9: 233-247.
92. Henning, U., B. Hohn, and I. Sonntag (1973) Cell envelope shape of *Escherichia coli* K-12. The ghost membrane. European Journal of Biochemistry 39: 27-36.
93. Henning, U., and I. Haller (1975) Mutants of *Escherichia coli* K12 lacking all "major" proteins of the outer cell envelope membrane. FEBS Letters 55: 161-164.
94. Herrmann, C., G.A. Martin, and A. Wittinghofer (1995) Quantitative analysis of the complex between p21ras and the Ras-binding domain of the human Raf-1 protein kinase. Journal of Biological Chemistry 270: 2901-2905.
95. Hibi, M. et al. (1990) Molecular cloning and expression of an IL-6 signal transducer, gp130. Cell 63: 1149-1157.
96. Hobom, G. et al. (1995) OmpA fusion proteins for presentation of foreign antigens on the bacterial outer membrane. Development and Biology Standard 84: 255-62.
97. Hoogenboom, H.R., A.D. Griffiths, K.S. Johnson, D.J. Chiswell, P. Hudson, and G. Winter (1991) Mult-subunit proteins on the surface of filamentous phage: methodologies for displaying antibody (Fab) heavy and light chains. Nucleic Acids Research 19: 4133-4137.
98. Huse, W.D., L. Sastry, S.A. Iverson, A.S. Kang, M. Alting-Mees, D.R. Burton, S.J. Benkovic, and R.A. Lerner (1989) Generation of a large combinatorial library of the immunoglobulin repertoire in phage lambda. Science 246: 1275-1281.
99. Janda, K.D. (1994) Tagged versus untagged libraries: methods for the generation and screening of combinatorial chemical libraries. Proceedings of the National Academy of Science, USA: 10779-10785.
100. Janknecht, R. et al. (1995) Signal integration at the c-fos promoter. Carcinogenesis 16: 443-450.
101. Kasahara, N, A.M Dozy, and Y.W. Kan (1994) Tissue specific targeting of retroviral vectors through ligand-receptor interactions. Science 266:1373-1375.
102. Keegan, K. and J.A. Cooper (1996) Use of the two hybrid system to detect the association of the protein-tyrosine-phosphatase, SHPTP2, with another SH2-containing protein, Grb7. Oncogene 12(7): 1537-44.
103. Kinet, J.-P. (1989) Antibody-cell interaction: Fc receptors. Cell 57: 351-354.
104. Klose, M., A. Störiko, Y-D. Stierhof, I. Hindennach, B. Mutschler, and U. Henning (1993) Membrane assembly of the outer membrane protein OmpA of *E. coli*. The Journal of Biological Chemistry 268: 25664-25670.
105. Koebnik, R., and V. Braun (1993) Insertion derivatives containing segments of up to 16 amino acids identify surface-and periplasm-encoded regions of the fhuA outer membrane receptor of *E. coli* K-12: Journal of Bacteriology 175: 826-839.
106. Konisky, J. (1982) Colicins and other bacteriocins with established modes of action. Annual Review of Microbiology 36: 125-144.
107. Larsen, R.D., V.P. Rajan, M.M. Ruff, J. Kukowska-Latallo, and R.D. Cummings (1989) Isolation of a cDNA encoding a murine UDP galactose: □-D-galactosyl-1,4-N-acetyl-D-glucosaminide a-1,3-glactosyltransferase: Expression cloning by gene transfer. Proceedings of the National Academy of Science 86: 8227-8231.
108. Lawrence, J.G., H. Ochman, and D.L. Hartl (1991) Molecular and evolutionary relationships among enteric bacteria. Journal of General Microbiology 137: 1911-1921.
109. Lederberg, E.M. and S.N. Cohen (1974) Transformation of *Salmonella typhimurium* by plasmid deoxyribonucleic acid. Journal of Bacteriology 119: 1072-1074.
110. Lineberger, D.W., D.J. Graham, J.E. Tomassini, and R.J. Colonno (1990) Antibodies that block rhinovirus attachment map to domain 1 of the major group receptor. Journal of Virology 64: 2582-2587.
111. Lis, H., and N. Sharon (1993) Protein glycosylation: Structural and functional aspects. European Journal of Biochemistry 218: 1-27
112. Lisanti, M.P., I.W. Caras, M.A. Davitz, and E. Rodriguez-Boulan (1989) A glycophospholipid membrane anchor acts as an apical targetting signal in polarized epithelial cells. Journal of Cell Biology 109: 2145-2156.
113. Low, M.G. (1987) Biochemistry of the glycosyl-phosphatidylinositol membrane protein anchors. Biochemical Journal 244: 1-13.
114. Luciw, P.A. (1996) Human Immunodeficieny Viruses and their replication. In Fields, B.N. et al.:
115. Lugtenberg, B., and L. van Alphen (1983) Molecular architecture and functioning of the outer membrane of *E. coli* and other gram negative bacteria. Biochimica et Biophysica Acta 737: 51-115.
116. Madaule, P. et al. (1995) A novel partner for the GTP-bound forms of rho and rac. FEBS Letters 377(2): 243-8.
117. Makino, S., J.P. van Putten, and T.F. Meyer (1991) Phase variation of the opacity outer membrane protein controls invasion by *Neisseria gonorrhoeae* into human epithelial cells. The EMBO Journal 10: 1307-1315.
118. Marks, J.D., H.R. Hoogenbom, T.P. Bonnert, J. McCafferty, A.D. Griffiths, and G. Winter (1991) By-passing immunization: human antibodies from V-gene libraries displayed on phage. Journal of Molecular Biology 222: 581-597.
119. Marshall, M.S. (1995) Ras target proteins in eukaryotic cells. FASEB Journal 9: 1311-1318.
120. Mauri, I., M. Maddaloni, S. Lohmer. M. Motto, F. Salamini, R. Thompson, and E. Martegani (1993) Functional expression of the transcriptional activator Opaque-2 of *Zea mays* in transformed yeast. Molecular General Genetics 241: 319-326.
121. McCafferty, J., A.D. Griffiths, G. Winter, D.J. Chiswell (1990) Phage antibodies: filamentous phage displaying antibody variable domains. Nature 348: 552-554.
122. Model, P., and M. Russell (1988) The filamentous bacteriophages. In Calendar, R. (1988) ed., The Bacteriophages vols. 1 and 2. Plenum, New York.
123. Montag, D., I. Riede, M.L. Eschbach, M. Degen, and U. Henning (1987) Receptor-recognizing proteins of T-even type bacteriophages: Constant and hypervariable regions and an unusual case of evolution. Journal of Molecular Biology 196: 165-174.
124. Moodie, S.A. et al.(1993) Complexes of Ras-GTP with Raf-1 and mitogen activated protein kinase kinase. Science 260: 1658-1661.
125. Moran, P., and I.W. Caras (1991) Fusion of sequence elements from non-anchored proteins to generate a fully functional signal for glycophosphatidylinositol membrane anchor attachment. Journal of Cell Biology 115: 1595-1600.
126. Morona, R., M. Klose, and U. Henning (1984) *Escherichia coli* K-12 Outer membrane protein (ompA) as a bacteriophage receptor: analysis of mutant genes expressing altered proteins. Journal of Bacteriology 159: 570-578.
127. Morona, R., C. Kramer, and U. Henning (1985) Bacteriophage receptor area of outer membrane protein ompA of *Escherichia coli* K-12. Journal of Bacteriology 164: 539-543.
128. Mosely, B., M.P. Beckmann, C.J. March, R.L. Idzerda, and S. Gimpel (1989) The murine interleukin-4 receptor: Molecular cloning and characterization of secreted and membrane bound forms. Cell 59: 335-48.
129. O'Dowd, B.F., R.J. Lefkowitz, and M.G. Caron (1989) Structure of the adrenergic and related receptors. Annual Review of Neuroscience 12: 67-83.
130. Olyhoek, A.J. et al. (1991) Cloning and expression in *E. coli* of *opc*, the gene for an unusual Class 5 outer membrane protein from Neisseria meningitides. Microbial Pathogenesis 11: 249-257.
131. Ondetti, M.D., B. Rubin, and D.W. Cushman (1977) Design of specific inhibitors of Angiotensin converting enzyme: new class of orally active antihypertensive agents. Science 196: 441-444.
132. Onu, M., G. Raab, K. Lau, J.A. Abraham, and M. Klagsbrun (1994) Purification and Characterization of Transmembrane Forms of Heparin-binding EGF-like Growth Factor. Journal of Biological Chemistry 269(49): 31315-21.
133. Ophir, R. and J.M. Gershoni (1995) Biased random mutagenesis of peptides: determination of mutation frequency by computer simulation. Protein Engineering 8: 143-146.
134. Parmley, S.F., and G.P. Smith (1988) Antibody-selectable filamentous fd phage vectors: affinity purification of target genes. Gene 73: 305-318.
135. Pattus, F., D. Massotte, H.U. Wilmsen, J. Lakey, D. Tsernoglou, A. Tucker, and M.W. Parker (1990) Colicins: prokaryotic killer-pores. Experientia 46: 180 192.
136. Paul, W.E. ed. (1993) Chapters 1-3. Fundamental Immunology. Raven Press, New York.
137. Paulson, J.C. (1989) Glycoproteins: what are the sugar chains for? Trends in Biochemical Science 14: 272-276.
138. Phizicky, E.M. and S. Fields (1995) Protein-protein interactions: methods for detection and analysis. Microbiological Reviews 59 (1): 94-123.
139. Pullman, A., J. Jortner and B. Pullman (1992) Membrane Proteins: Structures, Interactions, and Models. Kluwer Academic Publishers: Dordrecht.
140. Raulet, D.H. (1989) The structure, function, and molecular genetics of the g/d T cell receptor. Annual Review of Immunology 7: 175-207.
141. Ried, G., R. Koebnik, I. Hindennach, B. Mutschler, and U. Henning (1994) Membrane topology and assembly of the outer membrane protein OmpA of *Escherichia coli* K12. Molecular General Genetics 243: 127-135.
142. Rose, J.K., and J.E. Bergmann (1982) Expression from cloned cDNA of cell-surface forms of the glycoprotein of vesicular stomatitis virus in eukaryotic cells. Cell 30: 753-62.
143. Rossmann, M.G. et al. (1985) Structure of a human common cold virus and functional relationship to other picornaviruses. Nature 317: 145-153.
144. Ruppert, A. et al. (1994) OmpA-FMDV-VP1 fusion proteins: production, cell surface exposure and immune responses ot the major antigenic domain of foot-and-mouth disease virus. Vaccine 12(6): 492-8.
145. Saier, M.H. Jr., P.K. Werner, and M. Muller (1989) Insertion of proteins into bacterial membranes: Mechanism, characteristics and comparisons with the eukaryotic process. Microbiological Reviews 53: 333-366.
146. Sambrook, J., E.F. Fritsch, and T. Maniatis (1989) Molecular Cloning: A Laboratory Manual. Cold Spring Harbor Lab, Cold Spring Harbor, NY.
147. Scott, J.K. and G.P. Smith (1990) Searching for peptide ligands with an epitope library. Science 249: 386-390.
148. Seed, B. (1987) An LFA-3 cDNA encodes a phospholipid linked membrane protein homologous to its receptor CD2. Nature 329: 840-842.
149. Shaw, A.S., P.J. Rottier, and J.K. Rose (1988) Evidence for the loop model of signal-sequence insertion into the endoplasmic reticulum. Proceedings of the National Academy of Science USA 85: 7592-7596.
150. Shevach, E.M. (1993) Accessory Molecules. pp. 531-570 in Paul, W.E. ed. Fundamental Immunology Raven Press, New York.
151. Shia, M.A., and H.F. Lodish (1989) The two subunits of the human asialoglycoprotein receptor have different fates when expressed alone in fibroblasts. Proceedings of the National Academy of Science USA 86: 1158-1162.
152. Silve, S., C. Volland, C. Garnier, R. Jund, M.R. Chevallier, and T.R. Haguenauer (1991) Membrane insertion of uracil permease, a polytopic yeast plasma membrane protein. Molecular Cell Biology 11: 1114-1124.
153. Singer, S.J. (1990) The structure and insertion of integral proteins in membranes. Annual Review of Cell Biology 6: 247-296.
154. Sirakova, T., P.E. Kolattukudy, D. Murwin, J. Billy, E. Leake, D. Lim, T. Demaria, and L. Bakaletz (1994) Role of fimbriae expressed by nontypeable *Haemophilus influenzae* in pathogenesis of and protection against otitis media and relatedness of the fimbrin subunit to outer membrane protein A. Infection and Immunity 62: 2002-2020.
155. Smith, G.P. (1985) Filamentous fusion phage: novel expression vectors that display cloned antigens on the virion surface. Science 249: 386-390.
156. Sorkin, B.C., J.J. Hemperly, G.M. Edelman, and B.A. Cunningham (1988) Structure of the gene for the liver cell adhesion molecule, L-CAM. Proceedings of the National Academy of Science USA 85: 7617-7621.
157. Staunton, D.E., S.D. Marlin, C. Stratowa, M.L. Dustin, and T.A. Springer (1988) Primary structure of ICAM-1 demonstrates interaction between members of the immunoglobulin and integrin supergene families. Cell 52: 925-933.
158. Staunton, D.E., V.J. Merluzzi, R. Rothlein, R. Barton, S.D. Marlin, and T.A. Springer (1989) A cell adhesion molecule, ICAM-1, is the major surface receptor for rhinovurses. Cell 56: 849-853.
159. Staunton, D.E., M.L. Dustin, H.P. Erickson, and T.A. Springer (1990) The arrangement of the immunoglobulin-like domains of ICAM-1 and the binding sites for LFA-1 and rhinovirus. Cell: 61: 243-354.
160. Swanson, J, O. Barrera, J. Sola, and J. Boslego (1988) Expression of outer membrane protein II by *gonococci* in experimental gonorrhea. Journal of Emprical Medicine 168: 2121-2129.
161. Takeyasu, K., M.M. Tamkun, N.R. Siegel, and D.M. Fambrough (1987) Expression of hybrid (Na++K+)-ATPase molecules after transfection of mouse Ltk cells with DNA encoding the beta-subunit of an avian brain sodium pump. Journal of Biological Chemistry 262: 10733-10740.
162. Tawfik, D.S. et al. (1995) Unexpectedly high occurrence of catalytic antibodies in MRL/1 pr and SJL mice immunized with a transition-state analog: is there a linkage to autoimmunity? Proceedings of the National Academy of Sciences, USA 92(6): 2145-9.
163. Tuerk, C., and L. Gold (1990) Systematic evolution of ligands by exponential enrichment: RNA ligands to bacteriophage T4 DNA polymerase. Science 249: 505-510.
164. Tykocinski, M.L., H.K. Shu, D.J. Ayers, E.I. Walter, R.R. Getty, R.K. Groger, C.A. Hauer, and M.E. Medof (1988) Glycolipid reanchoring of T-lymphocyte surface antigen CD8 using the 3' end sequence of decay-accelerating factor's mRNA. Proceedings of the National Academy of Science USA 85: 3555-3559.
165. Tyler, K.L. and B.N. Fields (1996) Pathogenesis of viral infections. In Fields, B.N. et al.: 173-218.
166. van Zeijl, M., S.V. Johann, E. Class, J. Cunningham, R. Eddy, T.B. Shows, and B. O'Hara (1994) A human amphotrophic retrovirus receptor is a second member of the gibbon ape leukemia virus receptor family. Proceedings of the National Academy of Science USA 91: 1168-1172.
167. Virji, M., K. Makepeace, D.J. Ferguson, M. Achtman, J. Sarkari, and E.R. Moxon (1992) Expression of the Opc protein correlates with invasion of epithelial and endothelial cells by *Neisseria meningitidis*. Molecular Microbiology 6: 1271-1279.
168. Vogel, H., and F. J"hnig (1986) Models for the structure of outer membrane proteins of *Escherichia coli* derived from Raman spectroscopy and prediction methods. Journal of Molecular Biology 190: 191-199.
169. Voijtek, A.B. et al. (1993) Mammalian Ras interacts directly with serine/threonine kinase Raf. Cell 74: 205-214.
170. Ward, R.L. et al. (1996) Retrieval of human antibodies from phage-display libraries using enzymatic cleavage. Journal of Immunological Methods 189 (1): 73-82.
171. Weiser, J.N., and E.C. Gotschlich (1991) Outer membrane protein A contributes to serum resistance and pathogenicity of *E. coli* K-1. Infection and Immunity 59: 2252-2258.
172. Wessels, H.P., and M. Spiess (1988) Insertion of a multispanning membrane protein occurs sequentially and requires only one signal sequence. Cell 55: 61-70.
173. Wessels, H.P., J.P. Beltzer, and M. Spiess (1991) Analysis of protein topology in the endoplasmic reticulum. Methods in Cell Biology 34: 287-301.
174. Winkler, H.H. and D.H. Duckworth (1971) Journal of Bacteriology 107: 259-267.
175. Winter, G. and C. Milstein (1991) Man-made antibodies. Nature 349: 293-299.
176. Yasukawa, K. et al. (1990) Purification and characterization of soluble human IL-6 receptor expressed in CHO cells. Journal of Biochemistry 108: 673-676.
177. Zeilestra-Ryalls, J. et al. (1993) Sequence analysis and phenotypic characterization of groEL mutations that block lambda and T4 Bacteriophage growth. Journal of Bacteriology 175(4): 1134-43.
178. Zhang, Y.B., and J.K. Broome-Smith (1990) Correct insertion of a simple eukaryotic plasma-membrane protein into the cytoplasmic membrane of *Escherichia coli*. Gene 96: 51-57.
179. Ziedel, M. et al. (1995) Genetic and functional characterization of human autoantibodies using combinatorial phage display libraries. Annals of the New York Academy of Science 764: 559-64.
180. Zlotnick, G.W., V.T. Sanfilippo, J.A. Mattler, D.H. Kirkley, R.A. Boykins, and R.C. Seid Jr. (1988) Purification and characterization of a peptidoglycan-associated lipoprotein from *Haemophilus influenzae*. Journal of Biological Chemistry 263: 9790-9794.

## Claims

1. A method for determining the interaction between a (poly)peptide of interest and a target compound wherein at least one interactant is unknown, comprising the steps
a) constructing a prokaryotic or eukaryotic cell population by engineering it to express a membrane polypeptide which has the (poly)peptide of interest in its extracellular domain, said extracellular domain being exhibited in one or more copies on each cell's surface immediately adjacent to an adhesion domain for a toxic agent, said adhesion domain and said extracellular domain belonging to the same membrane polypeptide, such that upon interaction of the target compound and the (poly)peptide of interest the toxic agent is occluded from the adhesion domain,
b) exposing the cell population to a target compound or a population of target compounds for a period of time sufficient for the target compound to interact with the (poly)peptide of interest,
c) exposing the cell population, simultaneously with or after step b), to the toxic agent for a period of time sufficient for the agent to attach to its adhesion domain,
d) selecting for the clones which are at a growth advantage due to the interaction of the (poly)peptide of interest with the target compound,
e) allowing the selected clones to multiply,
f) identifying the unknown interaction partner and isolating it.

2. The method of claim 1 wherein said membrane polypeptide is selected from glycolipid-anchored polypeptides, bitopic integral membrane polypeptides, polytopic integral membrane polypeptides, or membrane-bound isoforms of a secreted polypeptide.

3. The method of claim 1 wherein the unknown interaction partner is the (poly)peptide of interest.

4. The method of claim 3 wherein said extracellular domain of the membrane protein contains a highly variable region which is, upon interaction with the target compound, identified as the (poly)peptide of interest or a section thereof.

5. The method of claim 4 wherein said variable domain comprises (poly)peptides encoded by cDNA library or sections thereof.

6. The method of claim 1 wherein said membrane polypeptide is heterologous for the cells.

7. The method of claim 1 or 4 wherein said membrane polypeptide is genetically engineered to contain the extracellular domain and the adhesion domain residing adjacent to each other on the cellular surface.

8. The method of claim 7 wherein said extracellular domain contains a highly variably region.

9. The method of claim 1 wherein said target compound is composed of a mixture of compounds.

10. The method of claim 9 in the form of a high-throughput screening assay wherein the cells are exposed to a multiplicity of target compounds from which an unknown interaction partner for a (poly)peptide of interest is to be identified.

11. The method of claim 1 wherein said interaction is defined by the ability of the (poly)peptide of interest to tag or partition the target compound.

12. The method of claim 1 wherein said interaction is an antagonizing action.

13. The method of claim 1 wherein said interaction is an agonizing action.

14. The method of claim 1 wherein the target compound is an antibody or an antibody fragment of the (poly)peptide of interest.

15. The method of claim 1 wherein the target compound is a compound that is secreted by the cells.

16. The method of claim 1 wherein said toxic agent is a pathogenic agent.

17. The method of claim 16 wherein the pathogenic agent is a bacteriophage.

18. The method of claim 16 wherein the pathogenic agent is a virus.

19. The method of claim 16 wherein said pathogenic agent has a stronger or weaker effect on the cells than the corresponding naturally occuring pathogenic agent.

20. The method of claim 16 wherein the parameters selected from virulence of the pathogenic agent, infective efficiency of the pathogenic agent and/or concentration of the pathogenic agent; concentration of the target compound; growth rate of the cell population and number of variable regions exhibited on the surface of each cell, are adjusted to levels that result in a selection of clones that exhibit variable domains that interact with the target compound with an affinity greater than a prescribed level.

21. The method of claim 4 further comprising iterating steps b) through e) until the cell population comprises the desired number of distinct clones, all of which exhibit variable extracellular domains that interact with the target compound above a desired interaction level.

22. The method of claim 4 further comprising the steps: introducing new sequence variations at the variable regions of clones selected in step d); allowing the clones selected in step d), now bearing new sequence variations in their variable domains, to multiply in a nutrient medium; iterating steps g) through i) until selected clones exhibit the desired type of interaction with the target compound.

23. The method of claim 1 wherein said membrane protein exhibits on the surface of the cell an additional domain adjacent to both the variable domain and the adhesion domain, which additional domain is known to interact with the target compound in a defined manner.

24. A method according to claim 4 for identifying (poly)peptides of interest that interact with a target compound and affect its function characterized by the following steps screening the variable regions exhibited by the clones selected in step d) and amplified in step e) for their ability to affect the function of the target compound.

25. A method according to claim 4 for identifying polypeptides of interest as enzymes, said method further comprising placing the cell population in a nutrient medium in which each cell's supply of a vital compound depends upon that cell's variable region acting enzymatically upon a compound structurally similar but not identical to the target compound.

## Patentansprüche

1. Verfahren zur Bestimmung der Wechselwirkung zwischen einem (Poly)peptid, an dem Interesse besteht, und einer Zielverbindung, wobei mindestens einer der Wechselwirkungspartner unbekannt ist, mit den Schritten:
a) Herstellen einer prokaryontischen oder eukaryontischen Zellpopulation, die so hergestellt wird, dass sie ein Membran-Polypeptid exprimiert, welches das (Poly)peptid, an dem Interesse besteht, in seiner extrazellulären Domäne aufweist, wobei die extrazelluläre Domäne in einer oder mehreren Kopien an jeder Zelloberfläche unmittelbar benachbart einer Adhäsionsdomäne für ein toxisches Agens präsentiert wird, wobei die Adhäsionsdomäne und die extrazelluläre Domäne zum selben Membran-Polypeptid gehören, so dass nach einer Wechselwirkung der Zielverbindung und des (Poly)peptids, an dem Interesse besteht, das toxische Agens von der Adhäsionsdomäne ausgeschlossen ist,
b) Aussetzen der Zellpopulation einer Zielverbindung oder einer Population von Zielverbindungen für eine Zeitdauer, die ausreicht, damit die Zielverbindung mit dem (Poly)peptid, an dem Interesse besteht, in Wechselwirkung tritt,
c) Aussetzen der Zellpopulation - gleichzeitig mit oder nach Schritt b) - dem toxischen Agens für eine Zeitdauer, die ausreicht, dass das Agens an seiner Adhäsionsdomäne anhaftet,
d) Auswählen jener Klone, die infolge der Wechselwirkung des (Poly)peptids, an dem Interesse besteht, mit der Zielverbindung einen Wachstumsvorteil haben,
e) Vermehren-Lassen der ausgewählten Klone,
f) Identifizieren des unbekannten Wechselwirkungspartners und Isolieren desselben.

2. Verfahren nach Anspruch 1, wobei das Membran-Polypeptid ausgewählt ist aus an Glykolipid verankerten Polypeptiden, bitopischen integralen Membran-Polypeptiden, polytopischen integralen Membran-Polypeptiden oder Membran-gebundenen Isoformen eines sezernierten Polypeptids.

3. Verfahren nach Anspruch 1, wobei der unbekannte Wechselwirkungspartner das (Poly)peptid ist, an dem Interesse besteht.

4. Verfahren nach Anspruch 3, wobei die extrazelluläre Domäne des Membran-Proteins eine stark variable Region enthält, die nach Wechselwirkung mit der Zielverbindung als das (Poly)peptid, an dem Interesse besteht, oder ein Teil davon, identifiziert wird.

5. Verfahren nach Anspruch 4, wobei die variable Domäne von einer cDNA-Bank kodierte (Poly)peptide oder Teile davon aufweist.

6. Verfahren nach Anspruch 1, wobei das Membran-Polypeptid für die Zellen heterolog ist.

7. Verfahren nach Anspruch 1 oder 4, wobei das Membran-Polypeptid gentechnisch so konstruiert wird, dass es die extrazelluläre Domäne und die Adhäsionsdomäne einander benachbart an der Zelloberfläche enthält.

8. Verfahren nach Anspruch 7, wobei die extrazelluläre Domäne eine stark variable Region enthält.

9. Verfahren nach Anspruch 1, wobei die Zielverbindung aus einer Mischung von Verbindungen zusammengesetzt ist.

10. Verfahren nach Anspruch 9 in Form eines Screening-Tests mit hohem Durchsatz, wobei die Zellen einer Mehrzahl von Zielverbindungen ausgesetzt werden, aus welchen ein unbekannter Wechselwirkungspartner für ein (Poly)peptid, an dem Interesse besteht, identifiziert werden soll.

11. Verfahren nach Anspruch 1, wobei die Wechselwirkung durch die Fähigkeit des (Poly)peptids, an dem Interesse besteht, sich an die Zielverbindung anzuhängen oder diese zu teilen definiert ist.

12. Verfahren nach Anspruch 1, wobei die Wechselwirkung eine antagonisierende Wirkung ist.

13. Verfahren nach Anspruch 1, wobei die Wechselwirkung eine agonisierende Wirkung ist.

14. Verfahren nach Anspruch 1, wobei die Zielverbindung ein Antikörper oder ein Antikörperfragment des (Poly)peptids, an dem Interesse besteht, ist.

15. Verfahren nach Anspruch 1, wobei die Zielverbindung eine Verbindung ist, die von den Zellen sezerniert wird.

16. Verfahren nach Anspruch 1, wobei das toxische Agens ein pathogenes Agens ist.

17. Verfahren nach Anspruch 16, wobei das pathogene Agens ein Bakteriophage ist.

18. Verfahren nach Anspruch 16, wobei das pathogene Agens ein Virus ist.

19. Verfahren nach Anspruch 16, wobei das pathogene Agens auf die Zellen eine stärkere oder schwächere Wirkung hat als das korrespondierende natürlich vorkommende pathogene Agens.

20. Verfahren nach Anspruch 16, wobei die Parameter ausgewählt aus Virulenz des pathogenen Agens, Infektionswirksamkeit des pathogenen Agens und/oder Konzentration des pathogenen Agens; Konzentration der Zielverbindung; Wachstumsrate der Zellpopulation und Anzahl der auf der Oberfläche jeder Zelle aufscheinenden variablen Regionen so eingestellt werden, dass sie zu einer Selektion von Klonen führen, die variable Domänen aufweisen, welche mit der Zielverbindung mit einer Affinität, die über einem vorgeschriebenen Niveau liegt, in Wechselwirkung treten.

21. Verfahren nach Anspruch 4, welches weiters eine Wiederholung der Schritte b) bis e) aufweist, bis die Zellpopulation die gewünschte Anzahl bestimmter Klone aufweist, die alle variable extrazelluläre Domänen aufweisen, welche mit der Zielverbindung über ein gewünschtes Maß an Wechselwirkung hinaus in Wechselwirkung treten.

22. Verfahren nach Anspruch 4, welches weiters die Schritte aufweist: Einführen neuer Sequenz-Variationen an den variablen Regionen von in Schritt d) ausgewählten Klonen; Vermehren-Lassen der in Schritt d) ausgewählten Klone, die nun neue Sequenz-Variationen in ihren variablen Domänen aufweisen, in einem Nährmedium; Wiederholen der Schritte g) bis i) bis ausgewählte Klone die gewünschte Art der Wechselwirkung mit der Zielverbindung aufweisen.

23. Verfahren nach Anspruch 1, wobei das Membran-Protein an der Oberfläche der Zelle eine zusätzliche Domäne benachbart sowohl der variablen Domäne als auch der Adhäsionsdomäne aufweist, von welcher zusätzlichen Domäne bekannt ist, dass sie mit der Zielverbindung auf festgelegte Weise in Wechselwirkung tritt.

24. Verfahren nach Anspruch 4 zur Identifizierung von (Poly)peptiden, an welchen Interesse besteht, die mit einer Zielverbindung in Wechselwirkung treten und ihre Funktion beeinflussen, gekennzeichnet durch die folgenden Schritte: Screenen der variablen Regionen, die die in Schritt d) ausgewählten und in Schritt e) amplifizierten Klone aufweisen, auf ihre Fähigkeit, die Funktion der Zielverbindung zu beeinflussen.

25. Verfahren nach Anspruch 4 zur Identifizierung von Polypeptiden, an welchen Interesse besteht, als Enzyme, welches Verfahren weiters das Platzieren der Zellpopulation in ein Nährmedium, in welchem die Versorgung jeder Zelle mit einer lebenswichtigen Verbindung von der variablen Region dieser Zelle abhängt, die enzymatisch auf eine Verbindung wirkt, die der Zielverbindung strukturell ähnlich jedoch nicht identisch mit dieser ist.

## Revendications

1. Méthode de détermination de l'interaction entre un (poly)peptide d'intérêt et un composé cible dans laquelle au moins un des composé interagissant est inconnu, comprenant les étapes consistant
a) à construire une population de cellules procaryotes ou eucaryotes en la concevant de telle sorte qu'elle exprime un polypeptide membranaire qui comprend le (poly)peptide d'intérêt dans son domaine extracellulaire, ledit domaine extracellulaire étant présenté en une ou plusieurs copies à la surface de chaque cellule, immédiatement adjacent à un domaine d'adhésion pour un agent toxique, ledit domaine d'adhésion et ledit domaine extracellulaire appartenant au même polypeptide membranaire, de telle sorte qu'après interaction du composé cible et du (poly)peptide d'intérêt, l'agent toxique soit occlus du domaine d'adhésion.
b) à exposer la population de cellules à un composé cible ou à une population de composés cibles pendant une période de temps suffisante pour que le composé cible interagisse avec le (poly)peptide d'intérêt.
c) à exposer la population de cellules, simultanément à ou après l'étape b), à l'agent toxique pendant une période de temps suffisante pour que l'agent s'attache à son domaine d'adhésion,
d) à sélectionner les clones qui ont un avantage de croissance dû à l'interaction du (poly)peptide d'intérêt avec le composé cible,
e) à laisser les clones sélectionnés se multiplier,
f) à identifier le partenaire d'interaction inconnu et à l'isoler.

2. Méthode selon la revendication 1, dans laquelle ledit polypeptide membranaire est choisi parmi des polypeptides ancrés à un glycolipide, des polypeptides membranaires intégraux bitopiques, des polypeptides membranaires intégraux polytopiques, ou des isoformes d'un polypeptide sécrété liées à la membrane.

3. Méthode selon la revendication 1, dans laquelle le partenaire d'interaction inconnu est le (poly)peptide d'intérêt.

4. Méthode selon la revendication 3, dans laquelle ledit domaine extracellulaire de la protéine membranaire contient une région hautement variable qui est, après interaction avec le composé cible, identifié comme le (poly)peptide d'intérêt ou l'une de ses parties.

5. Méthode selon la revendication 4, dans laquelle ledit domaine variable comprend des (poly)peptides codés par une banque d'ADNc ou des parties de celle-ci.

6. Méthode selon la revendication 1, dans laquelle ledit polypeptide membranaire est hétérologue pour les cellules.

7. Méthode selon la revendication 1 ou 4, dans laquelle ledit peptide membranaire est préparé par des techniques de génie génétique de façon à contenir le domaine extracellulaire et le domaine d'adhésion situés en positions adjacentes l'une à l'autre sur la surface cellulaire.

8. Méthode selon la revendication 7, dans laquelle ledit domaine extracellulaire contient une région hautement variable.

9. Méthode selon la revendication 1, dans laquelle ledit composé cible est composé d'un mélange de composés.

10. Méthode selon la revendication 9, sous la forme d'un test de criblage à haut débit, dans laquelle les cellules sont exposées à de nombreux composés cibles parmi lesquels un partenaire d'interaction inconnu pour un(poly)peptide d'intérêt doit être identifié.

11. Méthode selon la revendication 1, dans laquelle ladite interaction est définie par la capacité du (poly)peptide d'intérêt à marquer ou partager le composé cible.

12. Méthode selon la revendication 1, dans laquelle ladite interaction est une action antagoniste.

13. Méthode selon la revendication 1, dans laquelle ladite interaction est une action agoniste.

14. Méthode selon la revendication 1, dans laquelle le composé cible est un anticorps ou un fragment d'anticorps du (poly)peptide d'intérêt.

15. Méthode selon la revendication 1, dans laquelle le composé cible est un composé sécrété par les cellules.

16. Méthode selon la revendication 1, dans laquelle ledit agent toxique est un agent pathogène.

17. Méthode selon la revendication 16, dans laquelle l'agent pathogène est un bactériophage.

18. Méthode selon la revendication 16, dans laquelle l'agent pathogène est un virus.

19. Méthode selon la revendication 16, dans laquelle ledit agent pathogène a un effet plus fort ou plus faible sur les cellules que l'agent pathogène naturel correspondant.

20. Méthode selon la revendication 16, dans laquelle les paramètres choisis parmi la virulence de l'agent pathogène, l'efficacité d'infection de l'agent pathogène et/ou la concentration de l'agent pathogène ; la concentration du composé cible ; la vitesse de croissance de la population cellulaire et le nombre de régions variables présentées à la surface de chaque cellule, sont ajustés à des niveaux qui permettent d'obtenir une sélection des clones qui présentent des domaines variables qui interagissent avec le composé cible avec une affinité plus grande qu'un niveau prescrit.

21. Méthode selon la revendication 4, comprenant en outre les étapes itératives b) à e) jusqu'à ce que la population cellulaire comprenne le nombre désiré de clones distincts, présentant tous des domaines extracellulaires variables qui interagissent avec le composé cible au dessus d'un niveau d'interaction désiré.

22. Méthode selon la revendication 4, comprenant en outre les étapes consistant : à introduire de nouvelles variations de séquence au niveau des régions variables des clones sélectionnés dans l'étape d) ; à permettre aux clones sélectionnés dans l'étape d), portant à présent de nouvelles variations de séquence dans leurs domaines variables, de se multiplier dans un milieu nutritif ; à répéter les étapes g) à i) jusqu'à ce que les clones sélectionnés présentent le type désiré d'interaction avec le composé cible.

23. Méthode selon la revendication 1, dans laquelle ladite protéine membranaire présente à la surface de la cellule un domaine supplémentaire adjacent tant au domaine variable qu'au domaine d'adhésion, ce domaine supplémentaire étant connu pour interagir avec le composé cible de manière définie.

24. Méthode selon la revendication 4, pour l'identification de (poly)peptides d'intérêt qui interagissent avec un composé cible et affectent sa fonction, caractérisée par les étapes suivantes de criblage des régions variables présentées par les clones sélectionnés dans l'étape d) et amplifiés dans l'étape e) pour leur capacité à affecter la fonction du composé cible.

25. Méthode selon la revendication 4, pour l'identification de (poly)peptides d'intérêt tels que des enzymes, ladite méthode comprenant en outre l'étape consistant à placer la population cellulaire dans un milieu nutritif dans lequel l'apport à chaque cellule d'un composé vital dépend de l'action enzymatique de la région variable de cette cellule sur un composé de structure similaire mais non identique au composé cible.
